# EUROPEAN PATENT APPLICATION

(11) **EP 4 144 838 A1**
(43) Date of publication of application: **08.03.2023**
(21) Application number: 21195143.9
(22) Date of filing: 06.09.2021
(51) Int. Cl.: C12N 9/10, C12N 9/12, C12P 5/02

(54) **ORGANISMS PRODUCING LESS CROTONIC ACID**

(71) Applicant: Global Bioenergies, 91000 Evry Cedex (FR)
(72) Inventor: VILLIER, Benoit, Tours, 37000 (FR); THIBAUT, Denis, Paris, 75013 (FR)
(74) Representative: Vossius & Partner Patentanwälte Rechtsanwälte mbB

(57) **Abstract**

The present invention relates to a recombinant organism or microorganism having a decreased pool of crotonic acid compared to the organism or microorganism from which it is derived due to at least: (i) a decreased conversion of acetaldehyde into crotonaldehyde; and/or (ii) a decreased conversion of crotonyl-CoA into crotonaldehyde; and/or (iii) a decreased conversion of crotonaldehyde into crotonic acid. Moreover, the present invention relates to the use of such a recombinant organism or microorganism for the production of alkenes with the enzyme ferulic acid decarboxylase. Further, the present invention relates to a method for the production of isobutene or butadiene by culturing such a recombinant organism or microorganism in a suitable culture medium under suitable conditions.

## Description

The present invention relates to a recombinant organism or microorganism having a decreased pool of crotonic acid compared to the organism or microorganism from which it is derived due to at least: (i) a decreased conversion of acetaldehyde into crotonaldehyde; and/or (ii) a decreased conversion of crotonyl-CoA into crotonaldehyde; and/or (iii) a decreased conversion of crotonaldehyde into crotonic acid. Moreover, the present invention relates to the use of such a recombinant organism or microorganism for the production of alkenes with the enzyme ferulic acid decarboxylase. Further, the present invention relates to a method for the production of isobutene or butadiene by culturing such a recombinant organism or microorganism in a suitable culture medium under suitable conditions.

### BACKGROUND OF THE INVENTION

A large number of chemical compounds are currently derived from petrochemicals. Alkenes (such as ethylene, propylene, the different butenes, or else the pentenes, for example) are used in the plastics industry, for example for producing polypropylene or polyethylene, and in other areas of the chemical industry and that of fuels. Butylene exists in four forms, one of which, isobutene (also referred to as isobutylene), enters into the composition of methyl-tert-butyl-ether (MTBE), an anti-knock additive for automobile fuel. Isobutene can also be used to produce isooctene, which in turn can be reduced to isooctane (2,2,4-trimethylpentane); the very high octane rating of isooctane makes it the best fuel for so-called "gasoline" engines. Alkenes such as isobutene are currently produced by catalytic cracking of petroleum products (or by a derivative of the Fischer-Tropsch process in the case of hexene, from coal or gas). The production costs are therefore tightly linked to the price of oil. Moreover, catalytic cracking is sometimes associated with considerable technical difficulties which increase process complexity and production costs.

The production by a biological pathway of alkenes such as isobutene is called for in the context of a sustainable industrial operation in harmony with geochemical cycles. The first generation of biofuels consisted in the fermentative production of ethanol, as fermentation and distillation processes already existed in the food processing industry. The production of second generation biofuels is in an exploratory phase, encompassing in particular the production of long chain alcohols (butanol and pentanol), terpenes, linear alkanes and fatty acids. Two recent reviews provide a general overview of research in this field: Ladygina et al. (Process Biochemistry 41 (2006), 1001) and Wackett (Current Opinions in Chemical Biology 21 (2008), 187).

The conversion of isovalerate to isobutene by the yeast *Rhodotorula minuta* has been described (Fujii et al. (Appl. Environ. Microbiol. 54 (1988), 583)), but the efficiency of this reaction is far from permitting an industrial application. The reaction mechanism was elucidated by Fukuda et al. (BBRC 201 (1994), 516) and involves a cytochrome P450 enzyme which decarboxylates isovalerate by reduction of an oxoferryl group Fe^{v}=O. Large-scale biosynthesis of isobutene by this pathway seems highly unfavourable, since it would require the synthesis and degradation of one molecule of leucine to form one molecule of isobutene. Also, the enzyme catalyzing the reaction uses heme as cofactor, poorly lending itself to recombinant expression in bacteria and to improvement of enzyme parameters. For all these reasons, it appears very unlikely that this pathway can serve as a basis for industrial exploitation. Other microorganisms have been described as being marginally capable of naturally producing isobutene from isovalerate; the yields obtained are even lower than those obtained with *Rhodotorula minuta* (Fukuda et al. (Agric. Biol. Chem. 48 (1984), 1679)). Gogerty et al. (Appl. Environm. Microbiol. 76 (2010), 8004-8010) and van Leeuwen et al. (Appl. Microbiol. Biotechnol. 93 (2012), 1377-1387) describe the production of isobutene from acetoacetyl-CoA by enzymatic conversions wherein the last step of the proposed pathway is the conversion of 3-hydroxy-3-methylbutyric acid (also referred to as 3-hydroxyisovalerate (HIV)) by making use of a mevalonate diphosphate decarboxylase.

This reaction for the production of isobutene from 3-hydroxy-3-methylbutyric acid is also described in WO2010/001078 which, in general terms, describes methods for generating alkenes through a biological process, in particular methods for producing terminal alkenes (in particular propylene, ethylene, 1-butylene, isobutylene or isoamylene) from molecules of the 3-hydroxyalkanoate type.

WO2012/052427 also describes a method for generating alkenes through a biological process while, in particular, a method for producing alkenes (for example propylene, ethylene, 1-butylene, isobutylene or isoamylene) from molecules of the 3-hydroxyalkanoate type is described. In this context, the reaction for the production of isobutene from 3-hydroxy-3-methylbutyric acid is also described in WO2012/052427.

WO 2016/042012 describes methods for producing said 3-hydroxy-3-methylbutyric acid. In particular, WO 2016/042012 describes methods for producing 3-hydroxy-3-methylbutyric acid comprising the step of enzymatically converting 3-methylcrotonyl-CoA into 3-methylcrotonic acid and the step of enzymatically further converting the thus produced 3-methylcrotonic acid into 3-hydroxy-3-methylbutyric acid.

In Gogerty et al. (loc. cit.) and in van Leeuwen et al. (loc. cit.) the production of 3-hydroxy-3-methylbutyric acid is proposed to be achieved by the conversion of 3-methylcrotonyl-CoA via 3-hydroxy-3-methylbutyryl-CoA. In order to further improve the efficiency and variability of methods for producing isobutene from renewable resources, alternative routes for the provision of isobutene and its precursors have been developed by providing methods for the production of isobutene comprising the enzymatic conversion of 3-methylcrotonic acid (also termed 3-methyl-2-butenoic acid, 3,3-dimethylacrylic acid or senecioic acid) into isobutene.

In particular, in WO 2017/085167, methods for the production of isobutene have been described comprising the enzymatic conversion of 3-methylcrotonic acid into isobutene, wherein the enzymatic conversion of 3-methylcrotonic acid into isobutene is achieved by making use of a prenylated FMN-dependent decarboxylase associated with an FMN prenyl transferase, wherein said FMN prenyl transferase catalyzes the prenylation of a flavin cofactor (FMN or FAD) utilizing dimethylallyl phosphate (DMAP) into a flavin-derived cofactor while these enzymes have artificially been implemented in a pathway which ultimately leads to the production of isobutene. Moreover, in WO 2017/085167, methods have been described, wherein such a method further comprises (a) providing the 3-methylcrotonic acid by the enzymatic conversion of 3-methylcrotonyl-CoA into 3-methylcrotonic acid, or (b) providing the 3-methylcrotonic acid by the enzymatic conversion of 3-hydroxyisovalerate (HIV) into 3-methylcrotonic acid.

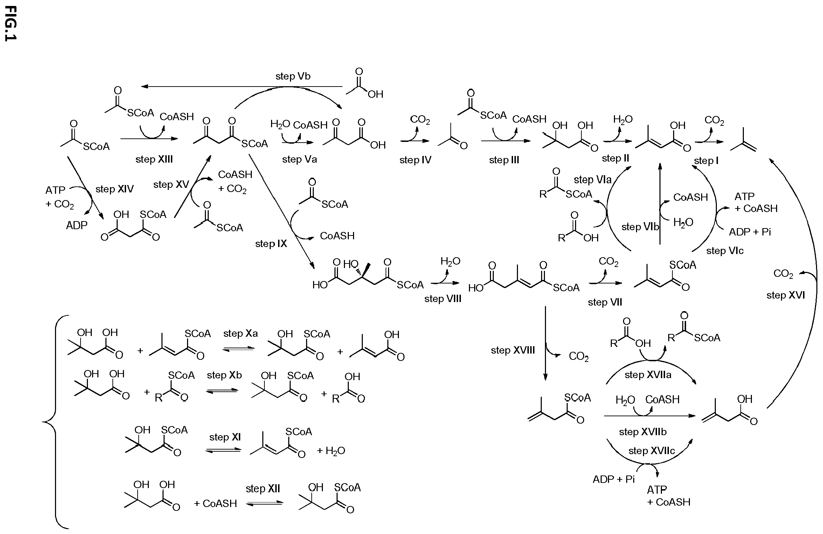

WO 2017/085167 also describes that this method which has been developed for the production of isobutene from 3-methylcrotonyl-CoA via 3-methylcrotonic acid or from 3-hydroxyisovalerate (HIV) via 3-methylcrotonic acid may be embedded in a pathway for the production of isobutene starting from acetyl-CoA which is a central component and an important key molecule in metabolism used in many biochemical reactions. The corresponding reactions are schematically shown in **Figure 1**.

In WO 2018/206262 it is described that 3-methylcrotonic acid is enzymatically converted into isobutene by making use of a prenylated FMN-dependent decarboxylase associated with an FMN prenyl transferase when dimethylallyl pyrophosphate (DMAPP) instead of DMAP is used. WO 2018/206262, moreover, describes that the enzymatic conversion of 3-methylcrotonic acid into isobutene which is achieved by making use of a prenylated FMN-dependent decarboxylase associated with an FMN prenyl transferase, wherein said FMN prenyl transferase catalyzes the prenylation of a flavin cofactor (FMN or FAD) utilizing dimethylallyl phosphate (DMAP) and/or dimethylallyl pyrophosphate (DMAPP) into a flavin-derived cofactor is a key step of the above overall metabolic pathway from acetyl-CoA into isobutene. It has been found that in this key step, the availability of dimethylallyl phosphate (DMAP) and/or dimethylallyl pyrophosphate (DMAPP) as well as the availability of the flavin cofactor FMN are limiting factors while in WO 2018/206262 improved methods by increasing the pool/amount of dimethylallyl phosphate (DMAP) and/or dimethylallyl pyrophosphate (DMAPP) in order to ensure the efficient biosynthesis of the prenylated flavin cofactor (FMN or FAD) are described.

WO 2020/188033 describes an improved method for the production of isobutene from acetyl-CoA, wherein the pool of available of acetyl-CoA in the production strain is increased through an increased uptake of pantothenate and/or an increased conversion of pantothenate into CoA.

Although, as described above, various approaches have been described in the prior art for producing isobutene by enzymatic conversions in biological systems, thereby allowing to use renewable resources as raw material, there is still a need to improve efficiency and effectiveness of such methods in order to increase yield and make them commercially attractive.

### SUMMARY OF THE INVENTION

The present invention meets this demand by providing a recombinant organism or microorganism having a decreased pool of crotonic acid compared to the organism or microorganism from which it is derived due to at least:
(i) a decreased conversion of acetaldehyde into crotonaldehyde; and/or
(ii) a decreased conversion of crotonyl-CoA into crotonaldehyde; and/or
(iii) a decreased conversion of crotonaldehyde into crotonic acid.

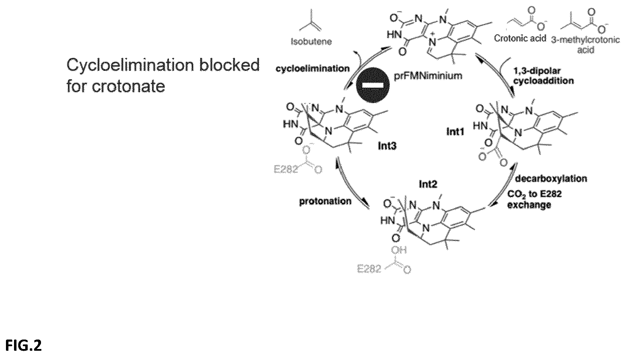

It has been surprisingly found by the inventors that crotonic acid is an irreversible inhibitor of the enzyme ferulic acid decarboxylase (FDC, see **FIG.2**), which is preferably used in industrial processes for the conversion of 3-methylcrotonic acid into isobutene. In recombinant organisms or microorganisms, 3-methylcrotonic acid, the substrate of FDC, can be produced from acetyl-CoA in a multistep enzymatic process. The final conversion of 3-methylcrotonic acid into isobutene can be carried out in the same recombinant organism or microorganism that has been used for the production of 3-methylcrotonic acid. In such a one-step process, it is required that the recombinant organism or microorganism encodes the enzyme FDC. Alternatively, isobutene may be produced from 3-methylcrotonic acid in a two-step process. For that, a fermentation culture medium of a recombinant organism or microorganism comprising the produced 3-methylcrotonic acid may be contacted with a recombinant organism or microorganism encoding the enzyme FDC in an in vivo or in vitro biotransformation reaction. In an alternative two-step process, a first recombinant organism or microorganism may be used to convert acetyl-CoA into 3-hydroxyisovaleric acid and the produced 3-hydroxyisovaleric acid may then be converted into isobutene by a second recombinant organism or microorganism (via 3-methylcrotonic acid and FDC).

When producing 3-methylcrotonic acid and/or isobutene in a recombinant organism or microorganism, crotonic acid could be a side product and, due to its inhibitory effect on FDC, even at trace amounts, decrease the productivity of the entire process. Thus, reducing and/or depleting the intracellular and extracellular levels of crotonic acid in a recombinant organism or microorganism that is used for the production of 3-methylcrotonic acid and/or isobutene can result in significantly improved product formation.

Herein, the inventors have identified acetaldehyde and crotonyl-CoA as two of the main sources of crotonic acid in a 3-methylcrotonic acid and/or isobutene production strain. In particular, it has been identified by the inventors that two molecules of acetaldehyde can condense into crotonaldehyde in the presence of an aldehyde lyase (or aldolase). Further, it has been identified that crotonyl-CoA can be reduced into crotonaldehyde by an aldehyde dehydrogenase and that the resulting crotonaldehyde can be oxidized to crotonic acid also by an aldehyde dehydrogenase. That is, the formation of crotonic acid in a 3-methylcrotonic acid and/or isobutene production strain may be prevented by reducing and/or inhibiting the conversion of acetaldehyde into crotonaldehyde and/or by reducing and/or inhibiting the conversion of crotonyl-CoA into crotonaldehyde and/or by reducing and/or inhibiting the conversion of crotonaldehyde into crotonic acid.

### A decreased pool of crotonic acid

The recombinant organism or microorganism according to the present invention is characterized in having a decreased pool of crotonic acid compared to the organism or microorganism from which it is derived due to:
(i) a decreased conversion of acetaldehyde into crotonaldehyde; and/or
(ii) a decreased conversion of crotonyl-CoA into crotonaldehyde; and/or
(iii) a decreased conversion of crotonaldehyde into crotonic acid.

The term "a decreased pool of crotonic acid" as used in the present invention means, in general terms, that the amount and/or the availability of crotonic acid in the recombinant (genetically modified) organism or microorganism or in the culture medium is lower than in the correspondingly non-modified organism or microorganism. In preferred embodiments, in the context of the present invention, "a decreased pool of crotonic acid" means that the amount and/or the availability of crotonic acid in the genetically modified, recombinant organism or microorganism is at least 5%, 10%, 20%, 30%, 40%, 50%, 60%, 70%, 80% or 90% lower than in the corresponding non-modified organism or microorganism.

It is to be understood that crotonic acid is a hydrophobic molecule that can readily diffuse across biological membranes. Due to its diffusion behaviour, the intracellular concentration of crotonic acid is expected to correlate at least to a certain extent with the crotonic acid concentration in the culture medium. Accordingly, the "pool of crotonic acid" is not strictly limited to the "intracellular pool" of crotonic acid in an organism or microorganism, but also extends to the culture medium in which the organism or microorganism is comprised. Accordingly, a first organism or microorganism may be determined to have a decreased pool of crotonic acid compared to a second organism or microorganism if the concentration of crotonic acid in the culture medium of the first organism or microorganism is lower than the concentration of crotonic acid in the culture medium of the second organism or microorganism. When making such comparisons, it is important to compare crotonic acid concentrations in cell cultures having comparable cell densities and, preferably, comparable culture volumes.

Within the present invention, it is to be understood that a recombinant organism or microorganism having a "decreased pool of crotonic acid" may also be defined as a recombinant organism or microorganism "producing less crotonic acid". That is, term "pool" is not to be strictly understood as the "intracellular pool".

The term "a decreased conversion of acetaldehyde into crotonaldehyde" as used in the present invention means, in general terms, that the expression and/or the activity of a corresponding enzyme described below in the recombinant (genetically modified) organism or microorganism is lower than in the correspondingly non-modified organism or microorganism. In preferred embodiments, in the context of the present invention, a "decreased conversion of acetaldehyde into crotonaldehyde" means that the expression and/or the activity of a corresponding enzyme described below in the genetically modified, recombinant organism or microorganism is at least 5%, 10%, 20%, 30%, 40%, 50%, 60%, 70%, 80% or 90% lower than in the corresponding non-modified organism or microorganism.

The term "a decreased conversion of crotonyl-CoA into crotonaldehyde" as used in the present invention means, in general terms, that the expression and/or the activity of a corresponding enzyme described below in the recombinant (genetically modified) organism or microorganism is lower than in the correspondingly non-modified organism or microorganism. In preferred embodiments, in the context of the present invention, a " decreased conversion of crotonyl-CoA into crotonaldehyde" means that the expression and/or the activity of a corresponding enzyme described below in the genetically modified, recombinant organism or microorganism is at least 5%, 10%, 20%, 30%, 40%, 50%, 60%, 70%, 80% or 90% lower than in the corresponding non-modified organism or microorganism.

The term "a decreased conversion of crotonaldehyde into crotonic acid" as used in the present invention means, in general terms, that the expression and/or the activity of a corresponding enzyme described below in the recombinant (genetically modified) organism or microorganism is lower than in the correspondingly non-modified organism or microorganism. In preferred embodiments, in the context of the present invention, a "decreased conversion of crotonaldehyde into crotonic acid" means that the expression and/or the activity of a corresponding enzyme described below in the genetically modified, recombinant organism or microorganism is at least 5%, 10%, 20%, 30%, 40%, 50%, 60%, 70%, 80% or 90% lower than in the corresponding non-modified organism or microorganism.

Methods and assays for measuring the pool of crotonic acid in a cell have been described in the art (Lie et al., Biosynthesis of butenoic acid through fatty acid biosynthesis pathway in Escherichia coli, Appl Microbiol Biotechnol; DOI 10.1007/s00253-014-6233-2). In brief, crotonic acid can passively diffuse across the cell membrane due to its hydrophobicity. Consequently, the pool of crotonic acid in a cell can be readily determined by culturing said cell in a liquid medium and measuring the concentration of crotonic acid in the supernatant by a suitable method, such as HPLC or GC-MS.

In certain embodiments, the pool of crotonic acid in an organism or microorganism may be determined using HPLC (Ma et al., Simultaneous determination of organic acids and saccharides in lactic acid fermentation broth from biomass using high performance liquid chromatography. Se Pu. 2012 Jan;30(1):62-6. doi: 10.3724/sp.j.1123.2011.09033). For that, the supernatant of a liquid cell culture comprising an organism or microorganism according to the invention may be filtered through a 0.22-µm syringe filter for HPLC analysis. The sample may be measured by HPLC (Agilent 1260 series, Germany) equipped with an Aminex HPX-87H 300 mm x 7.8 mm column (Bio-Rad) and a diode array detector at 210 nm. Analysis may be performed with a mobile phase of 6 mM H₂SO₄ at a flow rate of 0.5 mL/min at 55 °C. The concentrations of crotonic acid may be quantitatively determined with a calibration curve using linear regression. The external standard method may be used to get the regression equations.

Methods and assays for measuring a (decreased) conversion of acetaldehyde into crotonaldehyde over the organism or microorganism from which it is derived are well known to the person skilled in the art. For example, a recombinant (genetically modified) organism or microorganism may be cultured under similar conditions as a correspondingly non-modified organism or microorganism and whole cell lysates may be prepared from both organisms or microorganisms. To determine the conversion of acetaldehyde into crotonaldehyde, the cell lysates may be contacted with acetaldehyde under suitable conditions and the formation of crotonaldehyde may be determined with a suitable analytic method. Suitable methods for detecting and or quantifying the levels of crotonaldehyde have been disclosed by Krutsakorn et al. (In vitro production of n-butanol from glucose. Metab Eng. 2013 Nov;20:84-91).

Methods and assays for measuring a (decreased) conversion of crotonyl-CoA into crotonaldehyde over the organism or microorganism from which it is derived are well known to the person skilled in the art. For example, a recombinant (genetically modified) organism or microorganism may be cultured under similar conditions as a correspondingly non-modified organism or microorganism and whole cell lysates may be prepared from both organisms or microorganisms. To determine the conversion of crotonyl-CoA into crotonaldehyde, the cell lysates may be contacted with crotonyl-CoA under suitable conditions and the formation of crotonaldehyde may be determined with a suitable analytic method. Suitable methods for detecting and or quantifying the levels of crotonaldehyde have been disclosed by Krutsakorn et al. (In vitro production of n-butanol from glucose. Metab Eng. 2013 Nov;20:84-91).

Methods and assays for measuring a (decreased) conversion of crotonaldehyde into crotonic acid over the organism or microorganism from which it is derived are well known to the person skilled in the art. For example, a recombinant (genetically modified) organism or microorganism may be cultured under similar conditions as a correspondingly non-modified organism or microorganism and whole cell lysates may be prepared from both organisms or microorganisms. To determine the conversion of crotonaldehyde into crotonic acid, the cell lysates may be contacted with crotonaldehyde under suitable conditions and the formation of crotonic acid may be determined with a suitable analytic method. Suitable methods for detecting and or quantifying the levels of crotonic acid are disclosed above.

Methods and assays for measuring a (decreased) conversion of acetaldehyde into crotonic acid over the organism or microorganism from which it is derived are well known to the person skilled in the art. For example, a recombinant (genetically modified) organism or microorganism may be cultured under similar conditions as a correspondingly non-modified organism or microorganism and whole cell lysates may be prepared from both organisms or microorganisms. To determine the conversion of acetaldehyde into crotonic acid, the cell lysates may be contacted with acetaldehyde under suitable conditions and the formation of crotonic acid may be determined with a suitable analytic method. Suitable methods for detecting and or quantifying the levels of crotonic acid are disclosed above.

Methods and assays for measuring a (decreased) conversion of crotonyl-CoA into crotonic acid over the organism or microorganism from which it is derived are well known to the person skilled in the art. For example, a recombinant (genetically modified) organism or microorganism may be cultured under similar conditions as a correspondingly non-modified organism or microorganism and whole cell lysates may be prepared from both organisms or microorganisms. To determine the conversion of crotonyl-CoA into crotonic acid, the cell lysates may be contacted with crotonyl-CoA under suitable conditions and the formation of crotonic acid may be determined with a suitable analytic method. Suitable methods for detecting and or quantifying the levels of crotonic acid are disclosed above.

Generally, (i) a decreased conversion of acetaldehyde into crotonaldehyde; and/or (ii) a decreased conversion of crotonyl-CoA into crotonaldehyde; and/or (iii) a decreased conversion of crotonaldehyde into crotonic acid can be achieved by reducing the metabolic flux from acetaldehyde to crotonic acid and/or from crotonyl-CoA to crotonic acid.

A recombinant organism or microorganism having a decreased pool of crotonic acid over the organism or microorganism from which it is derived (while this decreased pool of crotonic acid is due to (i) a decreased conversion of acetaldehyde into crotonaldehyde; and/or (ii) a decreased conversion of crotonyl-CoA into crotonaldehyde; and/or (iii) a decreased conversion of crotonaldehyde into crotonic acid can be achieved by different recombinant modifications which are described in more detail further below.

Generally, "recombinant" in this context denotes the artificial genetic modification of an organism or microorganism, either by addition, removal, or modification of a chromosomal or extra-chromosomal gene or regulatory motif such as a promoter, or by fusion of organisms, or by addition of a vector of any type, for example plasmidic.

Within the present invention, a decreased conversion of acetaldehyde to crotonic acid, preferably via crotonaldehyde, may be achieved by reducing the levels and/or the activity of one or more enzymes that are involved in the conversion of acetaldehyde to crotonic acid. Preferably, a decreased conversion of acetaldehyde to crotonic acid, preferably via crotonaldehyde, may be achieved by reducing the levels and/or the activity of one or more enzymes that are involved in the conversion of acetaldehyde to crotonaldehyde and/or in the conversion of crotonaldehyde to crotonic acid.

Within the present invention, a decreased conversion of crotonyl-CoA to crotonic acid, preferably via crotonaldehyde, may be achieved by reducing the levels and/or the activity of one or more enzymes that are involved in the conversion of crotonyl-CoA to crotonic acid. Preferably, a decreased conversion of crotonyl-CoA to crotonic acid, preferably via crotonaldehyde, may be achieved by reducing the levels and/or the activity of one or more enzymes that are involved in the conversion of crotonyl-CoA to crotonaldehyde and/or in the conversion of crotonaldehyde to crotonic acid.

In a particular embodiment, the invention relates to the recombinant organism or microorganism according to the invention, wherein the reduced level of an enzyme is due to (i) a complete or partial deletion of a gene encoding the respective enzyme in said organism or microorganism; or (ii) a modification in a regulatory element of a gene encoding the respective enzyme in said organism or microorganism.

That is, in certain embodiments, a reduced metabolic flux from acetaldehyde and/or crotonyl-CoA to crotonic acid may be achieved by introducing one or more deletion mutations into the chromosome of an organism or microorganism. Preferably, such deletion mutations result in reduced expression of one or more genes encoding enzymes that are involved in the conversion of acetaldehyde and/or crotonyl-CoA to crotonic acid, preferably via crotonaldehyde.

In certain embodiments, the deletion is a full deletion of a gene encoding an enzyme that is involved in the conversion of acetaldehyde and/or crotonyl-CoA into crotonic acid. A full deletion is a deletion, wherein all codons of a gene are removed from the chromosome of an organism or microorganism. A full deletion of a gene may also include the additional deletion of gene regulatory elements, such as promoters, or even neighbouring genes.

In certain embodiments, the deletion may be a partial deletion of a gene encoding an enzyme that is involved in the conversion of acetaldehyde and/or crotonyl-CoA to crotonic acid. In a partial deletion mutant, only a fragment of a gene is removed from the chromosome of an organism or microorganism. Preferably, the partial deletion results in a non-functional enzyme. Non-functional enzymes can be obtained by deleting parts of the gene that encode essential domains of an enzyme, such as domains comprising the active site, or by introducing a frame shift into the gene.

Apart from modifying the coding sequence of a gene that encodes an enzyme that is involved in the conversion of acetaldehyde and/or crotonyl-CoA to crotonic acid, the expression level of such genes may also be reduced by modifying a regulatory element of said gene. The term "regulatory element," as used herein refers to a genetic element which controls some aspect of the expression of nucleic acid sequences. For example, a promoter is a regulatory element which facilitates the initiation of transcription of an operably linked coding region. Other regulatory elements are ribosome-binding sites, splicing signals, polyadenylation signals, termination signals, etc.

Reducing the level of an enzyme that is involved in the conversion of acetaldehyde and/or crotonyl-CoA to crotonic acid in an organism or microorganism may be achieved by fully or partially deleting the promoter and/or further regulatory elements of a gene encoding said enzyme. Alternatively, or in addition, the expression level of an enzyme that is involved in the conversion of acetaldehyde and/or crotonyl-CoA to crotonic acid may be reduced in an organism or microorganism by introducing point mutations and/or inserting nucleic acid molecules into a regulatory element of a gene encoding an enzyme that is involved in the conversion of acetaldehyde and/or crotonyl-CoA to crotonic acid. For example in bacteria, introducing point mutations into the ribosome binding site can drastically reduce the expression of a downstream nucleic acid.

The skilled person is well aware of methods of molecular engineering that allow deleting chromosomal DNA and/or inserting foreign DNA into the chromosome of an organism or microorganism.

To reduce the metabolic flux from acetaldehyde and/or crotonyl-CoA to crotonic acid, the level of at least one enzyme that is involved in the conversion of acetaldehyde and/or crotonyl-CoA to crotonic acid, preferably via crotonaldehyde, may be reduced. In particular, the expression level of an enzyme that is involved in the conversion of acetaldehyde and/or crotonyl-CoA to crotonic acid, preferably via crotonaldehyde, may be reduced by at least 10%, 20%, 30%, 40%, 50%, 60%, 70%, 80%, 90% or 100%. The skilled person is aware that the level of an enzyme can be reduced by 100%, for example by deleting the entire gene encoding said enzyme. However, in certain embodiments, only a partial reduction of enzyme levels may be desired, for example if a complete removal of an enzyme would result in reduced viability. In such embodiments, it would be preferred to reduce enzyme levels by modifying a regulatory element of a gene encoding said enzyme.

Without being bound to theory, methods and assays for measuring the (level of) expression of a protein include Western Blot, ELISA etc. In another embodiment, the measurement of the (level of) expression is done by measuring the amount of the corresponding RNA. Corresponding methods are well known to the person skilled in the art and include, e.g., Northern Blot or reverse transcription quantitative PCR (RT-qPCR).

In a particular embodiment, the invention relates to the recombinant organism or microorganism according to the invention, wherein the reduced activity of an enzyme is due to (i) an inactivating mutation in a gene encoding the respective enzyme in said organism or microorganism; and/or (ii) the addition of an inhibitor of the respective enzyme.

Instead of reducing the expression level of at least one enzyme that is involved in the conversion of acetaldehyde and/or crotonyl-CoA to crotonic acid, preferably via crotonaldehyde, the activity of the one or more enzyme may also be modified.

That is, the metabolic flux from acetaldehyde and/or crotonyl-CoA to crotonic acid may also be reduced by introducing mutations into a gene encoding an enzyme that is involved in the conversion of acetaldehyde and/or crotonyl-CoA to crotonic acid, preferably via crotonaldehyde. For example, introducing a point mutation or a foreign nucleic acid into a gene encoding an enzyme that is involved in the conversion of acetaldehyde and/or crotonyl-CoA to crotonic acid may result in an inactive gene product or a gene product with reduced activity. In particular, mutations in the active site of an enzyme are likely to result in enzyme variants with reduced or abolished activity.

Alternatively, the activity of an enzyme in an organism or microorganism may be reduced by adding a suitable inhibitor. The inhibitor may be an endogenous inhibitor that is produced by the organism or microorganism itself or an exogenous inhibitor that is added to the organism or microorganism. In certain embodiments, a precursor molecule of an inhibitor may be added to the organism or microorganism, which is then converted into an inhibitor by the organism or microorganism itself. An endogenous inhibitor is said to be "added" to the organism or microorganism, if the organism or microorganism comprises a genetic modification that results in increased production of said inhibitor. In contrast, addition of an exogenous inhibitor to an organism or microorganism may be achieved by adding the inhibitor, or a precursor thereof, to the culture medium.

To reduce the metabolic flux from acetaldehyde and/or crotonyl-CoA to crotonic acid, the activity of at least one enzyme that is involved in the conversion of acetaldehyde and/or crotonyl-CoA to crotonic acid, preferably via crotonaldehyde, may be reduced. In particular, the activity of an enzyme that is involved in the conversion of acetaldehyde and/or crotonyl-CoA to crotonic acid, preferably via crotonaldehyde, may be reduced by at least 10%, 20%, 30%, 40%, 50%, 60%, 70%, 80%, 90% or 100%. The skilled person is aware that the level of an enzyme can be reduced by 100%, for example by replacing one or more essential amino acids in the active site of an enzyme. However, in certain embodiments, only a partial reduction of enzyme activity may be desired, for example if a complete reduction of enzyme activity would result in reduced viability.

Methods and assays for measuring activity of an enzyme are well known to the person skilled in the art. Preferably, the term activity relates to the specific activity of an enzyme. The term "specific activity", as used herein, is defined as the units of activity in a given amount of protein. Thus, the specific activity is not directly measured but is calculated by dividing (1) the activity in units/ml of the enzyme sample by (2) the concentration of protein in that sample, so the specific activity is expressed as units/mg.

To determine the specific activity of an enzyme, or a mutant variant thereof, the enzyme may be recombinantly expressed and purified by methods known in the art. The specific activity of said enzyme or enzyme variant may then be determined with a suitable substrate. Further, the influence of an inhibitor on the specific activity may be determined with purified enzyme.

### Decreasing the conversion of acetaldehyde and/or crotonyl-CoA to crotonic acid

According to the present invention, a decreased pool of crotonic acid can be achieved by decreasing the conversion of acetaldehyde and/or crotonyl-CoA into crotonic acid. Acetaldehyde can be enzymatically converted into crotonic acid as follows:
(i) acetaldehyde is enzymatically converted into crotonaldehyde; and
(ii) said crotonaldehyde is enzymatically converted into crotonic acid.

Crotonyl-CoA can be enzymatically converted into crotonic acid as follows:
(i) crotonyl-CoaA is enzymatically converted into crotonaldehyde; and
(ii) said crotonaldehyde is enzymatically converted into crotonic acid.

In the following, these individual steps are described in more detail.

### The enzymatic conversion of acetaldehyde into crotonaldehyde

The enzymatic conversion of acetaldehyde into crotonaldehyde can be achieved through the condensation of two acetaldehyde molecules. Preferably, the condensation of two acetaldehyde molecules is catalyzed by an aldehyde lyase (EC 4.1.2.-). In a preferred embodiment, the aldehyde lyase catalyzes the following reaction:

2 CH₃CHO → CH₃CH=CHCHO + H₂O

Aldehyde lyases are known from a variety of organisms, including eukaryotic and prokaryotic organisms such as plants, animals, fungi and bacteria. Thus, reducing the expression level or activity of an aldehyde lyase may result in a decreased pool of crotonic acid in a variety of organism or microorganisms.

Within the present invention, the aldehyde lyase (EC 4.1.2.-) may be any aldehyde lyase that catalyzes the condensation of two acetaldehyde molecules. In certain embodiments, the aldehyde lyase is a deoxyribose-phosphate aldolase (EC 4.1.2.4).

A deoxyribose-phosphate aldolase is an enzyme that catalyzes the condensation of acetaldehyde and D-glyceraldehyde 3-phosphate to form 2-deoxy-D-ribose 5-phosphate:

2-deoxy-D-ribose 5-phosphate ↔ D-glyceraldehyde 3-phosphate + acetaldehyde

However, it has been previously reported that the enzyme has a broad substrate specificity and accepts other aldehydes than D-glyceraldehyde 3-phosphate as substrate. For example, it has been reported by Dick et al. (Mechanism-based inhibition of an aldolase at high concentrations of its natural substrate acetaldehyde: structural insights and protective strategies; Chem. Sci., 2016, 7, 4492-4502) that deoxyribose-phosphate aldolases can catalyse the condensation of two acetaldehyde molecules into crotonaldehyde. Accordingly, decreasing the expression level and/or the activity of an aldehyde lyase, such as a deoxyribose-phosphate aldolase, in a recombinant organism or microorganism may result in a decreased pool of crotonaldehyde and, ultimately, crotonic acid.

That is, in certain embodiments, the organism or microorganism of the invention is an organism or microorganism having decreased deoxyribose-phosphate aldolase levels and/or activity. In certain embodiments, the organism or microorganism of the present invention may be a microorganism having decreased deoxyribose-phosphate aldolase levels and/or activity. In certain embodiments, the organism or microorganism of the present invention may be a yeast having decreased deoxyribose-phosphate aldolase levels and/or activity. In certain embodiments, the organism or microorganism of the present invention may be a bacterium having decreased deoxyribose-phosphate aldolase levels and/or activity. In certain embodiments, the organism or microorganism of the present invention may be an *E. coli* cell having decreased deoxyribose-phosphate aldolase levels and/or activity.

In *E. coli,* the deoxyribose-phosphate aldolase is encoded by the gene *deoC.* Thus, in a certain embodiment, the organism or microorganism of the present invention is *E. coli* having decreased levels and/or activity of the gene product of the *deoC* gene. The nucleic acid sequence of the *deoC gene* is provided in SEQ ID NO:1. In certain embodiments, the nucleic acid sequence as set forth in SEQ ID NO:1 may be deleted in the organism or microorganism according to the invention. In certain embodiments, a part of the nucleic acid sequence as set forth in SEQ ID NO:1 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700 nucleotides of the nucleic acid sequence as set forth in SEQ ID NO:1 may be deleted in the organism or microorganism of the invention.

*E. coli deoC* sequence (SEQ ID NO:1):

ATGACTGATCTGAAAGCAAGCAGCCTGCGTGCACTGAAATTGATGGACCTGACCACCCTGAATGACGACG
ACACCGACGAGAAAGTGATCGCCCTGTGTCATCAGGCCAAAACTCCGGTCGGCAATACCGCCGCTATCTG
TATCTATCCTCGCTTTATCCCGATTGCTCGCAAAACTCTGAAAGAGCAGGGCACCCCGGAAATCCGTATC
GCTACGGTAACCAACTTCCCACACGGTAACGACGACATCGACATCGCGCTGGCAGAAACCCGTGCGGCAA
TCGCCTACGGTGCTGATGAAGTTGACGTTGTGTTCCCGTACCGCGCGCTGATGGCGGGTAACGAGCAGGT

TGGTTTTGACCTGGTGAAAGCCTGTAAAGAGGCTTGCGCGGCAGCGAATGTACTGCTGAAAGTGATCATC
GAAACCGGCGAACTGAAAGACGAAGCGCTGATCCGTAAAGCGTCTGAAATCTCCATCAAAGCGGGTGCGG
ACTTCATCAAAACCTCTACCGGTAAAGTGGCTGTGAACGCGACGCCGGAAAGCGCGCGCATCATGATGGA
AGTGATCCGTGATATGGGCGTAGAAAAAACCGTTGGTTTCAAACCGGCGGGCGGCGTGCGTACTGCGGAA
GATGCGCAGAAATATCTCGCCATTGCAGATGAACTGTTCGGTGCTGACTGGGCAGATGCGCGTCACTACC
GCTTTGGCGCTTCCAGCCTGCTGGCAAGCCTGCTGAAAGCGCTGGGTCACGGCGACGGTAAGAGCGCCAG
CAGCTACTAA

As described in more detail above, reducing the level of an aldehyde lyase and, in particular a deoxyribose-phosphate aldolase, in a recombinant organism or microorganism according to the invention may be achieved by fully or partially deleting the coding sequence of a gene encoding said enzyme or by deleting/modifying one or more regulatory elements of a gene encoding said enzyme. Alternatively, the activity of an aldehyde lyase and, in particular a deoxyribose-phosphate aldolase, in an organism or microorganism according to the invention may be decreased by introducing inactivating mutations into the gene encoding said enzyme or through the addition of inhibitors of said enzyme.

For example, the molecules chloral hydrate, hydroxylamine, octanol and propionaldehyde have been reported by Racker et al. (Enzymatic synthesis and breakdown of desoxyribose phosphate, J. Biol. Chem. 1951, 196, 347-365) to inhibit *E. coli* deoxyribose-phosphate aldolase. Jennewein et al. (Directed evolution of an industrial biocatalyst: 2-deoxy-D-ribose 5-phosphate aldolase, Biotechnol. J., 2006, 1, 537-548) further reported chloroacetaldehyde to efficiently inhibit *E. coli* deoxyribose-phosphate aldolase.

Thus, in a certain embodiment, the invention relates to the organism or microorganism according to the invention, wherein the activity of a deoxyribose-phosphate aldolase is decreased due to the addition of the inhibitor chloral hydrate. In a certain embodiment, the invention relates to the organism or microorganism according to the invention, wherein the activity of a deoxyribose-phosphate aldolase is decreased due to the addition of the inhibitor hydroxylamine. In a certain embodiment, the invention relates to the organism or microorganism according to the invention, wherein the activity of a deoxyribose-phosphate aldolase is decreased due to the addition of the inhibitor octanol. In a certain embodiment, the invention relates to the organism or microorganism according to the invention, wherein the activity of a deoxyribose-phosphate aldolase is decreased due to the addition of the inhibitor chloroacetaldehyde.

In a certain embodiment, the invention relates to the organism or microorganism according to the invention, wherein the activity of a deoxyribose-phosphate aldolase is decreased due to the addition of an inhibitor, wherein the inhibitor is selected from the group consisting of: acrolein, chloral hydrate, chloroacetaldehyde, D-glyceraldehyde, hydroxylamine, NaBH₄, octanol, p-hydroxymercuribenzoate, propionaldehyde and Zn²⁺.

### The enzymatic conversion of crotonyl-CoA into crotonaldehyde

The enzymatic conversion of *crotonyl-CoA* into crotonaldehyde can be achieved through the reduction of crotonyl-CoA into crotonaldehyde. Preferably, the reduction of crotonyl-CoA is catalyzed by an NADH or NADPH-dependent aldehyde dehydrogenase (EC 1.2.1.-). In a preferred embodiment, the NADH or NADPH-dependent aldehyde dehydrogenase catalyzes the following reaction:

Crotonyl-CoA + NAD(P)H + H⁺ → crotonaldehyde + NAD(P)⁺ + CoA

NADH or NADPH-dependent aldehyde dehydrogenases are known from a variety of organisms, including eukaryotic and prokaryotic organisms such as plants, animals, fungi and bacteria. Thus, reducing the expression level or activity of an aldehyde dehydrogenase may result in a decreased pool of crotonic acid in a variety of organism or microorganisms.

Within the present invention, the NADH or NADPH-dependent aldehyde dehydrogenase (EC 1.2.1.-) may be any NADH or NADPH-dependent aldehyde dehydrogenase that catalyzes the reduction of crotonyl-CoA into crotonaldehyde. In certain embodiments, the NADH or NADPH-dependent aldehyde dehydrogenase is an acetaldehyde dehydrogenase (acetylating) (EC 1.2.1.10).

That is, in certain embodiments, the recombinant organism or microorganism of the invention is an organism or microorganism having decreased acetaldehyde dehydrogenase (acetylating) levels and/or activity. In certain embodiments, the organism or microorganism of the present invention may be a microorganism having decreased acetaldehyde dehydrogenase (acetylating) levels and/or activity. In certain embodiments, the organism or microorganism of the present invention may be a yeast having decreased acetaldehyde dehydrogenase (acetylating) levels and/or activity. In certain embodiments, the organism or microorganism of the present invention may be a bacterium having decreased acetaldehyde dehydrogenase (acetylating) levels and/or activity. In certain embodiments, the organism or microorganism of the present invention may be an *E. coli cell* having decreased acetaldehyde dehydrogenase (acetylating) levels and/or activity.

In *E. coli,* acetaldehyde dehydrogenases (acetylating) are encoded by the genes *mhpF, adhE, eutE.* Thus, in a certain embodiment, the organism or microorganism of the present invention is *E. coli* having decreased levels and/or activity of the gene product(s) of the *mhpF,* and/or *adhE* and/or *eutE* gene(s).

The nucleic acid sequence of the *mhpF* gene is provided in SEQ ID NO:2. In certain embodiments, the nucleic acid sequence as set forth in SEQ ID NO:2 may be deleted in the organism or microorganism according to the invention. In certain embodiments, a part of the nucleic acid sequence as set forth in SEQ ID NO:2 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900 nucleotides of the nucleic acid sequence as set forth in SEQ ID NO:2 may be deleted in the organism or microorganism of the invention.

*E. coli mhpF* sequence (SEQ ID NO:2):

ATGAGTAAGCGTAAAGTCGCCATTATCGGTTCTGGCAACATTGGTACCGATCTGATGATTAAAATTTTGC
GTCACGGTCAGCATCTGGAGATGGCGGTGATGGTTGGCATTGATCCTCAGTCCGACGGTCTGGCGCGCGC
CAGACGTATGGGCGTCGCCACCACCCATGAAGGGGTGATCGGACTGATGAACATGCCTGAATTTGCTGAT

ATCGACATTGTATTTGATGCGACCAGCGCCGGTGCTCATGTGAAAAACGATGCCGCTTTACGCGAAGCGA
AACCGGATATTCGCTTAATTGACCTGACGCCTGCTGCCATCGGCCCTTACTGCGTGCCGGTGGTTAACCT
CGAGGCGAACGTCGATCAACTGAACGTCAACATGGTCACCTGCGGCGGCCAGGCCACCATTCCAATGGTG
GCGGCAGTTTCACGCGTGGCGCGTGTTCATTACGCCGAAATTATCGCTTCTATCGCCAGTAAATCTGCCG
GACCTGGCACGCGTGCCAATATCGATGAATTTACGGAAACCACTTCCCGAGCCATTGAAGTGGTGGGCGG
CGCGGCAAAAGGGAAGGCGATTATTGTGCTTAACCCAGCAGAGCCACCGTTGATGATGCGTGACACGGTG
TATGTATTGAGCGACGAAGCTTCACAAGATGATATCGAAGCCTCAATCAATGAAATGGCTGAGGCGGTGC
AGGCTTACGTACCGGGTTATCGCCTGAAACAGCGCGTGCAGTTTGAAGTTATCCCGCAGGATAAACCGGT
CAATTTACCGGGCGTGGGGCAATTCTCCGGACTGAAAACAGCGGTCTGGCTGGAAGTCGAAGGCGCAGCG
CATTATCTGCCTGCCTATGCGGGCAACCTCGACATTATGACTTCCAGTGCGCTGGCGACAGCGGAAAAAA
TGGCCCAGTCACTGGCGCGCAAGGCAGGAGAAGCGGCATGA

The nucleic acid sequence of the *adhE* gene is provided in SEQ ID NO:3. In certain embodiments, the nucleic acid sequence as set forth in SEQ ID NO:3 may be deleted in the organism or microorganism according to the invention. In certain embodiments, a part of the nucleic acid sequence as set forth in SEQ ID NO:3 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1250, 1500, 1750, 2000 nucleotides of the nucleic acid sequence as set forth in SEQ ID NO:3 may be deleted in the organism or microorganism of the invention.

*E. coli adhE* sequence (SEQ ID NO:3):

ATGGCTGTTACTAATGTCGCTGAACTTAACGCACTCGTAGAGCGTGTAAAAAAAGCCCAGCGTGAATATG
CCAGTTTCACTCAAGAGCAAGTAGACAAAATCTTCCGCGCCGCCGCTCTGGCTGCTGCAGATGCTCGAAT
CCCACTCGCGAAAATGGCCGTTGCCGAATCCGGCATGGGTATCGTCGAAGATAAAGTGATCAAAAACCAC
TTTGCTTCTGAATATATCTACAACGCCTATAAAGATGAAAAAACCTGTGGTGTTCTGTCTGAAGACGACA
CTTTTGGTACCATCACTATCGCTGAACCAATCGGTATTATTTGCGGTATCGTTCCGACCACTAACCCGAC
TTCAACTGCTATCTTCAAATCGCTGATCAGTCTGAAGACCCGTAACGCCATTATCTTCTCCCCGCACCCG
CGTGCAAAAGATGCCACCAACAAAGCGGCTGATATCGTTCTGCAGGCTGCTATCGCTGCCGGTGCTCCGA
AAGATCTGATCGGCTGGATCGATCAACCTTCTGTTGAACTGTCTAACGCACTGATGCACCACCCAGACAT
CAACCTGATCCTCGCGACTGGTGGTCCGGGCATGGTTAAAGCCGCATACAGCTCCGGTAAACCAGCTATC
GGTGTAGGCGCGGGCAACACTCCAGTTGTTATCGATGAAACTGCTGATATCAAACGTGCAGTTGCATCTG
TACTGATGTCCAAAACCTTCGACAACGGCGTAATCTGTGCTTCTGAACAGTCTGTTGTTGTTGTTGACTC
TGTTTATGACGCTGTACGTGAACGTTTTGCAACCCACGGCGGCTATCTGTTGCAGGGTAAAGAGCTGAAA
GCTGTTCAGGATGTTATCCTGAAAAACGGTGCGCTGAACGCGGCTATCGTTGGTCAGCCAGCCTATAAAA

TTGCTGAACTGGCAGGCTTCTCTGTACCAGAAAACACCAAGATTCTGATCGGTGAAGTGACCGTTGTTGA
TGAAAGCGAACCGTTCGCACATGAAAAACTGTCCCCGACTCTGGCAATGTACCGCGCTAAAGATTTCGAA
GACGCGGTAGAAAAAGCAGAGAAACTGGTTGCTATGGGCGGTATCGGTCATACCTCTTGCCTGTACACTG
ACCAGGATAACCAACCGGCTCGCGTTTCTTACTTCGGTCAGAAAATGAAAACGGCGCGTATCCTGATTAA
CACCCCAGCGTCTCAGGGTGGTATCGGTGACCTGTATAACTTCAAACTCGCACCTTCCCTGACTCTGGGT
TGTGGTTCTTGGGGTGGTAACTCCATCTCTGAAAACGTTGGTCCGAAACACCTGATCAACAAGAAAACCG
TTGCTAAGCGAGCTGAAAACATGTTGTGGCACAAACTTCCGAAATCTATCTACTTCCGCCGTGGCTCCCT
GCCAATCGCGCTGGATGAAGTGATTACTGATGGCCACAAACGTGCGCTCATCGTGACTGACCGCTTCCTG
TTCAACAATGGTTATGCTGATCAGATCACTTCCGTACTGAAAGCAGCAGGCGTTGAAACTGAAGTCTTCT
TCGAAGTAGAAGCGGACCCGACCCTGAGCATCGTTCGTAAAGGTGCAGAACTGGCAAACTCCTTCAAACC
AGACGTGATTATCGCGCTGGGTGGTGGTTCCCCGATGGACGCCGCGAAGATCATGTGGGTTATGTACGAA
CATCCGGAAACTCACTTCGAAGAGCTGGCGCTGCGCTTTATGGATATCCGTAAACGTATCTACAAGTTCC
CGAAAATGGGCGTGAAAGCGAAAATGATCGCTGTCACCACCACTTCTGGTACAGGTTCTGAAGTCACTCC
GTTTGCGGTTGTAACTGACGACGCTACTGGTCAGAAATATCCGCTGGCAGACTATGCGCTGACTCCGGAT
ATGGCGATTGTCGACGCCAACCTGGTTATGGACATGCCGAAGTCCCTGTGTGCTTTCGGTGGTCTGGACG
CAGTAACTCACGCCATGGAAGCTTATGTTTCTGTACTGGCATCTGAGTTCTCTGATGGTCAGGCTCTGCA
GGCACTGAAACTGCTGAAAGAATATCTGCCAGCGTCCTACCACGAAGGGTCTAAAAATCCGGTAGCGCGT
GAACGTGTTCACAGTGCAGCGACTATCGCGGGTATCGCGTTTGCGAACGCCTTCCTGGGTGTATGTCACT
CAATGGCGCACAAACTGGGTTCCCAGTTCCATATTCCGCACGGTCTGGCAAACGCCCTGCTGATTTGTAA
CGTTATTCGCTACAATGCGAACGACAACCCGACCAAGCAGACTGCATTCAGCCAGTATGACCGTCCGCAG
GCTCGCCGTCGTTATGCTGAAATTGCCGACCACTTGGGTCTGAGCGCACCGGGCGACCGTACTGCTGCTA
AGATCGAGAAACTGCTGGCATGGCTGGAAACGCTGAAAGCTGAACTGGGTATTCCGAAATCTATCCGTGA
AGCTGGCGTTCAGGAAGCAGACTTCCTGGCGAACGTGGATAAACTGTCTGAAGATGCATTCGATGACCAG
TGCACCGGCGCTAACCCGCGTTACCCGCTGATCTCCGAGCTGAAACAGATTCTGCTGGATACCTACTACG
GTCGTGATTATGTAGAAGGTGAAACTGCAGCGAAGAAAGAAGCTGCTCCGGCTAAAGCTGAGAAAAAAGC
GAAAAAATCCGCTTAA

The nucleic acid sequence of the *eutE* gene is provided in SEQ ID NO:4. In certain embodiments, the nucleic acid sequence as set forth in SEQ ID NO:4 may be deleted in the organism or microorganism according to the invention. In certain embodiments, a part of the nucleic acid sequence as set forth in SEQ ID NO:4 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300, 1400 nucleotides of the nucleic acid sequence as set forth in SEQ ID NO:4 may be deleted in the organism or microorganism of the invention.

*E. coli eutE* sequence (SEQ ID NO:4):

ATGAATCAACAGGATATTGAACAGGTGGTGAAAGCGGTACTGCTGAAAATGCAAAGCAGTGACACGCCGT
CCGCCGCCGTTCATGAGATGGGCGTTTTCGCGTCCCTGGATGACGCCGTTGCGGCAGCCAAAGTCGCCCA
GCAAGGGTTAAAAAGCGTGGCAATGCGCCAGTTAGCCATTGCTGCCATTCGTGAAGCAGGCGAAAAACAC
GCCAGAGATTTAGCGGAACTTGCCGTCAGTGAAACCGGCATGGGGCGCGTTGAAGATAAATTTGCAAAAA
ACGTCGCTCAGGCGCGCGGCACACCAGGCGTTGAGTGCCTCTCTCCGCAAGTGCTGACTGGCGACAACGG
CCTGACCCTAATTGAAAACGCACCCTGGGGCGTGGTGGCTTCGGTGACGCCTTCCACTAACCCGGCGGCA
ACCGTAATTAACAACGCCATCAGCCTGATTGCCGCGGGCAACAGCGTCATTTTTGCCCCGCATCCGGCGG
CGAAAAAAGTCTCCCAGCGGGCGATTACGCTGCTCAACCAGGCGATTGTTGCCGCAGGTGGGCCGGAAAA
CTTACTGGTTACTGTGGCAAATCCGGATATCGAAACCGCGCAACGCTTGTTCAAGTTTCCGGGTATCGGC
CTGCTGGTGGTAACCGGCGGCGAAGCGGTAGTAGAAGCGGCGCGTAAACACACCAATAAACGTCTGATTG
CCGCAGGCGCTGGCAACCCGCCGGTAGTGGTGGATGAAACCGCCGACCTCGCCCGTGCCGCTCAGTCCAT
CGTCAAAGGCGCTTCTTTCGATAACAACATCATTTGTGCCGACGAAAAGGTACTGATTGTTGTTGATAGC
GTAGCCGATGAACTGATGCGTCTGATGGAAGGCCAGCACGCGGTGAAACTGACCGCAGAACAGGCGCAGC
AGCTGCAACCGGTGTTGCTGAAAAATATCGACGAGCGCGGAAAAGGCACCGTCAGCCGTGACTGGGTTGG
TCGCGACGCAGGCAAAATCGCGGCGGCAATCGGCCTTAAAGTTCCGCAAGAAACGCGCCTGCTGTTTGTG
GAAACCACCGCAGAACATCCGTTTGCCGTGACTGAACTGATGATGCCGGTGTTGCCCGTCGTGCGCGTCG
CCAACGTGGCGGATGCCATTGCGCTAGCGGTGAAACTGGAAGGCGGTTGCCACCACACGGCGGCAATGCA
CTCGCGCAACATCGAAAACATGAACCAGATGGCGAATGCTATTGATACCAGCATTTTCGTTAAGAACGGA
CCGTGCATTGCCGGGCTGGGGCTGGGCGGGGAAGGCTGGACCACCATGACCATCACCACGCCAACCGGTG
AAGGGGTAACCAGCGCGCGTACGTTTGTCCGTCTGCGTCGCTGTGTATTAGTCGATGCGTTTCGCATTGT
TTAA

It is important to note that the recombinant organism or microorganism may also be characterized by reduced levels and or activities of two or more aldehyde dehydrogenases and, in particular, two or more of the acetaldehyde dehydrogenases (acetylating) (EC 1.2.1.10) listed above.

As described in more detail above, reducing the level of an aldehyde dehydrogenase and, in particular an acetaldehyde dehydrogenase (acetylating), in a recombinant organism or microorganism according to the invention may be achieved by fully or partially deleting the coding sequence of a gene encoding said enzyme or by deleting/modifying one or more regulatory elements of a gene encoding said enzyme. Alternatively, the activity of an aldehyde dehydrogenase and, in particular an acetaldehyde dehydrogenase (acetylating), in an organism or microorganism according to the invention may be decreased by introducing inactivating mutations into the gene encoding said enzyme or through the addition of inhibitors of said enzyme.

In a certain embodiment, the invention relates to the organism or microorganism according to the invention, wherein the activity of an acetaldehyde dehydrogenase (acetylating) is decreased due to the addition of an inhibitor, wherein the inhibitor is selected from the group consisting of: AgNO₃, benzaldehyde, Ca²⁺, chloroethanol, CuSO₄, disulfiram, HgCl₂, iodoacetamide, iodoacetate, Mg²⁺, Mn²⁺, Na₂HAsO₄, p-chloromercuribenzoate, Tris and valporate.

### The enzymatic conversion of crotonaldehyde into crotonic acid

The enzymatic conversion of crotonaldehyde into crotonic acid can be achieved by oxidizing crotonaldehyde. Preferably, the oxidation of crotonaldehyde is catalyzed by an aldehyde dehydrogenase (EC 1.2.-) with the addition of water.

Aldehyde dehydrogenases are known from a variety of organisms, including eukaryotic and prokaryotic organisms such as plants, animals, fungi and bacteria. Thus, reducing the level or activity of an aldehyde dehydrogenase may result in a decreased pool of crotonic acid in a variety of organism or microorganisms.

Within the present invention, the aldehyde dehydrogenase (EC 1.2.-) may be any aldehyde dehydrogenase that catalyzes the oxidation of crotonaldehyde into crotonic acid. Adehyde dehydrogenases using various cofactors have been described in the art. Within the present invention, the aldehyde dehydrogenase may be, without limitation, an NAD⁺ or NADP⁺-dependent aldehyde dehydrogenase (EC 1.2.1), an iron-sulfur protein-dependent aldehyde dehydrogenase (EC 1.2.7) or a FAD-independent aldehyde dehydrogenase (EC 1.2.99). That is, different cofactors may be used by aldehyde dehydrogenases when oxidizing crotonaldehyde to crotonic acid.

Aldehyde dehydrogenases have been reported to be rather unspecific enzymes and to accept a variety of aldehydes. Accordingly, decreasing the level and/or the activity of one or more aldehyde dehydrogenases in a recombinant organism or microorganism may result in a decreased pool of crotonic acid.

That is, in certain embodiments, the organism or microorganism of the invention is an organism or microorganism having decreased aldehyde dehydrogenases levels and/or activity. In certain embodiments, the organism or microorganism of the present invention may be a microorganism having decreased aldehyde dehydrogenases levels and/or activity. In certain embodiments, the organism or microorganism of the present invention may be a yeast having decreased aldehyde dehydrogenases levels and/or activity. In certain embodiments, the organism or microorganism of the present invention may be a bacterium decreased aldehyde dehydrogenases levels and/or activity. In certain embodiments, the organism or microorganism of the present invention may be an *E. coli* cell decreased aldehyde dehydrogenases levels and/or activity.

In certain embodiments, the aldehyde dehydrogenase is an NAD⁺ or NADP⁺-dependent aldehyde dehydrogenase (EC 1.2.1), catalysing the following reaction:

Aldehyde + NAD(P)⁺ + H₂O → carboxylate + 2 H⁺ + NAD(P)H

In *E. coli,* an NAD⁺ or NADP⁺-dependent aldehyde dehydrogenase is encoded by the gene *puuC* (EC 1.2.1.19 and 1.2.1.24 and 1.2.1.99). Thus, in a certain embodiment, the organism or microorganism of the present invention is *E. coli* having decreased levels and/or activity of the gene product of the *puuC* gene. The nucleic acid sequence of the *puuC* gene is provided in SEQ ID NO:5. In certain embodiments, the nucleic acid sequence as set forth in SEQ ID NO:5 may be deleted in the organism or microorganism according to the invention. In certain embodiments, a part of the nucleic acid sequence as set forth in SEQ ID NO:5 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:5 may be deleted in the organism or microorganism of the invention.

*E. coli puuC* sequence (SEQ ID NO:5):

ATGAATTTTCATCATCTGGCTTACTGGCAGGATAAAGCGTTAAGTCTCGCCATTGAAAACCGCTTATTTA
TTAACGGTGAATATACTGCTGCGGCGGAAAATGAAACCTTTGAAACCGTTGATCCGGTCACCCAGGCACC
GCTGGCGAAAATTGCCCGCGGCAAGAGCGTCGATATCGACCGTGCGATGAGCGCAGCACGCGGCGTATTT
GAACGCGGCGACTGGTCACTCTCTTCTCCGGCTAAACGTAAAGCGGTACTGAATAAACTCGCCGATTTAA
TGGAAGCCCACGCCGAAGAGCTGGCACTGCTGGAAACTCTCGACACCGGCAAACCGATTCGTCACAGTCT
GCGTGATGATATTCCCGGCGCGGCGCGCGCCATTCGCTGGTACGCCGAAGCGATCGACAAAGTGTATGGC
GAAGTGGCGACCACCAGTAGCCATGAGCTGGCGATGATCGTGCGTGAACCGGTCGGCGTGATTGCCGCCA
TCGTGCCGTGGAACTTCCCGCTGTTGCTGACTTGCTGGAAACTCGGCCCGGCGCTGGCGGCGGGAAACAG
CGTGATTCTAAAACCGTCTGAAAAATCACCGCTCAGTGCGATTCGTCTCGCGGGGCTGGCGAAAGAAGCA
GGCTTGCCGGATGGTGTGTTGAACGTGGTGACGGGTTTTGGTCATGAAGCCGGGCAGGCGCTGTCGCGTC
ATAACGATATCGACGCCATTGCCTTTACCGGTTCAACCCGTACCGGGAAACAGCTGCTGAAAGATGCGGG
CGACAGCAACATGAAACGCGTCTGGCTGGAAGCGGGCGGCAAAAGCGCCAACATCGTTTTCGCTGACTGC
CCGGATTTGCAACAGGCGGCAAGCGCCACCGCAGCAGGCATTTTCTACAACCAGGGACAGGTGTGCATCG
CCGGAACGCGCCTGTTGCTGGAAGAGAGCATCGCCGATGAATTCTTAGCCCTGTTAAAACAGCAGGCGCA
AAACTGGCAGCCGGGCCATCCACTTGATCCCGCAACCACCATGGGCACCTTAATCGACTGCGCCCACGCC
GACTCGGTCCATAGCTTTATTCGGGAAGGCGAAAGCAAAGGGCAACTGTTGTTGGATGGCCGTAACGCCG
GGCTGGCTGCCGCCATCGGCCCGACCATCTTTGTGGATGTGGACCCGAATGCGTCCTTAAGTCGCGAAGA
GATTTTCGGTCCGGTGCTGGTGGTCACGCGTTTCACATCAGAAGAACAGGCGCTACAGCTTGCCAACGAC
AGCCAGTACGGCCTTGGCGCGGCGGTATGGACGCGCGACCTCTCCCGCGCGCACCGCATGAGCCGACGCC
TGAAAGCCGGTTCCGTCTTCGTCAATAACTACAACGACGGCGATATGACCGTGCCGTTTGGCGGCTATAA
GCAGAGCGGCAACGGTCGCGACAAATCCCTGCATGCCCTTGAAAAATTCACTGAACTGAAAACCATCTGG
ATAAGCCTGGAGGCCTGA

Another NAD⁺ or NADP⁺-dependent aldehyde dehydrogenase in *E. coli* is encoded by the gene *feaB* (EC 1.2.1.39). Thus, in a certain embodiment, the organism or microorganism of the present invention is *E. coli* having decreased levels and/or activity of the gene product of the *feaB* gene. The nucleic acid sequence of the *feaB* gene is provided in SEQ ID NO:6. In certain embodiments, the nucleic acid sequence as set forth in SEQ ID NO:6 may be deleted in the organism or microorganism according to the invention. In certain embodiments, a part of the nucleic acid sequence as set forth in SEQ ID NO:6 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:6 may be deleted in the organism or microorganism of the invention.

*E. coli feaB* sequence (SEQ ID NO:6):

ATGACAGAGCCGCATGTAGCAGTATTAAGCCAGGTCCAACAGTTTCTCGATCGTCAACACGGTCTTTATA
TTGATGGTCGTCCTGGCCCCGCACAAAGTGAAAAACGGTTGGCGATCTTTGATCCGGCCACCGGGCAAGA
AATTGCGTCTACTGCTGATGCCAACGAAGCGGATGTAGATAACGCAGTCATGTCTGCCTGGCGGGCCTTT
GTCTCGCGTCGCTGGGCCGGGCGATTACCCGCAGAGCGTGAACGTATTCTGCTACGTTTTGCTGATCTGG
TGGAGCAGCACAGTGAGGAGCTGGCGCAACTGGAAACCCTGGAGCAAGGCAAGTCAATTGCCATTTCCCG
TGCTTTTGAAGTGGGCTGTACGCTGAACTGGATGCGTTATACCGCCGGGTTAACGACCAAAATCGCGGGT
AAAACGCTGGACTTGTCGATTCCCTTACCCCAGGGGGCGCGTTATCAGGCCTGGACGCGTAAAGAGCCGG
TTGGCGTAGTGGCGGGAATTGTGCCATGGAACTTTCCGTTGATGATTGGTATGTGGAAGGTGATGCCAGC
ACTGGCAGCAGGCTGTTCAATCGTGATTAAGCCTTCGGAAACCACGCCACTGACGATGTTGCGCGTGGCG
GAACTGGCCAGCGAGGCTGGTATCCCTGATGGCGTTTTTAATGTCGTCACCGGGTCAGGTGCTGTATGCG
GCGCGGCCCTGACGTCACATCCTCATGTTGCGAAAATCAGTTTTACCGGTTCAACCGCGACGGGAAAAGG
TATTGCCAGAACTGCTGCTGATCACTTAACGCGTGTAACGCTGGAACTGGGCGGTAAAAACCCGGCAATT
GTATTAAAAGATGCTGATCCGCAATGGGTTATTGAAGGCTTGATGACCGGAAGCTTCCTGAATCAAGGGC
AAGTATGCGCCGCCAGTTCGCGAATTTATATTGAAGCGCCGTTGTTTGACACGCTGGTTAGTGGATTTGA
GCAGGCGGTAAAATCGTTGCAAGTGGGACCGGGGATGTCACCTGTTGCACAGATTAACCCTTTGGTTTCT
CGTGCGCACTGCGACAAAGTGTGTTCATTCCTCGACGATGCGCAGGCACAGCAAGCAGAGCTGATTCGCG
GGTCGAATGGACCAGCCGGAGAGGGGTATTATGTTGCGCCAACGCTGGTGGTAAATCCCGATGCTAAATT
GCGCTTAACTCGTGAAGAGGTGTTTGGTCCGGTGGTAAACCTGGTGCGAGTAGCGGATGGAGAAGAGGCG
TTACAACTGGCAAACGACACGGAATATGGCTTAACTGCCAGTGTCTGGACGCAAAATCTCTCCCAGGCTC
TGGAATATAGCGATCGCTTACAGGCAGGGACGGTGTGGGTAAACAGCCATACCTTAATTGACGCTAACTT
ACCGTTTGGTGGGATGAAGCAGTCAGGAACGGGCCGTGATTTTGGCCCCGACTGGCTGGACGGTTGGTGT
GAAACTAAGTCGGTGTGTGTACGGTATTAA

Another NAD⁺ or NADP⁺-dependent aldehyde dehydrogenase in *E. coli* is encoded by the gene *patD* (EC 1.2.1.19). Thus, in a certain embodiment, the organism or microorganism of the present invention is *E. coli* having decreased levels and/or activity of the gene product of the *patD* gene. The nucleic acid sequence of the *patD* gene is provided in SEQ ID NO:7. In certain embodiments, the nucleic acid sequence as set forth in SEQ ID NO:7 may be deleted in the organism or microorganism according to the invention. In certain embodiments, a part of the nucleic acid sequence as set forth in SEQ ID NO:7 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:7 may be deleted in the organism or microorganism of the invention.

*E. coli patD* sequence (SEQ ID NO:7):

ATGCAACATAAGTTACTGATTAACGGAGAACTGGTTAGCGGCGAAGGGGAAAAACAGCCTGTCTATAATC
CGGCAACGGGGGACGTTTTACTGGAAATTGCCGAGGCATCCGCAGAGCAGGTCGATGCTGCTGTGCGCGC
GGCAGATGCAGCATTTGCCGAATGGGGGCAAACCACGCCGAAAGTGCGTGCGGAATGTCTGCTGAAACTG
GCTGATGTTATCGAAGAAAATGGTCAGGTTTTTGCCGAACTGGAGTCCCGTAATTGTGGCAAACCGCTGC
ATAGTGCGTTCAATGATGAAATCCCGGCGATTGTCGATGTTTTTCGCTTTTTCGCGGGTGCGGCGCGCTG
TCTGAATGGTCTGGCGGCAGGTGAATATCTTGAAGGTCATACTTCGATGATCCGTCGCGATCCGTTGGGG
GTCGTGGCTTCTATCGCACCGTGGAATTATCCGCTGATGATGGCCGCGTGGAAACTTGCTCCGGCGCTGG
CGGCAGGGAACTGCGTAGTGCTTAAACCATCAGAAATTACCCCGCTGACCGCGTTGAAGTTGGCAGAGCT
GGCGAAAGATATCTTCCCGGCAGGCGTGATTAACATACTGTTTGGCAGAGGCAAAACGGTGGGTGATCCG
CTGACCGGTCATCCCAAAGTGCGGATGGTGTCGCTGACGGGCTCTATCGCCACCGGCGAGCACATCATCA
GCCATACCGCGTCGTCCATTAAGCGTACTCATATGGAACTTGGTGGCAAAGCGCCAGTGATTGTTTTTGA
TGATGCGGATATTGAAGCAGTGGTCGAAGGTGTACGTACATTTGGCTATTACAATGCTGGACAGGATTGT
ACTGCGGCTTGTCGGATCTACGCGCAAAAAGGCATTTACGATACGCTGGTGGAAAAACTGGGTGCTGCGG
TGGCAACGTTAAAATCTGGTGCGCCAGATGACGAGTCTACGGAGCTTGGACCTTTAAGCTCGCTGGCGCA
TCTCGAACGCGTCGGCAAGGCAGTAGAAGAGGCGAAAGCGACAGGGCACATCAAAGTGATCACTGGCGGT
GAAAAGCGCAAGGGTAATGGCTATTACTATGCGCCGACGCTGCTGGCTGGCGCATTACAGGACGATGCCA
TCGTGCAAAAAGAGGTATTTGGTCCAGTAGTGAGTGTTACGCCCTTCGACAACGAAGAACAGGTGGTGAA
CTGGGCGAATGACAGCCAGTACGGACTTGCATCTTCGGTATGGACGAAAGATGTGGGCAGGGCGCATCGC
GTCAGCGCACGGCTGCAATATGGTTGTACCTGGGTCAATACCCATTTCATGCTGGTAAGTGAAATGCCGC
ACGGTGGGCAGAAACTTTCTGGTTACGGCAAGGATATGTCACTTTATGGGCTGGAGGATTACACCGTCGT
CCGCCACGTCATGGTTAAACATTAA

Another NAD⁺ or NADP⁺-dependent aldehyde dehydrogenase in *E. coli* is encoded by the gene *aldA* (EC 1.2.1.21 and 1.2.1.22). Thus, in a certain embodiment, the organism or microorganism of the present invention is *E. coli* having decreased levels and/or activity of the gene product of the *aldA* gene. The nucleic acid sequence of the *aldA* gene is provided in SEQ ID NO:8. In certain embodiments, the nucleic acid sequence as set forth in SEQ ID NO:8 may be deleted in the organism or microorganism according to the invention. In certain embodiments, a part of the nucleic acid sequence as set forth in SEQ ID NO:8 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:8 may be deleted in the organism or microorganism of the invention.

*E. coli aldA* sequence (SEQ ID NO:8):

ATGTCAGTACCCGTTCAACATCCTATGTATATCGATGGACAGTTTGTTACCTGGCGTGGAGACGCATGGA
TTGATGTGGTAAACCCTGCTACAGAGGCTGTCATTTCCCGCATACCCGATGGTCAGGCCGAGGATGCCCG
TAAGGCAATCGATGCAGCAGAACGTGCACAACCAGAATGGGAAGCGTTGCCTGCTATTGAACGCGCCAGT
TGGTTGCGCAAAATCTCCGCCGGGATCCGCGAACGCGCCAGTGAAATCAGTGCGCTGATTGTTGAAGAAG
GGGGCAAGATCCAGCAGCTGGCTGAAGTCGAAGTGGCTTTTACTGCCGACTATATCGATTACATGGCGGA
GTGGGCACGGCGTTACGAGGGCGAGATTATTCAAAGCGATCGTCCAGGAGAAAATATTCTTTTGTTTAAA
CGTGCGCTTGGTGTGACTACCGGCATTCTGCCGTGGAACTTCCCGTTCTTCCTCATTGCCCGCAAAATGG
CTCCCGCTCTTTTGACCGGTAATACCATCGTCATTAAACCTAGTGAATTTACGCCAAACAATGCGATTGC
ATTCGCCAAAATCGTCGATGAAATAGGCCTTCCGCGCGGCGTGTTTAACCTTGTACTGGGGCGTGGTGAA
ACCGTTGGGCAAGAACTGGCGGGTAACCCAAAGGTCGCAATGGTCAGTATGACAGGCAGCGTCTCTGCAG
GTGAGAAGATCATGGCGACTGCGGCGAAAAACATCACCAAAGTGTGTCTGGAATTGGGGGGTAAAGCACC
AGCTATCGTAATGGACGATGCCGATCTTGAACTGGCAGTCAAAGCCATCGTTGATTCACGCGTCATTAAT
AGTGGGCAAGTGTGTAACTGTGCAGAACGTGTTTATGTACAGAAAGGCATTTATGATCAGTTCGTCAATC
GGCTGGGTGAAGCGATGCAGGCGGTTCAATTTGGTAACCCCGCTGAACGCAACGACATTGCGATGGGGCC
GTTGATTAACGCCGCGGCGCTGGAAAGGGTCGAGCAAAAAGTGGCGCGCGCAGTAGAAGAAGGGGCGAGA
GTGGCGTTCGGTGGCAAAGCGGTAGAGGGGAAAGGATATTATTATCCGCCGACATTGCTGCTGGATGTTC
GCCAGGAAATGTCGATTATGCATGAGGAAACCTTTGGCCCGGTGCTGCCAGTTGTCGCATTTGACACGCT
GGAAGATGCTATCTCAATGGCTAATGACAGTGATTACGGCCTGACCTCATCAATCTATACCCAAAATCTG
AACGTCGCGATGAAAGCCATTAAAGGGCTGAAGTTTGGTGAAACTTACATCAACCGTGAAAACTTCGAAG
CTATGCAAGGCTTCCACGCCGGATGGCGTAAATCCGGTATTGGCGGCGCAGATGGTAAACATGGCTTGCA
TGAATATCTGCAGACCCAGGTGGTTTATTTACAGTCTTAA

Another NAD⁺ or NADP⁺-dependent aldehyde dehydrogenase in *E. coli* is encoded by the gene *sad* (EC 1.2.1.24). Thus, in a certain embodiment, the organism or microorganism of the present invention is *E. coli* having decreased levels and/or activity of the gene product of the sad gene. The nucleic acid sequence of the sad gene is provided in SEQ ID NO:9. In certain embodiments, the nucleic acid sequence as set forth in SEQ ID NO:9 may be deleted in the organism or microorganism according to the invention. In certain embodiments, a part of the nucleic acid sequence as set forth in SEQ ID NO:9 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 nucleotides of the nucleic acid sequence as set forth in SEQ ID NO:9 may be deleted in the organism or microorganism of the invention.

*E. coli sad* sequence (SEQ ID NO:9):

ATGACCATTACTCCGGCAACTCATGCAATTTCGATAAATCCTGCCACGGGTGAACAACTTTCTGTGCTGC
CGTGGGCTGGCGCTGACGATATCGAAAACGCACTTCAGCTGGCGGCAGCAGGCTTTCGCGACTGGCGCGA
GACAAATATAGATTATCGTGCTGAAAAACTGCGTGATATCGGTAAGGCTCTGCGCGCTCGTAGCGAAGAA
ATGGCGCAAATGATCACCCGCGAAATGGGCAAACCAATCAACCAGGCGCGCGCTGAAGTGGCGAAATCGG
CGAATTTGTGTGACTGGTATGCAGAACATGGTCCGGCAATGCTGAAGGCGGAACCTACGCTGGTGGAAAA
TCAGCAGGCGGTTATTGAGTATCGACCGTTGGGGACGATTCTGGCGATTATGCCGTGGAATTTTCCGTTA
TGGCAGGTGATGCGTGGCGCTGTTCCCATCATTCTTGCAGGTAACGGCTACTTACTTAAACATGCGCCGA
ATGTGATGGGCTGTGCACAGCTCATTGCCCAGGTGTTTAAAGATGCGGGTATCCCACAAGGCGTATATGG
CTGGCTGAATGCCGACAACGACGGTGTCAGTCAGATGATTAAAGACTCGCGCATTGCTGCTGTCACGGTG
ACCGGAAGTGTTCGTGCGGGAGCGGCTATTGGCGCACAGGCTGGAGCGGCACTGAAAAAATGCGTACTGG
AACTGGGCGGTTCGGATCCGTTTATTGTGCTTAACGATGCCGATCTGGAACTGGCGGTGAAAGCGGCGGT
AGCCGGACGTTATCAGAATACCGGACAGGTATGTGCAGCGGCAAAACGCTTTATTATCGAAGAGGGAATT
GCTTCGGCATTTACCGAACGTTTTGTGGCAGCTGCGGCAGCCTTGAAAATGGGCGATCCCCGTGACGAAG
AGAACGCTCTCGGACCAATGGCTCGTTTTGATTTACGTGATGAGCTGCATCATCAGGTGGAGAAAACCCT
GGCGCAGGGTGCGCGTTTGTTACTGGGCGGGGAAAAGATGGCTGGGGCAGGTAACTACTATCCGCCAACG
GTTCTGGCGAATGTTACCCCAGAAATGACCGCGTTTCGGGAAGAAATGTTTGGCCCCGTTGCGGCAATCA
CCATTGCGAAAGATGCAGAACATGCACTGGAACTGGCTAATGATAGTGAGTTCGGCCTTTCAGCGACCAT
TTTTACCACTGACGAAACACAGGCCAGACAGATGGCGGCACGTCTGGAATGCGGTGGGGTGTTTATCAAT
GGTTATTGTGCCAGCGACGCGCGAGTGGCCTTTGGTGGCGTGAAAAAGAGTGGCTTTGGTCGTGAGCTTT
CCCATTTCGGCTTACACGAATTCTGTAATATCCAGACGGTGTGGAAAGACCGGATCTGA

Another NAD⁺ or NADP⁺-dependent aldehyde dehydrogenase in *E. coli* is encoded by the gene *astD* (EC 1.2.1.71). Thus, in a certain embodiment, the organism or microorganism of the present invention is *E. coli* having decreased levels and/or activity of the gene product of the *astD* gene. The nucleic acid sequence of the *astD* gene is provided in SEQ ID NO:10. In certain embodiments, the nucleic acid sequence as set forth in SEQ ID NO:10 may be deleted in the organism or microorganism according to the invention. In certain embodiments, a part of the nucleic acid sequence as set forth in SEQ ID NO:10 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:10 may be deleted in the organism or microorganism of the invention.

*E. coli αstD* sequence (SEQ ID NO:10):

ATGACTTTATGGATTAACGGTGACTGGATAACGGGCCAGGGCGCATCGCGTGTGAAGCGTAATCCGGTAT
CGGGCGAGGTGTTATGGCAAGGCAATGATGCCGATGCCGCTCAGGTCGAGCAGGCTTGTCGGGCAGCCCG
TGCGGCGTTTCCGCGCTGGGCGCGGCTTTCATTTGCTGAACGTCATGCCGTTGTCGAACGCTTTGCCGCA
CTGCTGGAAAGCAATAAAGCCGAATTAACCGCGATTATTGCCAGAGAAACGGGTAAGCCGCGCTGGGAAG
CGGCAACCGAAGTGACGGCGATGATCAATAAAATCGCGATATCAATTAAGGCGTATCACGTTCGTACCGG
CGAGCAGCGTAGTGAAATGCCGGACGGCGCGGCGAGCCTGCGACATCGCCCGCACGGCGTGCTGGCGGTG
TTTGGGCCGTATAATTTCCCTGGTCATTTGCCGAACGGACATATCGTTCCGGCATTGCTGGCAGGTAACA
CCATTATCTTTAAACCCAGCGAACTGACACCGTGGAGTGGCGAAGCGGTAATGCGTTTATGGCAGCAGGC
TGGCTTGCCGCCAGGCGTGCTGAACCTGGTGCAGGGCGGGCGTGAAACGGGTCAGGCGCTGAGTGCGCTG
GAGGATCTCGACGGTTTGCTGTTTACCGGTAGCGCCAATACAGGCTACCAGTTGCATCGCCAGCTCTCCG
GTCAGCCGGAGAAAATTCTCGCCCTTGAGATGGGCGGTAATAATCCGCTAATTATCGATGAGGTGGCGGA
TATCGACGCGGCTGTCCATCTGACCATTCAGTCGGCGTTTGTCACAGCCGGTCAACGCTGCACCTGCGCC
CGCCGTTTATTGCTGAAAAGCGGGGCGCAGGGCGATGCGTTTCTTGCTCGTCTGGTTGCCGTCAGCCAGC
GATTAACGCCGGGCAACTGGGATGACGAACCGCAGCCGTTTATTGGCGGGCTGATTTCTGAACAGGCCGC
ACAGCAGGTGGTTACTGCATGGCAGCAACTGGAAGCGATGGGCGGACGACCCCTGCTTGCGCCGCGCTTA
TTACAAGCAGGGACATCGTTGCTGACGCCGGGGATCATTGAAATGACAGGCGTTGCTGGCGTACCAGATG
AAGAGGTGTTCGGACCGTTATTGCGCGTCTGGCGTTATGATACTTTCGATGAAGCGATTCGAATGGCGAA
TAACACTCGCTTCGGACTCTCTTGCGGTCTGGTTTCCCCCGAGCGGGAAAAGTTCGATCAACTGTTGCTG
GAGGCGCGGGCGGGGATTGTTAACTGGAACAAACCGCTTACCGGTGCTGCCAGTACCGCGCCATTCGGCG
GCATTGGTGCTTCCGGTAACCATCGCCCCAGCGCCTGGTATGCCGCAGATTACTGCGCATGGCCGATGGC
GAGCCTGGAGTCGGACTCGTTAACATTGCCCGCCACGCTTAACCCCGGGCTGGATTTTTCCGATGAGGTG
GTGCGATGA

Another NAD⁺ or NADP⁺-dependent aldehyde dehydrogenase in *E. coli* is encoded by the gene *betB* (EC 1.2.1.8). Thus, in a certain embodiment, the organism or microorganism of the present invention is *E. coli* having decreased levels and/or activity of the gene product of the *betB* gene. The nucleic acid sequence of the *betB* gene is provided in SEQ ID NO:11. In certain embodiments, the nucleic acid sequence as set forth in SEQ ID NO:11 may be deleted in the organism or microorganism according to the invention. In certain embodiments, a part of the nucleic acid sequence as set forth in SEQ ID NO:11 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:11 may be deleted in the organism or microorganism of the invention.

*E. coli betB* sequence (SEQ ID NO:11):

ATGTCCCGAATGGCAGAACAGCAGCTTTATATACATGGTGGTTATACCTCCGCCACCAGCGGTCGCACCT
TCGAGACCATTAACCCGGCCAACGGTAACGTGCTGGCGACCGTGCAGGCCGCCGGGCGCGAGGATGTCGA
TCGCGCCGTGAAAAGCGCCCAGCAGGGGCAAAAAATCTGGGCGTCGATGACCGCCATGGAGCGCTCGCGT
ATTCTGCGTCGGGCCGTTGATATTCTGCGTGAACGCAATGACGAACTCGCAAAACTGGAAACCCTCGACA
CCGGAAAAGCATATTCGGAAACCTCAACCGTCGATATCGTTACCGGTGCGGACGTGCTGGAGTACTACGC
CGGGCTGATCCCGGCGCTGGAAGGCAGCCAGATCCCGTTGCGTGAAACGTCCTTTGTGTATACCCGCCGC
GAACCGCTGGGCGTAGTGGCAGGGATTGGCGCATGGAACTACCCGATCCAGATTGCCCTGTGGAAATCCG
CCCCGGCGCTGGCGGCAGGCAACGCAATGATTTTCAAACCGAGCGAAGTTACCCCGCTTACCGCGTTAAA
GCTGGCTGAAATTTACAGCGAAGCGGGCCTGCCGGACGGCGTATTTAACGTGTTGCCGGGCGTGGGCGCG
GAGACCGGGCAATATCTGACCGAGCATCCGGGCATTGCCAAAGTGTCATTTACCGGCGGTGTCGCCAGCG
GCAAAAAAGTGATGGCTAACTCGGCGGCCTCTTCCCTGAAAGAAGTGACCATGGAACTGGGCGGTAAATC
ACCGCTGATCGTTTTCGATGATGCGGATCTCGATCTCGCCGCCGATATCGCCATGATGGCAAACTTCTTC
AGCTCCGGTCAGGTGTGTACCAATGGCACCCGCGTCTTCGTTCCGGCGAAATGCAAAGCCGCATTTGAGC
AGAAAATTCTGGCGCGCGTTGAGCGCATTCGCGCGGGCGACGTTTTCGATCCGCAAACTAACTTCGGCCC
GCTGGTCAGCTTCCCGCATCGCGATAACGTGCTGCGCTATATCGCCAAAGGCAAAGAGGAAGGCGCGCGC
GTACTGTGCGGCGGCGATGTACTGAAAGGCGATGGCTTCGATAACGGCGCATGGGTTGCACCGACAGTGT
TCACCGATTGCAGCGACGATATGACCATCGTGCGTGAAGAGATCTTCGGGCCAGTGATGTCCATTCTGAC
CTACGAGTCGGAAGACGAAGTCATTCGCCGCGCTAACGATACCGACTACGGCCTGGCGGCGGGCATCGTG
ACAGCGGACCTGAACCGCGCGCATCGCGTCATTCATCAGCTGGAAGCGGGTATTTGCTGGATCAACACCT
GGGGCGAATCCCCGGCAGAGATGCCCGTTGGCGGCTACAAACACTCCGGCATTGGTCGCGAGAACGGCGT
GATGACGCTCCAGAGTTACACCCAGGTGAAGTCCATCCAGGTTGAGATGGCTAAATTCCAGTCCATATTC
TAA

Another NAD⁺ or NADP⁺-dependent aldehyde dehydrogenase in *E. coli* is encoded by the gene *aldB* (EC 1.2.1.4). Thus, in a certain embodiment, the organism or microorganism of the present invention is *E. coli* having decreased levels and/or activity of the gene product of the *aldB* gene. The nucleic acid sequence of the *aldB* gene is provided in SEQ ID NO:12. In certain embodiments, the nucleic acid sequence as set forth in SEQ ID NO:12 may be deleted in the organism or microorganism according to the invention. In certain embodiments, a part of the nucleic acid sequence as set forth in SEQ ID NO:12 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:12 may be deleted in the organism or microorganism of the invention.

*E. coli aldB* sequence (SEQ ID NO:12):

ATGACCAATAATCCCCCTTCAGCACAGATTAAGCCCGGCGAGTATGGTTTCCCCCTCAAGTTAAAAGCCC
GCTATGACAACTTTATTGGCGGCGAATGGGTAGCCCCTGCCGACGGCGAGTATTACCAGAATCTGACGCC
GGTGACCGGGCAGCTGCTGTGCGAAGTGGCGTCTTCGGGCAAACGAGACATCGATCTGGCGCTGGATGCT
GCGCACAAAGTGAAAGATAAATGGGCGCACACCTCGGTGCAGGATCGTGCGGCGATTCTGTTTAAGATTG
CCGATCGAATGGAACAAAACCTCGAGCTGTTAGCGACAGCTGAAACCTGGGATAACGGCAAACCCATTCG
CGAAACCAGTGCTGCGGATGTACCGCTGGCGATTGACCATTTCCGCTATTTCGCCTCGTGTATTCGGGCG
CAGGAAGGTGGGATCAGTGAAGTTGATAGCGAAACCGTGGCCTATCATTTCCATGAACCGTTAGGCGTGG
TGGGGCAGATTATCCCGTGGAACTTCCCGCTGCTGATGGCGAGCTGGAAAATGGCTCCCGCGCTGGCGGC
GGGCAACTGTGTGGTGCTGAAACCCGCACGTCTTACCCCGCTTTCTGTACTGCTGCTAATGGAAATTGTC
GGTGATTTACTGCCGCCGGGCGTGGTGAACGTGGTCAATGGCGCAGGTGGGGTAATTGGCGAATATCTGG
CGACCTCGAAACGCATCGCCAAAGTGGCGTTTACCGGCTCAACGGAAGTGGGCCAACAAATTATGCAATA
CGCAACGCAAAACATTATTCCGGTGACGCTGGAGTTGGGCGGTAAGTCGCCAAATATCTTCTTTGCTGAT
GTGATGGATGAAGAAGATGCCTTTTTCGATAAAGCGCTGGAAGGCTTTGCACTGTTTGCCTTTAACCAGG
GCGAAGTTTGCACCTGTCCGAGTCGTGCTTTAGTGCAGGAATCTATCTACGAACGCTTTATGGAACGCGC
CATCCGCCGTGTCGAAAGCATTCGTAGCGGTAACCCGCTCGACAGCGTGACGCAAATGGGCGCGCAGGTT
TCTCACGGGCAACTGGAAACCATCCTCAACTACATTGATATCGGTAAAAAAGAGGGCGCTGACGTGCTCA
CAGGCGGGCGGCGCAAGCTGCTGGAAGGTGAACTGAAAGACGGCTACTACCTCGAACCGACGATTCTGTT
TGGTCAGAACAATATGCGGGTGTTCCAGGAGGAGATTTTTGGCCCGGTGCTGGCGGTGACCACCTTCAAA
ACGATGGAAGAAGCGCTGGAGCTGGCGAACGATACGCAATATGGCCTGGGCGCGGGCGTCTGGAGCCGCA
ACGGTAATCTGGCCTATAAGATGGGGCGCGGCATACAGGCTGGGCGCGTGTGGACCAACTGTTATCACGC
TTACCCGGCACATGCGGCGTTTGGTGGCTACAAACAATCAGGTATCGGTCGCGAAACCCACAAGATGATG
CTGGAGCATTACCAGCAAACCAAGTGCCTGCTGGTGAGCTACTCGGATAAACCGTTGGGGCTGTTCTGA

Another NAD⁺ or NADP⁺-dependent aldehyde dehydrogenase in *E. coli* is encoded by the gene *gabD* (EC 1.2.1.79). Thus, in a certain embodiment, the organism or microorganism of the present invention is *E. coli* having decreased levels and/or activity of the gene product of the *gabD* gene. The nucleic acid sequence of the *gabD* gene is provided in SEQ ID NO:13. In certain embodiments, the nucleic acid sequence as set forth in SEQ ID NO:13 may be deleted in the organism or microorganism according to the invention. In certain embodiments, a part of the nucleic acid sequence as set forth in SEQ ID NO:13 may be deleted in the organism or microorganism according to the invention. That is, a continuous stretch comprising at least 1, 2, 3, 4, 5, 6, 7, 8, 9, 10, 20, 30, 40, 50, 100, 150, 200, 250, 300, 400, 500, 600, 700, 800, 900, 1000, 1100, 1200, 1300 or 1400 nucleotides of the nucleic acid sequence as set forth in SEQ ID NO:13 may be deleted in the organism or microorganism of the invention.

*E. coli gabD* sequence (SEQ ID NO:13):

ATGAAACTTAACGACAGTAACTTATTCCGCCAGCAGGCGTTGATTAACGGGGAATGGCTGGACGCCAACA
ATGGTGAAGCCATCGACGTCACCAATCCGGCGAACGGCGACAAGCTGGGTAGCGTGCCGAAAATGGGCGC
GGATGAAACCCGCGCCGCTATCGACGCCGCCAACCGCGCCCTGCCCGCCTGGCGCGCGCTCACCGCCAAA
GAACGCGCCACCATTCTGCGCAACTGGTTCAATTTGATGATGGAGCATCAGGACGATTTAGCGCGCCTGA
TGACCCTCGAACAGGGTAAACCACTGGCCGAAGCGAAAGGCGAAATCAGCTACGCCGCCTCCTTTATTGA
GTGGTTTGCCGAAGAAGGCAAACGCATTTATGGCGACACCATTCCTGGTCATCAGGCCGATAAACGCCTG
ATTGTTATCAAGCAGCCGATTGGCGTCACCGCGGCTATCACGCCGTGGAACTTCCCGGCGGCGATGATTA
CCCGCAAAGCCGGTCCGGCGCTGGCAGCAGGCTGCACCATGGTGCTGAAGCCCGCCAGTCAGACGCCGTT
CTCTGCGCTGGCGCTGGCGGAGCTGGCGATCCGCGCGGGCGTTCCGGCTGGGGTATTTAACGTGGTCACC
GGTTCGGCGGGCGCGGTCGGTAACGAACTGACCAGTAACCCGCTGGTGCGCAAACTGTCGTTTACCGGTT
CGACCGAAATTGGCCGCCAGTTAATGGAACAGTGCGCGAAAGACATCAAGAAAGTGTCGCTGGAGCTGGG
CGGTAACGCGCCGTTTATCGTCTTTGACGATGCCGACCTCGACAAAGCCGTGGAAGGCGCGCTGGCCTCG
AAATTCCGCAACGCCGGGCAAACCTGCGTCTGCGCCAACCGCCTGTATGTGCAGGACGGCGTGTATGACC
GTTTTGCCGAAAAATTGCAGCAGGCAGTGAGCAAACTGCACATCGGCGACGGGCTGGATAACGGCGTCAC
CATCGGGCCGCTGATCGATGAAAAAGCGGTAGCAAAAGTGGAAGAGCATATTGCCGATGCGCTGGAGAAA
GGCGCGCGCGTGGTTTGCGGCGGTAAAGCGCACGAACGCGGCGGCAACTTCTTCCAGCCGACCATTCTGG
TGGACGTTCCGGCCAACGCCAAAGTGTCGAAAGAAGAGACGTTCGGCCCCCTCGCCCCGCTGTTCCGCTT
TAAAGATGAAGCTGATGTGATTGCGCAAGCCAATGACACCGAGTTTGGCCTTGCCGCCTATTTCTACGCC
CGTGATTTAAGCCGCGTCTTCCGCGTGGGCGAAGCGCTGGAGTACGGCATCGTCGGCATCAATACCGGCA
TTATTTCCAATGAAGTGGCCCCGTTCGGCGGCATCAAAGCCTCGGGTCTGGGTCGTGAAGGTTCGAAGTA
TGGCATCGAAGATTACTTAGAAATCAAATATATGTGCATCGGTCTTTAA

As described in more detail above, reducing the level of an aldehyde dehydrogenase and, in particular any one of the NAD⁺/NADP⁺-dependent aldehyde dehydrogenases listed above, in an organism or microorganism of the invention may be achieved by fully or partially deleting the coding sequence of a gene encoding said enzyme or by deleting/modifying one or more regulatory elements of a gene encoding said enzyme. Alternatively, the activity of an aldehyde dehydrogenase and, in particular any one of the NAD⁺/NADP⁺-dependent aldehyde dehydrogenases listed above, in an organism or microorganism of the invention may be decreased by introducing inactivating mutations into the gene encoding said enzyme or through the addition of inhibitors of said enzyme.

It is important to note that the recombinant organism or microorganism may also be characterized by reduced levels and or activities of two or more aldehyde dehydrogenases and, in particular, two or more of the NAD⁺/NADP⁺-dependent aldehyde dehydrogenases listed above.

In a certain embodiment, the invention relates to the organism or microorganism according to the invention, wherein the activity of an aldehyde dehydrogenase is decreased due to the addition of an inhibitor, wherein the inhibitor is selected from the group consisting of: 1,2-cyclohexanedione, 2,4-dinitro-1-fluorobenzene, 2-mercaptoethanol, 3-thionicotinamide adenine dinucleotide, 4-hydroxy-2-nonenal, acetone, benomyl, benzoate, chloral hydrate, Co²⁺, Cu²⁺, diethyl decarbonate, dimethyl ampal thiolester, disulfiram, DMSO, EDTA, ethanediol, ethyl acetamide, Fe²⁺, guanidine hydrochloride, Hg²⁺, methyl ampal thiolester, Mg²⁺, Mn²⁺, N-acetylimidazole, N-bromosuccinimide, p-chloromercuribenzoate, p-hydroxymercuribenzoate, p-mercuribenzoate, Pb²⁺, PCMB, PEG-400, pentanal, phenylmethylsulfonyl fluoride, PMSF, pyrazole, trans-4-(N,N-dimethylamino)-cinnamaldehyde, urea and ZnCl₂.

A more efficient reduction of the metabolic flux from acetaldehyde to crotonic acid may be achieved by decreasing the levels and/or the activities of (i) one or more enzyme that catalyzes the conversion of acetaldehyde into crotonaldehyde and (ii) one or more enzyme that catalyzes the conversion of crotonaldehyde into crotonic acid. That is, in a preferred embodiment, the invention relates to a recombinant organism or microorganism according to the invention, wherein the levels and/or activities of at least one aldehyde lyase and at least one aldehyde dehydrogenase are decreased. In a more preferred embodiment, the invention relates to a recombinant microorganism according to the invention, wherein the microorganism is *E. coli* and wherein the levels and/or activities of the gene products of the genes *deoC* and *puuC* are decreased in said *E. coli* cells.

### The enzymatic conversion of acetyl-CoA into isobutene

Within the present invention, it is preferred that the organism or microorganism is capable of producing isobutene, preferably from acetyl-CoA. As described above, crotonic acid has been surprisingly identified to inhibit the conversion of 3-methylcrotonic acid to isobutene by the enzyme ferulic acid decarboxylase, despite an extensive evolution and improvement of its 3-methylcrotonic acid decarboxylase activity and the close proximity between the structures of the substrates crotonic acid and 3-methylcrotonic acid.

Methods for the production of isobutene from 3-methylcrotonyl-CoA via 3-methylcrotonic acid or from 3-hydroxyisovalerate (HIV) via 3-methylcrotonic acid have been described (see **Figure 1** for an overview). Methods as well as recombinant organisms and microorganisms utilizing these pathways and enzymatic conversions have, in particular, been described in WO 2017/085167, WO 2018/206262 and WO 2020/188033.

In the following, the major reactions of the individual enzymatic conversions as described in the prior art WO 2017/085167, WO 2018/206262, WO2010/001078, WO2012/052427 and WO 2016/042012, respectively, and as schematically illustrated in **Figure 1** are described in more detail.

However, the present invention is not limited to these major reactions but also relates to all other routes for the individual steps of the conversion of acetyl-CoA into isobutene as described in the prior art documents WO 2017/085167, WO 2018/206262, WO2010/001078, WO2012/052427 and WO 2016/042012. The disclosure of these documents, in particular with respect to preferred embodiments of the enzymes for the individual conversions of the pathways described therein, is herewith incorporated by reference in its entirety. Accordingly, in preferred embodiments, it is preferable to use the enzymes selected from the preferred embodiments described in these prior art documents in connection with the respective enzymatic conversion. Thus, the same applies to the enzymatic conversions of the present invention described in the following as has been set forth in WO 2017/085167, WO 2018/206262, WO2010/001078, WO2012/052427 and WO 2016/042012, respectively.

### The enzymatic conversion of acetyl-CoA into acetoacetyl-CoA

According to the present invention, the conversion of acetyl-CoA into acetoacetyl-CoA can be achieved by different routes. One possibility is to first convert acetyl-CoA into malonyl-CoA (**step XIV** as shown in **Figure 1**) and then to further condense said malonyl-CoA and acetyl-CoA into acetoacetyl-CoA (**step XV** as shown in **Figure 1**)**.** Another possibility is to directly condense in a single enzymatic reaction two molecules of acetyl-CoA into acetoacetyl-CoA (**step XIII** as shown in **Figure 1**).

The enzymatic conversion of acetyl-CoA into malonyl-CoA preferably makes use of an acetyl-CoA carboxylase (EC 6.4.1.2) (**step XIV** as shown in **Figure 1**). This naturally occurring reaction fixes CO₂ on acetyl-CoA utilizing ATP resulting in malonyl-CoA.

Moreover, the enzymatic condensation of malonyl-CoA and acetyl-CoA into said acetoacetyl-CoA preferably makes use of an acetoacetyl-CoA synthase (EC 2.3.1.194) (**step XV** as shown in **Figure 1**). This is a natural occurring reaction and condenses malonyl-CoA and acetyl-CoA in a decarboxylation reaction.

Alternatively, the enzymatic conversion of acetyl-CoA into said acetoacetyl-CoA consists of a single enzymatic reaction in which acetyl-CoA is directly converted into acetoacetyl-CoA by the enzymatic condensation of two molecules of acetyl-CoA into acetoacetyl-CoA. Preferably, this enzymatic conversion is achieved by making use of an acetyl-CoA acetyltransferase (EC 2.3.1.9). This reaction is a naturally occurring reaction (**step XIII** as shown in **Figure 1**).

### The enzymatic conversion of acetoacetyl-CoA into 3-hydroxy-3-methylglutaryl-CoA

The enzymatic conversion of acetoacetyl-CoA into 3-hydroxy-3-methylglutaryl-CoA is an enzymatic condensation of acetoacetyl-CoA and acetyl-CoA into said 3-hydroxy-3-methylglutaryl-CoA (see **step IX** of **Figure 1**).

This condensation preferably makes use of a 3-hydroxy-3-methylglutaryl-CoA synthase (also referred to as HMG-CoA synthase). HMG-CoA synthases are classified in EC 2.3.3.10 (formerly, HMG-CoA synthase has been classified as EC 4.1.3.5 but has been transferred to EC 2.3.3.10). The term "HMG-CoA synthase" refers to any enzyme which is able to catalyze the reaction where acetyl-CoA condenses with acetoacetyl-CoA to form 3-hydroxy-3-methylglutaryl-CoA (HMG-CoA). HMG-CoA synthase is part of the mevalonate pathway. Several pathways have been identified for the synthesis of isopentenyl pyrophosphate (IPP), i.e. the mevalonate pathway and the 2-*C*-methyl-D-erythritol 4-phosphate/1-deoxy-D-xylulose 5-phosphate (MEP/DOXP) pathway. HMG-CoA synthase catalyzes the biological Claisen condensation of acetyl-CoA with acetoacetyl-CoA and is a member of a superfamily of acyl-condensing enzymes that includes beta-ketothiolases, fatty acid synthases (beta-ketoacyl carrier protein synthase) and polyketide synthases.

### The enzymatic conversion of 3-hydroxy-3-methylglutaryl-CoA into 3-methylglutaconyl-CoA

The enzymatic conversion of 3-hydroxy-3-methylglutaryl-CoA into 3-methylglutaconyl-CoA is an enzymatic dehydration reaction which occurs naturally, and which is catalyzed, e.g., by enzymes classified as 3-methylglutaconyl-coenzyme A hydratase (EC 4.2.1.18). Accordingly, the enzymatic conversion of 3-hydroxy-3-methylglutaryl-CoA into 3-methylglutaconyl-CoA preferably makes use of a 3-methylglutaconyl-coenzyme A hydratase (EC 4.2.1.18) (as shown in **step VIII** of **Figure 1**).

The conversion of 3-hydroxy-3-methylglutaryl-CoA into 3-methylglutaconyl-CoA can also be achieved by making use of a 3-hydroxy-3-methylglutaryl-coenzyme A dehydratase activity which has been identified, e.g., in *Myxococcus xanthus* and which is encoded by the liuC gene (Li et al., Angew. Chem. Int. Ed. 52 (2013), 1304-1308). The 3-hydroxy-3-methylglutaryl-coenzyme A dehydratase derived from *Myxococcus xanthus* has the Uniprot accession number Q1D5Y4.

The enzymatic conversion of 3-hydroxy-3-methylglutaryl-CoA into 3-methylglutaconyl-CoA can also be achieved by making use of a 3-hydroxyacyl-CoA dehydratase or an enoyl-CoA hydratase. 3-hydroxyacyl-CoA dehydratases and enoyl-CoA hydratases catalyze the same reaction while the name of one of these enzymes denotes one direction of the corresponding reaction while the other name denotes the reverse reaction. As the reaction is reversible, both enzyme names can be used. 3-hydroxyacyl-CoA dehydratases and enoyl-CoA hydratases belong to enzymes classified as EC 4.2.1.

### The enzymatic conversion of 3-methylglutaconyl-CoA into 3-methylcrotonyl-CoA

The conversion of 3-methylglutaconyl-CoA into 3-methylcrotonyl-CoA may be catalyzed by different enzymes, e.g., by making use of (i) a methylcrotonyl-CoA carboxylase (EC 6.4.1.4); or (ii) a geranoyl-CoA carboxylase (EC 6.4.1.5) (as shown in **step VII** of **Figure 1**).

In another preferred embodiment the conversion of 3-methylglutaconyl-CoA via decarboxylation into 3-methylcrotonyl-CoA is catalyzed by a 3-methylglutaconyl-CoA decarboxylase, e.g. a 3-methylglutaconyl-CoA decarboxylase of *Myxococcus xanthus* encoded by the liuB gene. This gene codes for an enzyme having the two subunits AibA and AibB (Li et al., Angew. Chem. Int. Ed. 52 (2013), 1304-1308).

### The enzymatic conversion of 3-methylcrotonyl-CoA into isobutene via 3-methylcrotonic acid

The conversion of 3-methylcrotonyl-CoA into 3-methylcrotonic acid can, e.g., be achieved in different ways, e.g., by three alternative enzymatic routes described in the following and as shown in **Figure 1** (**step VIa**, **step VIb** or **step VIc** as shown in **Figure 1**).

Thus, the enzymatic conversion of 3-methylcrotonyl-CoA into 3-methylcrotonic acid may be achieved by
(a) a single enzymatic reaction in which 3-methylcrotonyl-CoA is directly converted into 3-methylcrotonic acid, preferably by making use of a CoA transferase (EC 2.8.3.-), preferably a propionate:acetate-CoA transferase (EC 2.8.3.1), an acetate CoA-transferase (EC 2.8.3.8) or a succinyl-CoA:acetate CoA-transferase (EC 2.8.3.18) (**step VIa** as shown in **Figure 1**);
(b) a single enzymatic reaction in which 3-methylcrotonyl-CoA is directly converted into 3-methylcrotonic acid, preferably by making use of a thioester hydrolase (EC 3.1.2), preferably an acetyl-CoA hydrolase (EC 3.1.2.1), an ADP-dependent short-chain-acyl-CoA hydrolase (EC 3.1.2.18) or an acyl-CoA hydrolase (EC 3.1.2.20) (**step VIb** as shown in **Figure 1**); or
(c) two enzymatic steps comprising
   (i) first enzymatically converting 3-methylcrotonyl-CoA into 3-methylcrotonyl phosphate; and
   (ii) then enzymatically converting the thus obtained 3-methylcrotonyl phosphate into said 3-methylcrotonic acid (**step VIc** as shown in **Figure 1**).

As regards (c), the enzymatic conversion of 3-methylcrotonyl-CoA into 3-methylcrotonic acid is achieved by two enzymatic steps comprising (i) first enzymatically converting 3-methylcrotonyl-CoA into 3-methylcrotonyl phosphate; and (ii) then enzymatically converting the thus obtained 3-methylcrotonyl phosphate into said 3-methylcrotonic acid.

The conversion of 3-methylcrotonyl-CoA into 3-methylcrotonyl phosphate can, e.g., be achieved by the use of a phosphate butyryltransferase (EC 2.3.1.19) or a phosphate acetyltransferase (EC 2.3.1.8).

The conversion of 3-methylcrotonyl phosphate into 3-methylcrotonic acid can, e.g., be achieved by making use of an enzyme which is classified as EC 2.7.2.-, i.e., a phosphotransferase. Such enzymes use a carboxy group as acceptor. Thus, the conversion of 3-methylcrotonyl phosphate into 3-methylcrotonic acid can, e.g., be achieved by making use of an enzyme with a carboxy group as acceptor (EC 2.7.2.-). In a preferred embodiment, the conversion of 3-methylcrotonyl phosphate into 3-methylcrotonic acid is achieved by the use of a propionate kinase (EC 2.7.2.15), an acetate kinase (EC 2.7.2.1), a butyrate kinase (EC 2.7.2.7) or a branched-chain-fatty-acid kinase (EC 2.7.2.14).

As mentioned above, the conversion of 3-methylcrotonyl-CoA into 3-methylcrotonic acid can also be achieved by two alternative conversions wherein 3-methylcrotonyl-CoA is directly converted into 3-methylcrotonic acid.

Preferably, in one embodiment, 3-methylcrotonyl-CoA is directly converted into 3-methylcrotonic acid by hydrolyzing the thioester bond of 3-methylcrotonyl-CoA into 3-methylcrotonic acid by making use of an enzyme which belongs to the family of thioester hydrolases (in the following referred to as thioesterases (EC 3.1.2.-)); **step VIb** as shown in **Figure 1**.

Thioesterases (TEs; also referred to as thioester hydrolases) are enzymes which are classified as EC 3.1.2. Presently thioesterases are classified as EC 3.1.2.1 through EC 3.1.2.30 while TEs which are not yet classified/unclassified are grouped as enzymes belonging to EC 3.1.2.-. Cantu et al. (Protein Science 19 (2010), 1281-1295) describe that there are 23 families of thioesterases which are unrelated to each other as regards the primary structure. However, it is assumed that all members of the same family have essentially the same tertiary structure. Thioesterases hydrolyze the thioester bond between a carbonyl group and a sulfur atom.

In a preferred embodiment, a thioesterase employed according to the present invention for converting 3-methylcrotonyl-CoA into 3-methylcrotonic acid is selected from the group consisting of:
- acetyl-CoA hydrolase (EC 3.1.2.1);
- palmitoyl-CoA hydrolase (EC 3.1.2.2);
- 3-hydroxyisobutyryl-CoA hydrolase (EC 3.1.2.4);
- oleoyl-[acyl-carrier-protein] hydrolase (EC 3.1.2.14);
- ADP-dependent short-chain-acyl-CoA hydrolase (EC 3.1.2.18);
- ADP-dependent medium-chain-acyl-CoA hydrolase (EC 3.1.2.19);
- 1,4-dihydroxy-2-naphthoyl-CoA hydrolase (EC 3.1.2.28); and
- acyl-CoA hydrolase (EC 3.1.2.20).

In more preferred embodiments, a thioesterase/thioester hydrolase (EC 3.1.2.-) employed according to the present invention is an acetyl-CoA hydrolase (EC 3.1.2.1), an ADP-dependent short-chain-acyl-CoA hydrolase (EC 3.1.2.18), a 1,4-dihydroxy-2-naphthoyl-CoA hydrolase (EC 3.1.2.28), and an acyl-CoA hydrolase (EC 3.1.2.20).

In an alternative embodiment, 3-methylcrotonyl-CoA is directly converted into 3-methylcrotonic acid, preferably by making use of an enzyme which belongs to the family of CoA-transferases (EC 2.8.3.-) capable of transferring the CoA group of 3-methylcrotonyl-CoA to a carboxylic acid (**step VIa** as shown in **Figure 1**).

CoA-transferases are found in organisms from all lines of descent. Most of the CoA-transferases belong to two well-known enzyme families (referred to in the following as families I and II) and there exists a third family which had been identified in anaerobic metabolic pathways of bacteria. A review describing the different families can be found in Heider (FEBS Letters 509 (2001), 345-349).

Preferably, the CoA-transferase employed according to the present invention for the direct conversion of 3-methylcrotonyl-CoA into 3-methylcrotonic acid is selected from the group consisting of:
- propionate:acetate-CoA transferase (EC 2.8.3.1);
- acetate CoA-transferase (EC 2.8.3.8); and
- butyrate-acetoacetate CoA-transferase (EC 2.8.3.9).

In more preferred embodiments, CoA transferases (EC 2.8.3.-) are a propionate:acetate-CoA transferase (EC 2.8.3.1), an acetate CoA-transferase (EC 2.8.3.8) and a succinyl-CoA:acetate CoA-transferase (EC 2.8.3.18).

Exemplary combinations of enzymes to achieve the conversion of acetyl-CoA to isobutene are listed in Tables 1 and 2.

**Table 1**

| **Step** | **Enzyme** | **Gene** | **NCBI reference** | **Uniprot Accession number** |
|---|---|---|---|---|
| I | Acetyl-CoA transferase from Clostridium acetobulyticum | thIA | WP_010966157.1 | P45359 |
| II | Hydroxymethylglutaryl-CoA synthase from Enterococcus faecalis | mvaS | WP_002357756.1 | Q9FD71 |
| III | Enoyl-CoA hydratase/isomerase from *Pseudomonas sp. UW4* (ECH) | PputUW4_01474 | WP_015094072.1 | K9NHK2 |
| IV | Glutaconate CoA-transferase from Myxococcus xanthus (AibA/B) | MXAN_4264 | WP_011554268.1 | Q1D4I3 |
| | | MXAN_4265 | WP_011554267.1 | Q1D4I4 |
| V | 1,4-Dihydroxy-2-naphtoyl-CoA hydrolase from Escherichia coli | Ydil, menl | NP_416201.1 | P77781 |
| VI | Variant of UbiD-like decarboxylase decarboxylase from Streptomyces sp.769 (UbiD) (A241D-G402A-S403C-C404L-P406A-L448W) | GZL_07100 | | A0A0A8EV26 |
| | Flavin prenyl transferase from Escherichia coli (UbiX) | ubiX | WP_000825700.1 | P0AG03 |

**Table 2**

| **Step** | **Enzyme** | **Gene** | **NCBI reference** | **Uniprot Accession number** |
|---|---|---|---|---|
| I | Acetyl-CoA transferase from *Clostridium kluyverii* | *thIA3* | EDK35683.1 | A5N317 |
| II | Hydroxymethylglutaryl-CoA synthase from *Enterococcus faecalis* | mvaS | WP_002357756.1 | Q9FD71 |
| III | Enoyl-CoA hydratase/isomerase from Pseudomonas sp. UW4 (ECH) | *PputUW4_01474* | WP_015094072.1 | K9NHK2 |
| IV | Glutaconate CoA-transferase from Myxococcus xanthus (AibA/B) | MXAN_4264 | WP_011554268.1 | Q1D4I3 |
| | | MXAN_4265 | WP_011554267.1 | Q1D4I4 |
| V | Acyl-CoA thioesterase | *tesB* | AAC73555.1 | P0AGG2 |
| VI | Variant of UbiD-like decarboxylase from *Hypocrea atroviridis (Trichoderma atroviride)* (UbiD) with N-terminal MBP fusion | *FDC1* | XP_013946967.1 | G9NLP8 |
| | Flavin prenyl transferase from Escherichia coli (UbiX) | ubiX | WP_000825700.1 | P0AG03 |

### An alternative route for the provision of 3-methylcrotonic acid

As outlined above, 3-methylcrotonic acid (which is then further enzymatically converted into isobutene as described in detail further below) can be enzymatically provided from acetyl-CoA by the enzymatic conversion of acetyl-CoA into acetoacetyl-CoA (**step XIV, step XV**, **step XIII** as shown in **Figure 1**), the enzymatic conversion of acetoacetyl-CoA into 3-hydroxy-3-methylglutaryl-CoA (**step IX** of **Figure 1**), the enzymatic conversion of 3-hydroxy-3-methylglutaryl-CoA into 3-methylglutaconyl-CoA (**step VIII** of **Figure 1**), the enzymatic conversion of 3-methylglutaconyl-CoA into 3-methylcrotonyl-CoA (**step VII** of **Figure 1**) and the enzymatic conversion of 3-methylcrotonyl-CoA into 3-methylcrotonic acid.

In an alternative route, according to the present invention, 3-methylcrotonic acid can be provided by another possible pathway from acetyl-CoA. In this pathway, acetyl-CoA is enzymatically converted into acetoacetyl-CoA as described above.

According to this alternative route, acetoacetyl-CoA is then enzymatically converted into acetoacetate (**step Va** or **Vb** of **Figure 1**), acetoacetate is further enzymatically converted into acetone (**step IV** of **Figure 1**), acetone is further enzymatically converted into 3-hydroxyisovalerate (HIV) (**step III** of **Figure 1**), which is then further enzymatically converted into said 3-methylcrotonic acid.

The individual enzymatic steps of this alternative pathway are described in more detail in the following.

### The enzymatic conversion of acetoacetyl-CoA into acetoacetate

The conversion of acetoacetyl-CoA into acetoacetate can be achieved by two different routes. One possibility is the conversion of acetoacetyl-CoA into acetoacetate by hydrolysing the CoA thioester of acetoacetyl-CoA into acetoacetate (**step Va** as shown in **Figure 1**). In another, more preferred, aspect the CoA group of acetoacetyl-CoA is transferred on acetate, resulting in the formation of acetoacetate and acetyl-CoA (**step Vb** as shown in **Figure 1**).

As mentioned, in one aspect, the CoA thioester of acetoacetyl-CoA is hydrolyzed to result in acetoacetate. According to this aspect of the present invention, the enzymatic conversion of acetoacetyl-CoA into acetoacetate is achieved by preferably making use of an acetoacetyl-CoA hydrolase (EC 3.1.2.11) which naturally catalyzes this reaction.

As mentioned, in another, more preferred, possibility, the CoA group of acetoacetyl-CoA is transferred on acetate, resulting in the formation of acetoacetate and acetyl-CoA. According to this possibility of the present invention, the enzymatic conversion of acetoacetyl-CoA into acetoacetate is achieved by preferably making use of an enzyme which is capable of transferring the CoA group of acetoacetyl-CoA on acetate.

Preferably, such an enzyme capable of transferring the CoA group of acetoacetyl-CoA on acetate belongs to the family of CoA transferases (EC 2.8.3.-).

Thus, the present invention relates to a method for the enzymatic conversion of acetoacetyl-CoA into acetoacetate by making use of an enzyme capable of transferring the CoA group of acetoacetyl-CoA on acetate, preferably a CoA transferase (EC 2.8.3.-). A preferred example of an enzyme catalysing the conversion of acetoacetyl-CoA into acetoacetate which can be employed in the method of the present invention is an enzyme classified as an acetate CoA transferase (EC 2.8.3.8).

### The enzymatic conversion of acetoacetate into acetone

The conversion of acetoacetate into acetone is schematically illustrated in **step IV** of **Figure 1**. This reaction is a decarboxylation reaction and is a natural occurring reaction in organisms capable of producing acetone, i.e., organisms of the genus Clostridia. According to the present invention, the conversion of acetoacetate into said acetone preferably makes use of an acetoacetate decarboxylase (EC 4.1.1.4).

### The enzymatic condensation of acetone and acetyl-CoA into 3-hydroxyisovalerate (HIV)

The condensation of acetone and acetyl-CoA into said 3-hydroxyisovalerate (HIV) is schematically illustrated in **step III** of **Figure 1**. This condensation preferably makes use of an enzyme which is capable of catalyzing the formation of a covalent bond between the carbon atom of the oxo (i.e., the C=O) group of acetone and acetyl-CoA, in particular the methyl group of acetyl-CoA. According to this reaction scheme, the oxo group of acetone reacts as an electrophile and the methyl group of acetyl-CoA reacts as a nucleophile.

Enzymes which are capable of enzymatically condensing acetone and acetyl-CoA into 3-hydroxyisovalerate (HIV) are known in the art and have, e.g., been described in WO 2011/032934.

Preferably, the enzyme employed in the enzymatic condensation of acetone and acetyl-CoA into 3-hydroxyisovalerate (HIV) is an enzyme with the activity of a HMG CoA synthase (EC 2.3.3.10) and/or a PksG protein and/or an enzyme with the activity of a C-C bond cleavage/condensation lyase (preferably enzymes classified as isopropylmalate synthase (EC 2.3.3.13), as homocitrate synthase (EC 2.3.3.14) or as 4-hydroxy-2-ketovalerate aldolase (EC 4.1.3.39)), such as a HMG CoA lyase (EC 4.1.3.4).

### The enzymatic conversion of 3-hydroxyisovalerate (HIV) into 3-methylcrotonic acid

The enzymatic conversion of 3-hydroxyisovalerate (HIV) into 3-methylcrotonic acid is schematically illustrated in **step II** of **Figure 1**. This conversion preferably makes use of an enzyme catalyzing the dehydration of a β-hydroxy acid (i.e., e.g., 3-hydroxyisovalerate (HIV)) into an α,β-unsaturated acid (i.e., e.g., 3-methylcrotonic acid). The term "dehydration" generally refers to a reaction involving the removal of H₂O. Preferably, such an enzyme belongs to the family of hydro-lyases (EC 4.2.-.-).

Preferred examples of such enzymes which are classified as EC 4.2.-.- (i.e., hydro-lyases) are:
aconitase (EC 4.2.1.3);
fumarase (EC 4.2.1.2); and
enoyl-CoA hydratase/dehydratease (EC 4.2.1.17).

### The enzymatic conversion of 3-methylcrotonic acid into isobutene

The enzymatic conversion of 3-methylcrotonic acid into isobutene is schematically shown in **step I** of **Figure 1**). This conversion can be achieved by a decarboxylation by making use of a prenylated FMN-dependent decarboxylase associated with an FMN prenyl transferase. "Decarboxylation" is generally a chemical reaction that removes a carboxyl group and releases carbon dioxide (CO₂).

The enzymatic conversion of 3-methylcrotonic acid into isobutene utilizing a prenylated FMN-dependent decarboxylase associated with an FMN prenyl transferase relies on a reaction of two consecutive steps catalyzed by the two enzymes, i.e., the prenylated FMN-dependent decarboxylase (catalyzing the actual decarboxylation of 3-methylcrotonic acid into isobutene) with an associated FMN prenyl transferase which provides the modified flavin cofactor.

The flavin cofactor may preferably be FMN or FAD. FMN (flavin mononucleotide; also termed riboflavin-5'-phosphate) is a biomolecule produced from riboflavin (vitamin B2) by the enzyme riboflavin kinase and functions as prosthetic group of various reactions. FAD (flavin adenine dinucleotide) is a redox cofactor, more specifically a prosthetic group, involved in several important reactions in metabolism.

Thus, in the conversion of 3-methylcrotonic acid into isobutene, in a first step, a flavin cofactor (FMN or FAD) is modified into a (modified) flavin-derived cofactor. This modification is catalyzed by said FMN prenyl transferase. FMN prenyl transferase prenylates the flavin ring of the flavin cofactor (FMN or FAD) into a (modified) prenylated flavin cofactor. More specifically, FMN prenyl transferase catalyzes the prenylation of a flavin cofactor (FMN or FAD) utilizing dimethylallyl phosphate (DMAP) or dimethylallyl pyrophosphate (DMAPP) into a flavin-derived cofactor.

In a second step, the actual conversion of 3-methylcrotonic acid into isobutene is catalyzed by said prenylated FMN-dependent decarboxylase via a 1,3-dipolar cycloaddition based mechanism wherein said prenylated FMN-dependent decarboxylase uses the prenylated flavin cofactor (FMN or FAD) provided by the associated FMN prenyl transferase.

In a preferred embodiment, said FMN prenyl transferase which modifies the flavin cofactor (FMN or FAD) into a (modified) flavin-derived cofactor (utilizing dimethylallyl phosphate (DMAP) or dimethylallyl pyrophosphate (DMAPP)) is a phenylacrylic acid decarboxylase (PAD)-type protein, or the closely related prokaryotic enzyme UbiX, an enzyme which is involved in ubiquinone biosynthesis in prokaryotes.

In *Escherichia coli,* the protein UbiX (also termed 3-octaprenyl-4-hydroxybenzoate carboxy-lyase) has been shown to be involved in the third step of ubiquinone biosynthesis.

In a preferred embodiment, the modification of a flavin cofactor (FMN or FAD) into the corresponding (modified) flavin-derived cofactor is catalyzed by the FMN-containing protein phenylacrylic acid decarboxylase (PAD). The enzymes involved in the modification of the flavin cofactor (FMN or FAD) into the corresponding modified flavin-derived cofactor were initially annotated as decarboxylases (EC 4.1.1.-). Some phenylacrylic acid decarboxylases (PAD) are now annotated as flavin prenyl transferases as EC 2.5.1.-. Enzymes capable of catalyzing the enzymatic reaction described herein for flavin prenyl transferases have recently also been annotated as flavin prenyl transferases as EC 2.5.1.129.

In a more preferred embodiment, the conversion of 3-methylcrotonic acid into isobutene makes use of a phenylacrylic acid decarboxylase (PAD)-type protein as the FMN prenyl transferase which modifies a flavin cofactor (FMN or FAD) into the corresponding (modified) flavin-derived cofactor wherein said phenylacrylic acid decarboxylase (PAD)-type protein is derived from Candida albicans (Uniprot accession number Q5A8L8), Aspergillus niger (Uniprot accession number A3F715), Saccharomyces cerevisiae (Uniprot accession number P33751) or Cryptococcus gattii (Uniprot accession number E6R9Z0).

In another preferred embodiment, the modification of a flavin cofactor (FMN or FAD) into the corresponding (modified) flavin-derived cofactor is catalyzed by the FMN-containing protein 3-octaprenyl-4-hydroxybenzoate carboxy-lyase also termed UbiX (initially annotated EC 4.1.1.-). As mentioned above, the enzymes involved in the modification of the flavin cofactor (FMN or FAD) into the corresponding modified flavin-derived cofactor were initially annotated as decarboxylases. Some phenylacrylic acid decarboxylases (PAD) are now annotated as flavin prenyl transferases as EC 2.5.1.-.

As mentioned above, enzymes capable of catalyzing the enzymatic reaction described herein for flavin prenyl transferases have recently also been annotated as flavin prenyl transferases as EC 2.5.1.129.

In a more preferred embodiment, the conversion of 3-methylcrotonic acid into isobutene makes use of a 3-octaprenyl-4-hydroxybenzoate carboxy-lyase (also termed UbiX) as the FMN prenyl transferase which modifies the flavin cofactor (FMN or FAD) into the corresponding (modified) flavin-derived cofactor wherein said 3-octaprenyl-4-hydroxybenzoate carboxy-lyase (also termed UbiX) is derived from Escherichia coli (Uniprot accession number P0AG03), Bacillus subtilis (Uniprot accession, number A0A086WXG4), Pseudomonas aeruginosa (Uniprot accession number A0A072ZCW8) or Enterobactersp. DC4 (Uniprot accession number W7P6B1).

In another preferred embodiment, the modification of a flavin cofactor (FMN or FAD) into the corresponding (modified) flavin-derived cofactor is catalyzed by an Ubx-like flavin prenyl transferase derived from E. coli encoded by kpdB and ecdB, respectively (UniProt accession number A0A023LDW3 and UniProt accession number P69772, respectively), and an Ubx-like flavin prenyl transferase derived from Klebsiella pneumoniae encoded by kpdB (UniProt accession number Q462H4).

In another preferred embodiment, the modification of a flavin cofactor (FMN or FAD) into the corresponding (modified) flavin-derived cofactor is catalyzed by a flavin prenyl transferase.

As mentioned above, the actual decarboxylation, i.e., the conversion of 3-methylcrotonic acid into isobutene is catalyzed by an prenylated FMN-dependent decarboxylase via a 1,3-dipolar cycloaddition based mechanism wherein said prenylated FMN-dependent decarboxylase uses the prenylated flavin cofactor (FMN or FAD) provided by any of the above described associated FMN prenyl transferases.

In a preferred embodiment, said prenylated FMN-dependent decarboxylase catalyzing the decarboxylation of 3-methylcrotonic acid into isobutene is catalyzed by a ferulic acid decarboxylase (FDC). Ferulic acid decarboxylases (FDC) belong to the enzyme class EC 4.1.1.-. In an even more preferred embodiment, the conversion of 3-methylcrotonic acid into isobutene makes use of a ferulic acid decarboxylases (FDC) which is derived from Saccharomyces cerevisiae (Uniprot accession number Q03034), Enterobacter sp. (Uniprot accession number V3P7U0), Bacillus pumilus (Uniprot accession number Q45361), Aspergillus niger (Uniprot accession number A2R0P7) or Candida dubliniensis (Uniprot accession number B9WJ66).

In another more preferred embodiment, the conversion of 3-methylcrotonic acid into isobutene makes use of a protocatechuate decarboxylase (EC 4.1.1.63).

In a preferred embodiment of the present invention, the PCA decarboxylase employed in the method of the present invention is a PCA decarboxylase which is derived from Klebsiella pneumoniae (Uniprot accession number B9AM6), Leptolyngbya sp. (Uniprot accession number A0A0S3U6D8), or Phascolarctobacterium sp. (Uniprot accession number R6IIV6).

In another preferred embodiment, said prenylated FMN-dependent decarboxylase catalyzing the decarboxylation of 3-methylcrotonic acid into isobutene is an enzyme which is closely related to the above ferulic acid decarboxylase (FDC), namely a 3-polyprenyl-4-hydroxybenzoate decarboxylase (also termed UbiD). 3-polyprenyl-4-hydroxybenzoate decarboxylase belongs to the UbiD decarboxylase family classified as EC 4.1.1.-.

In a more preferred embodiment, the conversion of 3-methylcrotonic acid into isobutene makes use of a 3-polyprenyl-4-hydroxybenzoate decarboxylase (UbiD) which is derived from Hypocrea atroviridis (UniProt Accession number G9NLP8), Sphaerulina musiva (UniProt Accession number M3DF95), Penecillinum requeforti (UniProt Accession number W6QKP7), Fusarium oxysporum f. sp. lycopersici (UniProt Accession number W9LTH3), Saccharomyces kudriavzevii (UniProt Accession number J8TRN5), Saccaromyces cerevisiae, Aspergillus parasiticus, Candida albicans, Grosmannia clavigera, Escherichia coli (Uniprot accession number P0AAB4), Bacillus megaterium (Uniprot accession number D5DTL4), Methanothermobacter sp. CaT2 (Uniprot accession number T2GKK5), Mycobacterium chelonae 1518 (Uniprot accession number X8EX86) or Enterobacter cloacae (Uniprot accessin number V3DX94).

In another more preferred embodiment, the conversion of 3-methylcrotonic acid into isobutene makes use of an UbiD-like decarboxylase which is derived from Streptomyces sp (UniProt Accession number A0A0A8EV26).

In an even more preferred embodiment, the UbiD-like decarboxylase which is derived from Streptomyces sp. is an enzyme comprising the amino acid sequence of SEQ ID NO: 14 or a sequence which is at least n % identical to SEQ ID NO: 14 with n being an integer between 10 and 100, preferably 10, 15, 20, 25, 30, 35, 40, 45, 50, 55, 60, 65, 70, 75, 80, 85, 90, 91, 92, 93, 94, 95, 96, 97, 98 or 99 and wherein the enzyme has the enzymatic activity of converting 3-methylcrotonic acid into isobutene.

Streptomyces sp. UbiD (SEQ ID NO:14)

MYVQMVFVKEVPVQHPSDLREHIEALEKLGDLDRVHDEVDWNLEAAAQTRYSTEHHLPAP
LFENVAGVAEGFRLLGAPAALSSDPSRPYARVALSVGLRPEATGREVVEHLVAARHRPGV
PPVAVAAEAAPVKANVLLGDEADLNRFPVPFVHEGDGNRYANTYGVIIAQTPDGSWTNWS
IARIMMIDGKHMTGLVMHPQHIAQVWQQWADLGKPMPYALVQGGDPAIPYVGGIPIGDGV
AESAYIGALIGRPLEVVKAELSDLMVPAGAEIVIEGHLSVQRDGVEGPFGEFAGYIPRET
SLQPVYTVEAITHRDAPIWPLVAEGKPTDDFHTVTGIGEAAGALDAIREAGLPAASAWAP
LSAASHWLVVTAPGNWRELLPGVSEEQYARRVGEAVFGTKFGSCLPQVFLLDDDFDPTDD
ADLLWALATRVHPDGRVVRFEDGPVLPLLTCYTPQERHAARATKVVHEALLSAPGEREPQ
STFADAYPAEVRAKVRARYPN

In another more preferred embodiment, the conversion of 3-methylcrotonic acid into isobutene makes use of an UbiD-like decarboxylase which is derived from *Yersinia frederiksenii* (Uniprot Accession Number: A0A0T9UUQ9).

In an even more preferred embodiment, the UbiD-like decarboxylase which is derived from *Yersinia frederiksenii* is an enzyme comprising the amino acid sequence of SEQ ID NO: 15 or a sequence which is at least n % identical to SEQ ID NO: 15 with n being an integer between 10 and 100, preferably 10, 15, 20, 25, 30, 35, 40, 45, 50, 55, 60, 65, 70, 75, 80, 85, 90, 91, 92, 93, 94, 95, 96, 97, 98 or 99 and wherein the enzyme has the enzymatic activity of converting 3-methylcrotonic acid into isobutene.

*Yersinia frederiksenii* UbiD (SEQ ID NO:15)

MFLKSLREQINLLKRQGDLRIINRPVDSYLEAAAIIRRSTETKAPVPLMTNIKGYPGCSI
VGGLAALSSNPEYPLSRVAMSLGLSTTASAQSIVQYLIDGLKKTPYLPVEIDSNSAPCKQ
NILRGEDASLAHFPIPQVHQHDGNRYVNTWGVFIVESPDGQWCNWSIQRVQYLNDRQMIA
LVFPSQHISDIWEEWVKIGKPMPYALVQGCEPIVPYIAGLPLLERNVNEAEYIGALYGKG
IEVVKCETHDLRVPASSEVVIEGYFAITREGVEGPFGEFAGYIPNENSLQPILSITAISW
QDDPIWPVVAEGKPVDEYHTCSGIGDAAAIQNVLLTANLPVSFVWVPLYSACHLMIVSIN
RHWRQTHPDLTSEQLTKKVGDVIHQTRNSIKLPKIVVLDDDVDPADTESLLWALATRIHP
ERKRYFYESCILPLLSCYSEEERHNRKGKRVIIDALLPENHGAISSFDYAYPDDIKQRVL
MHWETDFSHD

Preferably, the UbiD-like decarboxylase which is derived from *Yersinia frederiksenii* has been genetically engineered to increase the conversion of 3-methylcrotonic acid into isobutene.

That is, in a particular embodiment, the UbiD-like decarboxylase which is derived from *Yersinia frederiksenii* may be an engineered enzyme having an improved activity in converting 3-methylcrotonic acid into isobutene over the corresponding enzyme from which it is derived and having an amino acid sequence as shown in SEQ ID NO:15 or an amino acid sequence having at least 55% sequence identity to SEQ ID NO:15.

In particular, one or more amino acid residues at a position selected from the group consisting of positions 7, 17, 27, 33, 35, 45, 46, 48, 51, 140, 144, 183, 184, 185, 222, 227, 284, 285, 286, 287, 288, 289, 290, 292, 321, 322, 327, 329, 330, 331, 337, 338, 355, 365, 378, 380, 384, 387, 388, 389, 391, 392, 393, 394, 395, 419, 424, 425, 428, 429, 431, 432, 434, 436, 437, 438, 439, 443, 444, 446, 447, 448, 449, 451, 453, 457, 458, 459, 460, 462, 464, 465, 467, 468, 470 and 471 in the amino acid sequence shown in SEQ ID NO:15 or at a position corresponding to any of these positions, may be substituted with another amino acid residue or deleted or wherein an insertion has been effected at one or more of these positions.

Specific mutant variants of an UbiD-like decarboxylase which is derived from *Yersinia frederiksenii* with improved activity with 3-methylcrotonic acid are provided in EP 21 16 6368.7, which is fully incorporated herein by reference. Preferably, the engineeredUbiD-like decarboxylase which is derived from *Yersinia frederiksenii* comprises any of the mutations listed below in Table 3. The list of improved variants is presented in the following Table 3. The increase in activity is described relative to the wild-type enzyme (with "+" representing a low increase in activity and "++++" representing a high increase in activity). The mutations are described as followed: R387A means the wild-type amino-acid arginine (R) at position 387 is replaced by an alanine (A); dG462 means the wild-type amino-acid glycine (G) at position 462 is deleted; i443aR means an arginine (R) has been inserted after the amino-acid at position 443 (if a second amino-acid is inserted, it will be annotated with i443b). All amino acid positions in Table 3 are given with respect to SEQ ID NO:15.

**Table 3**

| **Mutants** | **Activity relative to WT** |
|---|---|
| R387A | + |
| L432A | + |
| L434V | + |
| S436A | + |
| G380V-K391L-L432W-L434M | + |
| L432F-L434W | + |
| L432F-L434F | + |
| L432W-L434W | + |
| K391I-L432W-L434M | + |
| L432A-S436A | + |
| L432F-L434W-S436A | + |
| R387A-L432F-L434W-S436A | + |
| N388M-L432F-L434W | + |
| K394R-L432F-L434W | + |
| C430S-L432F-L434W | + |
| L432F-L434W-S436N | + |
| N388M-L432F-L434W-S436A | + |
| C430P-L432F-L434W-S436A | + |
| C430S-L432F-L434W-S436A | + |
| K394R-L432F-L434W-S436A | + |
| N388M-L432F-L434W-S436N | + |
| C430P-L432F-L434W-S436N | + |
| C430S-L432F-L434W-S436N | + |
| K394R-L432F-L434W-S436N | + |
| N388M-C430P-L432F-L434W-S436A | + |
| N388M-C430S-L432F-L434W-S436A | + |
| N388M-K394R-L432F-L434W-S436A | + |
| K394R-C430P-L432F-L434W-S436A | + |
| K394R-C430S-L432F-L434W-S436A | + |
| N388M-C430P-L432F-L434W-S436N | + |
| N388M-C430S-L432F-L434W-S436N | + |
| N388M-K394R-L432F-L434W-S436N | + |
| K394R-C430S-L432F-L434W-S436N | + |
| K394R-C430P-L432F-L434W-S436N | + |
| N388M-K394R-C430S-L432F-L434W-S436A | + |
| N388M-K394R-C430P-L432F-L434W-S436A | + |
| N388M-K394R-C430P-L432F-L434W-S436N | + |
| N388M-K394R-C430S-L432F-L434W-S436N | + |
| H141Y-N388M-K394R-C430P-L432F-L434W-S436N | + |
| N388M-K394R-C430K-L432F-L434W-S436N | + |
| N388M-K394R-C430N-L432F-L434W-S436N | + |
| N388M-K394R-I395V-C430P-L432F-L434W-S436N | + |
| N388M-C430A-L432F-L434W-S436N | + |
| N388M-K394A-C430P-L432F-L434W-S436N | + |
| N388M-K394C-C430P-L432F-L434W-S436N | + |
| N388M-K394H-C430P-L432F-L434W-S436N | + |
| N388Q-C430P-L432F-L434W-S436N | + |
| S185T-N388M-C430P-L432F-L434W-S436N | + |
| N388M-K394H-C430S-L432F-L434W-S436N | + |
| S185T-N388M-C430S-L432F-L434W-S436N | + |
| N388M-K394R-C430A-L432F-L434W-S436N | + |
| N388M-K394R-C430N-L432F-L434W-S436N-H444L | + |
| N388M-K394R-C430N-L432F-L434W-S436N-H444N | + |
| N388M-K394R-C430N-L432F-L434W-S436N-H444R | + |
| N388M-K394R-C430N-L432F-L434W-S436N-H444T | + |
| N388M-K394R-C430N-L432F-L434W-S436N-H444V | + |
| N285M-N388M-K394R-C430N-L432F-L434W-S436N | + |
| N287I-N388M-K394R-C430N-L432F-L434W-S436N | + |
| N287K-N388M-K394R-C430N-L432F-L434W-S436N | + |
| N287Q-N388M-K394R-C430N-L432F-L434 W-S436N | + |
| N287R-N388M-K394R-C430N-L432F-L434W-S436N | + |
| N287T-N388M-K394R-C430N-L432F-L434W-S436N | + |
| N287V-N388M-K394R-C430N-L432F-L434W-S436N | + |
| N332H-N388M-K394R-C430N-L432F-L434W-S436N | + |
| P284E-N388M-K394R-C430N-L432F-L434W-S436N | + |
| S322T-N388M-K394R-C430N-L432F-L434W-S436N | + |
| N388M-K394R-S429D-C430N-L432F-L434W-S436N | + |
| V382A-N388M-K394R-C430N-L432F-L434W-S436N | + |
| V382L-N388M-K394R-C430N-L432F-L434W-S436N | + |
| P284E-N285M-N388M-K394R-C430N-L432F-L434W-S436N | ++ |
| P284E-N287Q-N388M-K394R-C430N-L432F-L434W-S436N | ++ |
| P284E-N287V-N388M-K394R-C430N-L432F-L434W-S436N | ++ |
| P284E-N287R-N388M-K394R-C430N-L432F-L434W-S436N | ++ |
| P284E-N287T-N388M-K394R-C430N-L432F-L434W-S436N | ++ |
| P284E-N287K-N388M-K394R-C430N-L432F-L434W-S436N | ++ |
| P284E-S322T-N388M-K394R-C430N-L432F-L434W-S436N | ++ |
| N285M-N287Q-N388M-K394R-C430N-L432F-L434W-S436N | ++ |
| N285M-N287V-N388M-K394R-C430N-L432F-L434W-S436N | ++ |
| N285M-N287R-N388M-K394R-C430N-L432F-L434W-S436N | ++ |
| N285M-N287T-N388M-K394R-C430N-L432F-L434W-S436N | ++ |
| N285M-N287K-N388M-K394R-C430N-L432F-L434W-S436N | ++ |
| N285M-S322T-N388M-K394R-C430N-L432F-L434W-S436N | ++ |
| N287Q-S322T-N388M-K394R-C430N-L432F-L434W-S436N | ++ |
| N287V-S322T-N388M-K394R-C430N-L432F-L434W-S436N | ++ |
| N287R-S322T-N388M-K394R-C430N-L432F-L434W-S436N | ++ |
| N287T-S322T-N388M-K394R-C430N-L432F-L434W-S436N | ++ |
| N287K-S322T-N388M-K394R-C430N-L432F-L434W-S436N | ++ |
| P284E-N285M-N287Q-N388M-K394R-C430N-L432F-L434W-S436N | ++ |
| P284E-N285M-N287V-N388M-K394R-C430N-L432F-L434W-S436N | ++ |
| P284E-N285M-N287Q-S322T-N388M-K394R-C430N-L432F-L434W-S436N | ++ |
| P284E-N285M-N287T-N388M-K394R-C430N-L432F-L434 W-S436N | ++ |
| P284E-N285M-N287K-N388M-K394R-C430N-L432F-L434W-S436N | ++ |
| P284E-N285M-S322T-N388M-K394R-C430N-L432F-L434W-S436N | ++ |
| P284E-N287Q-S322T-N388M-K394R-C430N-L432F-L434W-S436N | ++ |
| P284E-N287V-S322T-N388M-K394R-C430N-L432F-L434W-S436N | ++ |
| P284E-N287R-S322T-N388M-K394R-C430N-L432F-L434W-S436N | ++ |
| P284E-N287T-S322T-N388M-K394R-C430N-L432F-L434W-S436N | ++ |
| P284E-N287K-S322T-N388M-K394R-C430N-L432F-L434W-S436N | ++ |
| N285M-N287Q-S322T-N388M-K394R-C430N-L432F-L434W-S436N | ++ |
| N285M-N287V-S322T-N388M-K394R-C430N-L432F-L434W-S436N | ++ |
| N285M-N287R-S322T-N388M-K394R-C430N-L432F-L434W-S436N | ++ |
| N285M-N287T-S322T-N388M-K394R-C430N-L432F-L434W-S436N | ++ |
| N285M-N287K-S322T-N388M-K394R-C430N-L432F-L434W-S436N | ++ |
| P284E-N285M-N287R-N388M-K394R-C430N-L432F-L434W-S436N | ++ |
| P284E-N285M-N287V-S322T-N388M-K394R-C430N-L432F-L434W-S436N | ++ |
| P284E-N285M-N287R-S322T-N388M-K394R-C430N-L432F-L434W-S436N | ++ |
| P284E-N285M-N287T-S322T-N388M-K394R-C430N-L432F-L434W-S436N | ++ |
| P284E-N285M-N287K-S322T-N388M-K394R-C430N-L432F-L434W-S436N | ++ |
| I283T-N285P-N287T-Q290E-S322T-N388M-K394R-C430A-L432F-L434W-S436N-dG462-dA463 | ++ |
| S189C-P284E-N287R-S322T-N388M-K394R-C430N-L432F-L434W-S436N | +++ |
| I283S-P284E-N287R-S322T-N388M-K394R-C430N-L432F-L434W-S436N | +++ |
| I283F-P284E-N287R-S322T-N388M-K394R-C430N-L432F-L434W-S436N | +++ |
| I283H-P284E-N287R-S322T-N388M-K394R-C430N-L432F-L434W-S436N | +++ |
| I283V-P284E-N287R-S322T-N388M-K394R-C430N-L432F-L434W-S436N | +++ |
| P284D-N287R-S322T-N388M-K394R-C430N-L432F-L434W-S436N | +++ |
| I283T-P284E-N287R-S322T-N388M-K394R-C430N-L432F-L434W-S436N | +++ |
| I283L-P284E-N287R-S322T-N388M-K394R-C430N-L432F-L434W-S436N | +++ |
| P284E-N285T-N287R-S322T-N388M-K394R-C430N-L432F-L434W-S436N | +++ |
| P284E-E286W-N287R-S322T-N388M-K394R-C430N-L432F-L434W-S436N | +++ |
| P284E-N287S-S322T-N388M-K394R-C430N-L432F-L434W-S436N | +++ |
| P284E-N285H-N287R-S322T-N388M-K394R-C430N-L432F-L434W-S436N | +++ |
| P284E-N287P-S322T-N388M-K394R-C430N-L432F-L434W-S436N | +++ |
| P284E-N287R-S288N-S322T-N388M-K394R-C430N-L432F-L434W-S436N | +++ |
| P284E-N287R-C321V-S322T-N388M-K394R-C430N-L432F-L434W-S436N | +++ |
| P284E-N287R-S322T-A329S-N388M-K394R-C430N-L432F-L434W-S436N | +++ |
| L6M-P284E-N287R-S322T-N388M-K394R-C430N-L432F-L434W-S436N | +++ |
| P284E-N287R-S322T-N338G-N388M-K394R-C430N-L432F-L434 W-S436N | +++ |
| P284E-N287R-S322T-V379I-N388M-K394R-C430N-L432F-L434W-S436N | +++ |
| P284E-N287R-S322T-V382L-N388M-K394R-C430N-L432F-L434W-S436N | +++ |
| P284E-N287R-S322T-N388Q-K394R-C430N-L432F-L434W-S436N | +++ |
| P284E-N287R-S322T-N388M-K394R-F426V-C430N-L432F-L434W-S436N | +++ |
| P284E-N287R-S322T-N388M-K394R-F426Y-C430N-L432F-L434W-S436N | +++ |
| P284E-N287R-S322T-N388M-K394R-F426W-C430N-L432F-L434W-S436N | +++ |
| V46I-P284E-N287R-S322T-N388M-K394R-C430N-L432F-L434W-S436N | +++ |
| P284E-N287R-S322T-N388M-K394R-C430N-L432F-L434W-S436W | +++ |
| P284E-N287R-S322T-N388M-K394R-C430N-L432F-L434W-S436N-C437M | +++ |
| P284E-N287R-S322T-N388M-K394R-C430N-L432F-L434W-S436N-E441D | +++ |
| P284E-N287R-S322T-N388M-K394R-C430N-L432F-L434W-S436N-E441T | +++ |
| P284E-N287R-S322T-N388M-K394R-C430N-L432F-L434W-S436N-E441H | +++ |
| P284E-N287R-S322T-N388M-K394R-C430N-L432F-L434W-S436N-H444L | +++ |
| P284E-N287R-S322T-N388M-K394R-C430N-L432F-L434W-S436N-K447W | +++ |
| P284E-N287R-S322T-N388M-K394R-C430N-L432F-L434W-S436N-K447F | +++ |
| F183Y-N285M-N287T-S322T-N388M-K394R-C430N-L432F-L434W-S436N | +++ |
| N285M-N287T-S322T-V333M-N388M-K394R-C430N-L432F-L434W-S436N | +++ |
| I283T-N285M-N287T-S322T-N388M-K394R-C430N-L432F-L434W-S436N | +++ |
| I283V-N285M-N287T-S322T-N388M-K394R-C430N-L432F-L434W-S436N | +++ |
| P284D-N285M-N287T-S322T-N388M-K394R-C430N-L432F-L434W-S436N | +++ |
| N285P-N287T-S322T-N388M-K394R-C430N-L432F-L434W-S436N | +++ |
| N285M-N287T-Q290E-S322T-N388M-K394R-C430N-L432F-L434W-S436N | +++ |
| N285M-N287T-I292L-S322T-N388M-K394R-C430N-L432F-L434W-S436N | +++ |
| N285M-N287T-I292V-S322T-N388M-K394R-C430N-L432F-L434W-S436N | +++ |
| N51D-N285M-N287T-S322T-N388M-K394R-C430N-L432F-L434W-S436N | +++ |
| N285M-N287T-C321T-S322T-N388M-K394R-C430N-L432F-L434 W-S436N | +++ |
| N285M-N287T-C321V-S322T-N388M-K394R-C430N-L432F-L434W-S436N | +++ |
| N285M-N287T-S322T-N332R-N388M-K394R-C430N-L432F-L434W-S436N | +++ |
| N285M-N287T-S322T-N332S-N388M-K394R-C430N-L432F-L434W-S436N | +++ |
| N285M-N287T-S322T-T366A-Q385W-N388M-K394R-C430N-L432F-L434W-S436N | +++ |
| N285M-N287T-S322T-Q385L-N388M-K394R-C430N-L432F-L434W-S436N | +++ |
| N285M-N287T-S322T-Q385P-N388M-K394R-C430N-L432F-L434W-S436N | +++ |
| N285M-N287T-S322T-Q385S-N388M-K394R-C430N-L432F-L434W-S436N | +++ |
| N285M-N287T-S322T-N388M-S389A-K394R-C430N-L432F-L434W-S436N | +++ |
| N285M-N287T-S322T-N388M-S389Q-K394R-C430N-L432F-L434W-S436N | +++ |
| N285M-N287T-S322T-N388M-L392Y-K394R-C430N-L432F-L434W-S436N | +++ |
| R7C-N285M-N287T-S322T-N388M-K394Q-C430N-L432F-L434W-S436N | +++ |
| N285M-N287T-S322T-N388M-K394H-C430N-L432F-L434 W-S436N | +++ |
| N285M-N287T-S322T-N388M-K394L-C430N-L432F-L434 W-S436N | +++ |
| N285M-N287T-S322T-N388M-K394R-F426H-C430N-L432F-L434 W-S436N | +++ |
| N285M-N287T-S322T-N388M-K394R-F426I-C430N-L432F-L434W-S436N | +++ |
| N285M-N287T-S322T-N388M-K394R-F426L-C430N-L432F-L434W-S436N | +++ |
| N285M-N287T-S322T-N388M-K394R-F426T-C430N-L432F-L434W-S436N | +++ |
| N285M-N287T-S322T-N388M-K394R-F426V-C430N-L432F-L434W-S436N | +++ |
| N285M-N287T-S322T-N388M-K394R-F426W-C430N-L432F-L434 W-S436N | +++ |
| N285M-N287T-S322T-N388M-K394R-C430A-L432F-L434 W-S436N | +++ |
| N285M-N287T-S322T-N388M-K394R-C430N-I431P-L432F-L434W-S436N | +++ |
| N285M-N287T-S322T-N388M-K394R-C430N-L432F-L434W-S436N-C437L | +++ |
| N285M-N287T-S322T-N388M-K394R-C430N-L432F-L434W-S436N-C437T | +++ |
| N285M-N287T-S322T-N388M-K394R-C430N-L432F-L434W-S436N-C437V | +++ |
| N285M-N287T-S322T-N388M-K394R-C430N-L432F-L434W-S436N-E441G | +++ |
| N285M-N287T-S322T-N388M-K394R-C430N-L432F-L434W-S436N-E441I | +++ |
| N285M-N287T-S322T-N388M-K394R-C430N-L432F-L434W-S436N-E441L | +++ |
| N285M-N287T-S322T-N388M-K394R-C430N-L432F-L434W-S436N-E441P | +++ |
| N285M-N287T-S322T-N388M-K394R-C430N-L432F-L434W-S436N-R443H | +++ |
| N285M-N287T-S322T-N388M-K394R-C430N-L432F-L434W-S436N-H444F | +++ |
| N285M-N287T-S322T-N388M-K394R-C430N-L432F-L434W-S436N-H444I | +++ |
| N285M-N287T-S322T-N388M-K394R-C430N-L432F-L434W-S436N-H444L | +++ |
| N285M-N287T-S322T-N388M-K394R-C430N-L432F-L434W-S436N-H444M | +++ |
| N285M-N287T-S322T-N388M-K394R-C430N-L432F-L434W-S436N-H444V | +++ |
| N285M-N287T-S322T-N388M-K394R-C430N-L432F-L434W-S436N-N445A | +++ |
| N285M-N287T-S322T-N388M-K394R-C430N-L432F-L434W-S436N-N445Q | +++ |
| N285M-N287T-S322T-N388M-K394R-C430N-L432F-L434W-S436N-N445T | +++ |
| N285M-N287T-S322T-N388M-K394R-C430N-L432F-L434W-S436N-R446D | +++ |
| N285M-N287T-S322T-N388M-K394R-C430N-L432F-L434W-S436N-R446G | +++ |
| N285M-N287T-S322T-N388M-K394R-C430N-L432F-L434W-S436N-R446P | +++ |
| N285M-N287T-S322T-N388M-K394R-C430N-L432F-L434W-S436N-K449W | +++ |
| N285M-N287T-S322T-N388M-K394R-C430N-L432F-L434W-S436N-K449N | +++ |
| E72K-P284E-N287Q-S322T-N388M-K394R-C430N-L432F-L434W-S436N | +++ |
| P284E-N287Q-S322T-N388M-K394R-C430N-L432F-L434W-S436N-K447H | +++ |
| P284E-N287Q-S322T-N332S-N388M-K394R-C430N-L432F-L434W-S436N | +++ |
| P284E-N287Q-S322T-N388M-K394R-C430N-L432F-L434W-S436M | +++ |
| P284E-N287Q-S322T-N388M-K394R-C430N-L432F-L434W-S436W | +++ |
| P284E-N287Q-S322T-T336H-N388M-K394R-C430N-L432F-L434W-S436N | +++ |
| P284E-N287Q-S322T-T336N-N388M-K394R-C430N-L432F-L434W-S436N | +++ |
| P284E-N287Q-S322T-T336Q-N388M-K394R-C430N-L432F-L434W-S436N | +++ |
| S89N-P284E-N287Q-S322T-A327S-N388M-K394R-C430N-L432F-L434W-S436N | +++ |
| I283C-P284E-N287Q-S322T-N388M-K394R-C430N-L432F-L434W-S436N | +++ |
| I283H-P284E-N287Q-S322T-N388M-K394R-C430N-L432F-L434W-S436N | +++ |
| I283S-P284E-N287Q-S322T-N388M-K394R-C430N-L432F-L434W-S436N | +++ |
| I283T-P284E-N287Q-S322T-N388M-K394R-C430N-L432F-L434W-S436N | +++ |
| I283V-P284E-N287Q-S322T-N388M-K394R-C430N-L432F-L434W-S436N | +++ |
| I283V-P284D-N287Q-S322T-N388M-K394R-C430N-L432F-L434W-S436N | +++ |
| P284E-N287Q-S322T-Q331G-N388M-K394R-C430N-L432F-L434W-S436N | +++ |
| P284E-N287Q-S322T-Q385F-N388M-K394R-C430N-L432F-L434W-S436N | +++ |
| P284E-N287Q-S322T-Q385P-N388M-K394R-C430N-L432F-L434W-S436N | +++ |
| P284E-N287Q-S322T-Q385R-N388M-K394R-C430N-L432F-L434W-S436N | +++ |
| P284E-N287Q-S322T-H384S-N388M-K394R-C430N-L432F-L434W-S436N | +++ |
| P284E-N287Q-S322T-N388M-K394G-C430N-L432F-L434W-S436N | +++ |
| P284E-N287Q-S322T-N388M-C430N-L432F-L434W-S436N | +++ |
| P284E-N287Q-S322T-N388M-K394L-C430N-L432F-L434W-S436N | +++ |
| R7C-P284E-N287Q-S322T-N388M-K394R-C430N-L432F-L434W-S436N | +++ |
| L48M-P284E-N287Q-S322T-N388M-K394R-C430N-L432F-L434W-S436N | +++ |
| P284E-N287Q-S322T-N388M-K394R-C430N-L432F-L434W-S436N-E440D | +++ |
| P284E-N287Q-S322T-N388M-K394R-C430N-L432F-L434W-S436N-E440I | +++ |
| P284E-N287Q-S322T-N388M-K394R-C430N-L432F-L434W-S436N-E441L | +++ |
| P284E-N287Q-S322T-N388M-K394R-C430N-L432F-L434W-S436N-E441T | +++ |
| P284E-N287Q-S322T-N388M-K394R-E428M-C430N-L432F-L434W-S436N | +++ |
| P284E-N287Q-S322T-N388M-K394R-E428T-C430N-L432F-L434W-S436N | +++ |
| P284E-N287Q-S322T-A327S-N388M-K394R-C430N-L432F-L434W-S436N | +++ |
| S189C-P284E-N287Q-S322T-N388M-K394R-C430N-L432F-L434W-S436N | +++ |
| P284E-N287Q-C321V-S322T-N388M-K394R-C430N-L432 F-L434W-S436N | +++ |
| P284E-N287Q-S322T-N388M-K394R-F426L-C430N-L432F-L434W-S436N | +++ |
| P284E-N287Q-S322T-N388M-K394R-F426Y-C430N-L432F-L434W-S436N | +++ |
| N285M-N287T-Q290E-S322T-N388M-K394R-C430A-L432 F-L434W-S436N | +++ |
| I283T-N285M-N287T-Q290E-S322T-N388M-K394R-C430A-L432F-L434W-S436N | +++ |
| N285P-N287T-Q290E-S322T-N388M-K394R-C430A-L432F-L434W-S436N | +++ |
| I283T-N285P-N287T-Q290E-S322T-N388M-K394R-C430A-L432F-L434W-S436N | +++ |
| P284D-N285P-N287T-Q290E-S322T-N388M-K394R-C430A-L432F-L434 W-S436N | +++ |
| I283T-N285P-N287T-S322T-N388M-K394R-C430N-L432F-L434W-S436N | +++ |
| I283T-N285M-N287T-Q290E-S322T-N388M-K394R-C430N-L432F-L434W-S436N | +++ |
| I283T-N285M-N287T-S322T-N388M-K394R-C430A-L432F-L434W-S436N | +++ |
| P284D-N285P-N287T-S322T-N388M-K394R-C430N-L432F-L434W-S436N | +++ |
| I283T-N285P-N287T-S322T-N388M-K394R-C430A-L432F-L434W-S436N | +++ |
| P284D-N285P-N287T-Q290E-S322T-N388M-K394R-C430N-L432F-L434 W-S436N | +++ |
| P284D-N285P-N287T-S322T-N388M-K394R-C430A-L432F-L434W-S436N | +++ |
| I283T-P284D-N285M-N287T-S322T-N388M-K394R-C430N-L432F-L434W-S436N | +++ |
| I283T-P284D-N285P-N287T-S322T-N388M-K394R-C430N-L432F-L434W-S436N | +++ |
| I283T-P284D-N285M-N287T-Q290E-S322T-N388M-K394R-C430N-L432F-L434W-S436N | +++ |
| I283T-P284D-N285M-N287T-S322T-N388M-K394R-C430A-L432F-L434W-S436N | +++ |
| I283T-P284D-N285P-N287T-Q290E-S322T-N388M-K394R-C430N-L432F-L434W-S436N | +++ |
| I283T-P284D-N285P-N287T-S322T-N388M-K394R-C430A-L432F-L434W-S436N | +++ |
| I283T-P284D-N285M-N287T-Q290E-S322T-N388M-K394R-C430A-L432F-L434W-S436N | +++ |
| I283T-P284D-N285P-N287T-Q290E-S322T-N388M-K394R-C430A-L432F-L434W-S436N | +++ |
| P284D-N285M-N287T-Q290E-S322T-N388M-K394R-C430N-L432F-L434W-S436N | +++ |
| P284D-N285M-N287T-S322T-N388M-K394R-C430A-L432F-L434W-S436N | +++ |
| N285P-N287T-Q290E-S322T-N388M-K394R-C430N-L432F-L434W-S436N | +++ |
| N285P-N287T-S322T-N388M-K394R-C430A-L432F-L434W-S436N | +++ |
| I283T-N285P-N287T-Q290E-S322T-N388M-K394R-C430N-L432F-L434W-S436N | +++ |
| I283T-P284D-N287R-S322T-N388M-K394R-C430N-L432F-L434W-S436N | +++ |
| I283T-P284D-N287P-S322T-N388M-K394R-C430N-L432F-L434W-S436N | +++ |
| I283T-P284D-N287R-S322T-N388M-C430N-L432F-L434W-S436N | +++ |
| I283T-P284D-E286W-N287P-S322T-N388M-K394R-C430N-L432F-L434W-S436N | +++ |
| I283T-P284D-N287P-S322T-N388M-C430N-L432F-L434W-S436N | +++ |
| I283C-P284E-E286W-N287P-S322T-N388M-K394R-C430N-L432F-L434W-S436N | +++ |
| I283T-P284E-E286W-N287P-S322T-N388M-K394R-C430N-L432F-L434 W-S436N | +++ |
| I283C-P284E-E286W-N287P-S322T-N388M-C430N-L432F-L434W-S436N | +++ |
| I283T-P284E-E286W-N287P-S322T-N388M-C430N-L432F-L434W-S436N | +++ |
| I283T-P284D-E286W-N287P-S322T-N388M-C430N-L432F-L434 W-S436N | +++ |
| I283C-P284E-N287R-S322T-N388M-K394R-C430N-L432F-L434W-S436N | +++ |
| I283C-P284D-N287P-S322T-N388M-K394R-C430N-L432F-L434W-S436N | +++ |
| I283C-P284D-N287R-S322T-N388M-C430N-L432F-L434W-S436N | +++ |
| I283C-P284D-N287P-S322T-N388M-C430N-L432F-L434W-S436N | +++ |
| I283C-P284E-N287R-S322T-N388M-C430N-L432F-L434W-S436N | +++ |
| I283T-P284E-N287R-S322T-N388M-C430N-L432F-L434W-S436N | +++ |
| P284D-N287R-S322T-N388M-C430N-L432F-L434W-S436N | +++ |
| I283C-P284E-E286W-N287R-S322T-N388M-C430N-L432F-L434W-S436N | +++ |
| I283C-P284E-N287P-S322T-N388M-C430N-L432F-L434W-S436N | +++ |
| I283T-P284E-N287P-S322T-N388M-C430N-L432F-L434 W-S436N | +++ |
| I283T-P284E-E286W-N287R-S322T-N388M-K394R-C430N-L432F-L434W-S436N | +++ |
| I283T-P284E-N287P-S322T-N388M-K394R-C430N-L432F-L434W-S436N | +++ |
| P284E-E286W-N287R-S322T-N388M-C430N-L432F-L434W-S436N | +++ |
| P284E-N287P-S322T-N388M-C430N-L432F-L434W-S436N | +++ |
| I283T-P284E-E286W-N287R-S322T-N388M-C430N-L432F-L434W-S436N | +++ |
| P284D-N287P-S322T-N388M-C430N-L432F-L434W-S436N | +++ |
| I283C-P284E-E286W-N287R-S322T-N388M-K394R-C430N-L432F-L434W-S436N | +++ |
| I283C-P284E-N287P-S322T-N388M-K394R-C430N-L432F-L434W-S436N | +++ |
| P284D-N287P-S322T-N388M-K394R-C430N-L432F-L434W-S436N | +++ |
| I283C-P284D-N287Q-S322T-N388M-K394R-C430N-L432F-L434 W-S436N | +++ |
| I283C-P284E-N287Q-S322T-N388M-C430N-L432F-L434W-S436N | +++ |
| I283C-P284D-N287Q-S322T-N388M-C430N-L432F-L434W-S436N | +++ |
| I283C-P284E-E286W-N287Q-S322T-N388M-C430N-L432F-L434W-S436N | +++ |
| I283T-P284D-N287Q-S322T-N388M-K394R-C430N-L432F-L434W-S436N | +++ |
| I283T-P284E-E286W-N287Q-S322T-N388M-K394R-C430N-L432F-L434 W-S436N | +++ |
| I283T-P284E-N287Q-S322T-N388M-C430N-L432F-L434W-S436N | +++ |
| I283T-P284D-N287Q-S322T-N388M-C430N-L432F-L434W-S436N | +++ |
| I283T-P284E-E286W-N287Q-S322T-N388M-C430N-L432F-L434 W-S436N | +++ |
| P284D-N287Q-S322T-N388M-C430N-L432F-L434W-S436N | +++ |
| P284E-E286W-N287Q-S322T-N388M-C430N-L432F-L434W-S436N | +++ |
| N285M-N287T-S322T-N388M-K394R-C430N-L432F-L434W-S436N-P458E | +++ |
| N285M-N287T-S322T-N388M-K394R-C430N-L432F-L434W-S436N-G462V | +++ |
| N285M-N287T-S322T-N388M-K394R-C430N-L432F-L434W-S436N-E459P | +++ |
| N285M-N287T-S322T-N388M-K394R-C430N-L432F-L434W-S436N-E459R | +++ |
| N285M-N287T-S322T-N388M-K394R-C430N-L432F-L434W-S436N-H461A | +++ |
| N285M-N287T-S322T-N388M-K394R-C430N-L432F-L434W-S436N-H461D | +++ |
| N285M-N287T-S322T-N388M-K394R-C430N-L432F-L434W-S436N-H461E | +++ |
| N285M-N287T-S322T-N388M-K394R-C430N-L432F-L434W-S436N-H461G | +++ |
| N285M-N287T-S322T-N388M-K394R-C430N-L432F-L434W-S436N-H461N | +++ |
| P45Q-N285M-N287T-S322T-N388M-K394R-C430N-L432F-L434W-S436N-H461S | +++ |
| N285M-N287T-S322T-N388M-K394R-C430N-L432F-L434W-S436N-G462A | +++ |
| N285M-N287T-S322T-N388M-K394R-C430N-L432F-L434W-S436N-G462C | +++ |
| N285M-N287T-S322T-N388M-K394R-C430N-L432F-L434W-S436N-G462P | +++ |
| N285M-N287T-S322T-N388M-K394R-C430N-L432F-L434W-S436N-G4621 | +++ |
| N285M-N287T-S322T-N388M-K394R-C430N-L432F-L434W-S436N-A463L | +++ |
| N285M-N287T-S322T-N388M-K394R-C430N-L432F-L434W-S436N-A463P | +++ |
| N285M-N287T-S322T-N388M-K394R-C430N-L432F-L434W-S436N-Y469D | +++ |
| N285M-N287T-S322T-N388M-K394R-C430N-L432F-L434W-S436N-Y469E | +++ |
| N285M-N287T-S322T-N388M-K394R-C430N-L432F-L434W-S436N-Y469S | +++ |
| N285M-N287T-S322T-N388M-K394R-C430N-L432F-L434W-S436N-Y469T | +++ |
| N285M-N287T-S322T-N388M-K394R-C430N-L432F-L434W-S436N-Y471F | +++ |
| H141L-N285M-N287T-S322T-N388M-K394R-C430N-L432F-L434W-S436N | +++ |
| N144S-N285M-N287T-S322T-N388M-K394R-C430N-L432F-L434W-S436N | +++ |
| R15H-N285M-N287T-S322T-N388M-K394R-C430N-L432F-L434W-S436N | +++ |
| P284E-N287Q-S322T-N388M-K394R-C430N-L432F-L434W-S436N-E459L | +++ |
| V46I-P284E-N287Q-S322T-N388M-K394R-C430N-L432F-L434W-S436N-N460V | +++ |
| P284E-N287Q-S322T-N388M-K394R-C430N-L432F-L434W-S436N-N460G | +++ |
| P284E-N287Q-S322T-N388M-K394R-C430N-L432F-L434W-S436N-N460I | +++ |
| P284E-N287Q-S322T-N388M-K394R-C430N-L432F-L434W-S436N-H461A | +++ |
| P284E-N287Q-S322T-N388M-K394R-C430N-L432F-L434W-S436N-H461G | +++ |
| P284E-N287Q-S322T-N388M-K394R-C430N-L432F-L434W-S436N-H461N | +++ |
| P284E-N287Q-S322T-N388M-K394R-C430N-L432F-L434W-S436N-H461T | +++ |
| P284E-N287Q-S322T-N388M-K394R-C430N-L432F-L434W-S436N-H461D | +++ |
| P284E-N287Q-S322T-N388M-K394R-C430N-L432F-L434W-S436N-A463E | +++ |
| P284E-N287Q-S322T-N388M-K394R-C430N-L432F-L434W-S436N-A463G | +++ |
| P284E-N287Q-S322T-N388M-K394R-C430N-L432F-L434W-S436N-F467W | +++ |
| P284E-N287Q-S322T-N388M-K394R-C430N-L432F-L434W-S436N-Y469D | +++ |
| P284E-N287Q-S322T-N388M-K394R-C430N-L432F-L434W-S436N-Y469N | +++ |
| P284E-N287Q-S322T-N388M-K394R-C430N-L432F-L434W-S436N-Y469S | +++ |
| P284E-N287Q-S322T-N388M-K394R-C430N-L432F-L434W-S436N-Y469T | +++ |
| P284E-N287Q-S322T-N388M-K394R-C430N-L432F-L434W-S436N-A470G | +++ |
| P284E-N287Q-S322T-N388M-K394R-C430N-L432F-L434W-S436N-Y471F | +++ |
| H141Q-P284E-N287Q-S322T-N388M-K394R-C430N-L432F-L434 W-S436N | +++ |
| H141S-P284E-N287Q-S322T-N388M-K394R-C430N-L432F-L434W-S436N | +++ |
| H141Y-P284E-N287Q-S322T-N388M-K394R-C430N-L432F-L434W-S436N | +++ |
| N285M-N287T-S322T-N388M-K394R-C430N-L432F-L434W-S436N-dP458 | +++ |
| N285M-N287T-S322T-N388M-K394R-C430N-L432F-L434W-S436N-dE459 | +++ |
| N285M-N287T-S322T-N388M-K394R-C430N-L432F-L434W-S436N-dN460 | +++ |
| N285M-N287T-S322T-N388M-K394R-C430N-L432F-L434W-S436N-dH461 | +++ |
| N285M-N287T-S322T-N388M-K394R-C430N-L432F-L434W-S436N-dG462 | +++ |
| N285M-N287T-S322T-N388M-K394R-C430N-L432F-L434W-S436N-dA463 | +++ |
| P284E-N287Q-S322T-N388M-K394R-C430N-L432F-L434W-S436N-dP458 | +++ |
| P284E-N287Q-S322T-N388M-K394R-C430N-L432F-L434W-S436N-dE459 | +++ |
| P284E-N287Q-S322T-N388M-K394R-C430N-L432F-L434W-S436N-dN460 | +++ |
| P284E-N287Q-S322T-N388M-K394R-C430N-L432F-L434W-S436N-dH461 | +++ |
| P284E-N287Q-S322T-N388M-K394R-C430N-L432F-L434 W-S436N-dG462 | +++ |
| P284E-N287Q-S322T-N388M-K394R-C430N-L432F-L434 W-S436N-dA463 | +++ |
| P284E-N287Q-S322T-N388M-K394R-C430N-L432F-L434W-S436N-H461G-Y469D | +++ |
| P284E-N287Q-S322T-N388M-K394R-C430N-L432F-L434W-S436N-A463G-Y469D | +++ |
| P284E-N287Q-S322T-N388M-K394R-C430N-L432F-L434W-S436N-H461G-A463G-Y469D | +++ |
| P284E-N287Q-S322T-N388M-K394R-C430N-L432F-L434W-S436N-N460G-H461G-A463G-Y469D | +++ |
| P284D-N287P-S322T-N388M-C430N-L432F-L434W-S436N-N460G | +++ |
| P284D-N287P-S322T-N388M-C430N-L432F-L434W-S436N-N460I | +++ |
| P284D-N287P-S322T-N388M-C430N-L432F-L434W-S436N-H461G | +++ |
| P284D-N287P-S322T-N388M-C430N-L432F-L434W-S436N-A463G | +++ |
| P284D-N287P-S322T-N388M-C430N-L432F-L434W-S436N-Y469D | +++ |
| P284D-N287P-S322T-N388M-C430N-L432F-L434W-S436N-N460G-H461G | +++ |
| P284D-N287P-S322T-N388M-C430N-L432F-L434W-S436N-N460G-A463G | +++ |
| P284D-N287P-S322T-N388M-C430N-L432F-L434W-S436N-N460G-Y469D | +++ |
| P284D-N287P-S322T-N388M-C430N-L432F-L434W-S436N-N460I-A463G | +++ |
| P284D-N287P-S322T-N388M-C430N-L432F-L434W-S436N-N460I-Y469D | +++ |
| P284D-N287P-S322T-N388M-C430N-L432F-L434W-S436N-H461G-A463G | +++ |
| P284D-N287P-S322T-N388M-C430N-L432F-L434W-S436N-H461G-Y469D | +++ |
| P284D-N287P-S322T-N388M-C430N-L432F-L434W-S436N-A463G-Y469D | +++ |
| P284D-N287P-S322T-N388M-C430N-L432F-L434W-S436N-N460G-H461G-A463G | +++ |
| P284D-N287P-S322T-N388M-C430N-L432F-L434W-S436N-N460G-H461G-Y469D | +++ |
| P284D-N287P-S322T-N388M-C430N-L432F-L434W-S436N-N460G-A463G-Y469D | +++ |
| P284D-N287P-S322T-N388M-C430N-L432F-L434W-S436N-N460I-H461G-Y469D | +++ |
| P284D-N287P-S322T-N388M-C430N-L432F-L434W-S436N-N460I-A463G-Y469D | +++ |
| P284D-N287P-S322T-N388M-C430N-L432F-L434W-S436N-H461G-A463G-Y469D | +++ |
| P284D-N287P-S322T-N388M-C430N-L432F-L434W-S436N-N460G-H461G-A463G-Y469D | +++ |
| P284D-N287P-S322T-N388M-C430N-L432F-L434W-S436N-N460I-H461G-A463G-Y469D | +++ |
| I283T-P284D-N287P-S322T-N388M-C430N-L432F-L434W-S436N-N460G | +++ |
| I283T-P284D-N287P-S322T-N388M-C430N-L432F-L434W-S436N-A463G | +++ |
| I283T-P284D-N287P-S322T-N388M-C430N-L432F-L434W-S436N-Y469D | +++ |
| I283T-P284D-N287P-S322T-N388M-C430N-L432F-L434W-S436N-N460G-A463G | +++ |
| I283T-P284D-N287P-S322T-N388M-C430N-L432F-L434W-S436N-N460G-Y469D | +++ |
| I283T-P284D-N287P-S322T-N388M-C430N-L432F-L434W-S436N-N460G-A463G-Y469D | +++ |
| I283T-P284D-N287P-S322T-N388M-C430N-L432F-L434W-S436N-N460I-H461G-Y469D | +++ |
| I283T-P284D-N287P-S322T-N388M-C430N-L432F-L434W-S436N-N460I-A463G-Y469D | +++ |
| I283T-P284D-N287P-S322T-N388M-C430N-L432F-L434W-S436N-H461G-A463G-Y469D | +++ |
| I283T-P284D-N287P-S322T-N388M-C430N-L432F-L434W-S436N-N460G-H461G-A463G-Y469D | +++ |
| I283T-P284D-N287P-S322T-N388M-C430N-L432F-L434W-S436N-N460I-H461G | +++ |
| I283T-P284D-N287P-S322T-N388M-C430N-L432F-L434W-S436N-N460I-A463G | +++ |
| I283T-P284D-N287P-S322T-N388M-C430N-L432F-L434W-S436N-N460I-Y469D | +++ |
| I283T-P284D-N287P-S322T-N388M-C430N-L432F-L434W-S436N-H461G-A463G | +++ |
| I283T-P284D-N287P-S322T-N388M-C430N-L432F-L434W-S436N-H461G-Y469D | +++ |
| I283T-P284D-N287P-S322T-N388M-C430N-L432F-L434W-S436N-A463G-Y469D | +++ |
| I283T-P284D-N287P-S322T-N388M-C430N-L432F-L434W-S436N-N460G-H461G-A463G | +++ |
| I283T-P284D-N287P-S322T-N388M-C430N-L432F-L434W-S436N-N460G-H461G-Y469D | +++ |
| I283T-P284D-N287Q-S322T-N388M-C430N-L432F-L434W-S436N-H461G | +++ |
| I283T-P284D-N287Q-S322T-N388M-C430N-L432F-L434W-S436N-A463G | +++ |
| I283T-P284D-N287Q-S322T-N388M-C430N-L432F-L434W-S436N-Y469D | +++ |
| I283T-P284D-N287Q-S322T-N388M-C430N-L432F-L434W-S436N-N460G-H461G | +++ |
| I283T-P284D-N287Q-S322T-N388M-C430N-L432F-L434W-S436N-N460G-A463G | +++ |
| I283T-P284D-N287Q-S322T-N388M-C430N-L432F-L434W-S436N-N460G-Y469D | +++ |
| I283T-P284D-N287Q-S322T-N388M-C430N-L432F-L434W-S436N-N460I-Y469D | +++ |
| I283T-P284D-N287Q-S322T-N388M-C430N-L432F-L434W-S436N-H461G-Y469D | +++ |
| I283T-P284D-N287Q-S322T-N388M-C430N-L432F-L434W-S436N-A463G-Y469D | +++ |
| I283T-P284D-N287Q-S322T-N388M-C430N-L432F-L434W-S436N-N460G-H461G-A463G | +++ |
| I283T-P284D-N287Q-S322T-N388M-C430N-L432F-L434W-S436N-N460G-H461G-Y469D | +++ |
| I283T-P284D-N287Q-S322T-N388M-C430N-L432F-L434W-S436N-N460I-H461G-Y469D | +++ |
| I283T-P284D-N287Q-S322T-N388M-C430N-L432F-L434W-S436N-N460G-H461G-A463G-Y469D | +++ |
| P284D-N287P-S322T-N388M-C430N-L432F-L434W-S436N-dG462 | +++ |
| I283T-P284D-N287P-S322T-N388M-C430N-L432F-L434W-S436N-dG462 | +++ |
| P284D-N287P-S322T-N388M-C430N-L432F-L434W-S436N-dA463 | +++ |
| I283T-P284D-N287P-S322T-N388M-C430N-L432F-L434W-S436N-dA463 | +++ |
| I283T-P284D-N287Q-S322T-N388M-C430N-L432F-L434W-S436N-dG462 | +++ |
| I283T-P284D-N287Q-S322T-N388M-C430N-L432F-L434W-S436N-dA463 | +++ |
| I283T-P284D-N285P-N287T-S322T-N388M-K394R-C430N-L432F-L434W-S436N-G462A | +++ |
| I283T-P284D-N285P-N287T-S322T-N388M-K394R-C430N-L432F-L434W-S436N-G462P | +++ |
| I283T-P284D-N285P-N287T-S322T-N388M-K394R-C430N-L432F-L434W-S436N-G462V | +++ |
| I283T-P284D-N285P-N287T-S322T-N388M-K394R-C430N-L432F-L434W-S436N-Y469D | +++ |
| I283T-P284D-N285P-N287T-S322T-N388M-K394R-C430N-L432F-L434W-S436N-G462A-A463P | +++ |
| I283T-P284D-N285P-N287T-S322T-N388M-K394R-C430N-L432F-L434W-S436N-G462A-Y469D | +++ |
| I283T-P284D-N285P-N287T-S322T-N388M-K394R-C430N-L432F-L434W-S436N-G462P-A463P | +++ |
| I283T-P284D-N285P-N287T-S322T-N388M-K394R-C430N-L432F-L434W-S436N-G462P-Y469D | +++ |
| N285M-N287T-S322T-N388M-K394R-C430N-L432F-L434W-S436N-G462V-A463P | +++ |
| N285M-N287T-S322T-N388M-K394R-C430N-L432F-L434W-S436N-G462V-Y469D | +++ |
| N285M-N287T-S322T-N388M-K394R-C430N-L432F-L434W-S436N-A463P-Y469D | +++ |
| N285M-N287T-S322T-N388M-K394R-C430N-L432F-L434W-S436N-G462A-A463P-Y469D | +++ |
| N285M-N287T-S322T-N388M-K394R-C430N-L432F-L434W-S436N-G462P-A463P-Y469D | +++ |
| I283T-P284D-N285P-N287T-S322T-N388M-K394R-C430N-L432F-L434W-S436N-G462V-A463P | +++ |
| I283T-P284D-N285P-N287T-S322T-N388M-K394R-C430N-L432F-L434W-S436N-G462V-Y469D | +++ |
| I283T-P284D-N285P-N287T-S322T-N388M-K394R-C430N-L432F-L434W-S436N-A463P-Y469D | +++ |
| I283T-P284D-N285P-N287T-S322T-N388M-K394R-C430N-L432F-L434W-S436N-G462A-A463P-Y469D | +++ |
| I283T-P284D-N285P-N287T-S322T-N388M-K394R-C430N-L432F-L434W-S436N-G462P-A463P-Y469D | +++ |
| I283T-P284D-N285P-N287T-S322T-N388M-K394R-C430N-L432F-L434W-S436N-G462V-A463P-Y469D | +++ |
| I283T-P284D-N285M-N287T-S322T-N388M-K394R-C430A-L432F-L434W-S436N-G462A | +++ |
| I283T-P284D-N285M-N287T-S322T-N388M-K394R-C430A-L432F-L434W-S436N-G462P | +++ |
| I283T-P284D-N285M-N287T-S322T-N388M-K394R-C430A-L432F-L434W-S436N-G462V | +++ |
| I283T-P284D-N285M-N287T-S322T-N388M-K394R-C430A-L432F-L434W-S436N-Y469D | +++ |
| I283T-P284D-N285M-N287T-S322T-N388M-K394R-C430A-L432F-L434W-S436N-G462A-A463P | +++ |
| I283T-P284D-N285M-N287T-S322T-N388M-K394R-C430A-L432F-L434W-S436N-G462A-Y469D | +++ |
| I283T-P284D-N285M-N287T-S322T-N388M-K394R-C430A-L432F-L434W-S436N-G462P-A463P | +++ |
| I283T-P284D-N285M-N287T-S322T-N388M-K394R-C430A-L432F-L434W-S436N-G462P-Y469D | +++ |
| I283T-P284D-N285M-N287T-S322T-N388M-K394R-C430A-L432F-L434W-S436N-G462V-A463P | +++ |
| I283T-P284D-N285M-N287T-S322T-N388M-K394R-C430A-L432F-L434W-S436N-G462V-Y469D | +++ |
| I283T-P284D-N285M-N287T-S322T-N388M-K394R-C430A-L432F-L434W-S436N-A463P-Y469D | +++ |
| I283T-P284D-N285M-N287T-S322T-N388M-K394R-C430A-L432F-L434W-S436N-G462A-A463P-Y469D | +++ |
| I283T-P284D-N285M-N287T-S322T-N388M-K394R-C430A-L432F-L434W-S436N-G462P-A463P-Y469D | +++ |
| I283T-P284D-N285M-N287T-S322T-N388M-K394R-C430A-L432F-L434W-S436N-G462V-A463P-Y469D | +++ |
| I283T-N285M-N287T-Q290E-S322T-N388M-K394R-C430A-L432F-L434W-S436N-G462A | +++ |
| I283T-N285M-N287T-Q290E-S322T-N388M-K394R-C430A-L432F-L434W-S436N-G462P | +++ |
| I283T-N285M-N287T-Q290E-S322T-N388M-K394R-C430A-L432F-L434W-S436N-G462V | +++ |
| I283T-N285M-N287T-Q290E-S322T-N388M-K394R-C430A-L432F-L434W-S436N-A463P | +++ |
| I283T-N285M-N287T-Q290E-S322T-N388M-K394R-C430A-L432F-L434W-S436N-Y469D | +++ |
| I283T-N285M-N287T-Q290E-S322T-N388M-K394R-C430A-L432F-L434W-S436N-G462A-A463P | +++ |
| I283T-N285M-N287T-Q290E-S322T-N388M-K394R-C430A-L432F-L434W-S436N-G462A-Y469D | +++ |
| I283T-N285M-N287T-Q290E-S322T-N388M-K394R-C430A-L432F-L434W-S436N-G462P-A463P | +++ |
| I283T-N285M-N287T-Q290E-S322T-N388M-K394R-C430A-L432F-L434W-S436N-G462P-Y469D | +++ |
| I283T-N285M-N287T-Q290E-S322T-N388M-K394R-C430A-L432F-L434W-S436N-G462V-A463P | +++ |
| I283T-N285M-N287T-Q290E-S322T-N388M-K394R-C430A-L432F-L434W-S436N-G462V-Y469D | +++ |
| I283T-N285M-N287T-Q290E-S322T-N388M-K394R-C430A-L432F-L434W-S436N-A463P-Y469D | +++ |
| I283T-N285M-N287T-Q290E-S322T-N388M-K394R-C430A-L432F-L434W-S436N-G462A-A463P-Y469D | +++ |
| I283T-N285M-N287T-Q290E-S322T-N388M-K394R-C430A-L432F-L434W-S436N-G462P-A463P-Y469D | +++ |
| I283T-N285M-N287T-Q290E-S322T-N388M-K394R-C430A-L432F-L434W-S436N-G462V-A463P-Y469D | +++ |
| I283T-N285P-N287T-Q290E-S322T-N388M-K394R-C430A-L432F-L434W-S436N-G462A | +++ |
| I283T-N285P-N287T-Q290E-S322T-N388M-K394R-C430A-L432F-L434W-S436N-G462P | +++ |
| I283T-N285P-N287T-Q290E-S322T-N388M-K394R-C430A-L432F-L434W-S436N-G462V | +++ |
| I283T-N285P-N287T-Q290E-S322T-N388M-K394R-C430A-L432F-L434W-S436N-A463P | +++ |
| I283T-N285P-N287T-Q290E-S322T-N388M-K394R-C430A-L432F-L434W-S436N-Y469D | +++ |
| I283T-N285P-N287T-Q290E-S322T-N388M-K394R-C430A-L432F-L434W-S436N-G462A-A463P | +++ |
| I283T-N285P-N287T-Q290E-S322T-N388M-K394R-C430A-L432F-L434W-S436N-G462A-Y469D | +++ |
| I283T-N285P-N287T-Q290E-S322T-N388M-K394R-C430A-L432F-L434W-S436N-G462P-A463P | +++ |
| I283T-N285P-N287T-Q290E-S322T-N388M-K394R-C430A-L432F-L434W-S436N-G462P-Y469D | +++ |
| I283T-N285P-N287T-Q290E-S322T-N388M-K394R-C430A-L432F-L434W-S436N-G462V-A463P | +++ |
| I283T-N285P-N287T-Q290E-S322T-N388M-K394R-C430A-L432F-L434W-S436N-G462V-Y469D | +++ |
| I283T-N285P-N287T-Q290E-S322T-N388M-K394R-C430A-L432F-L434W-S436N-A463P-Y469D | +++ |
| I283T-N285P-N287T-Q290E-S322T-N388M-K394R-C430A-L432F-L434W-S436N-G462A-A463P-Y469D | +++ |
| I283T-N285P-N287T-Q290E-S322T-N388M-K394R-C430A-L432F-L434W-S436N-G462P-A463P-Y469D | +++ |
| I283T-N285P-N287T-Q290E-S322T-N388M-K394R-C430A-L432F-L434W-S436N-G462V-A463P-Y469D | +++ |
| N285M-N287T-S322T-N388M-K394R-C430N-L432F-L434W-S436N-G462A-A463P | +++ |
| N285M-N287T-S322T-N388M-K394R-C430N-L432F-L434W-S436N-G462A-Y469D | +++ |
| N285M-N287T-S322T-N388M-K394R-C430N-L432F-L434W-S436N-G462P-A463P | +++ |
| N285M-N287T-S322T-N388M-K394R-C430N-L432F-L434W-S436N-G462P-Y469D | +++ |
| R7C-I283T-N285P-N287T-Q290E-S322T-Q385D-N388M-K394R-C430A-L432F-L434W-S436N-dG462-dA463 | +++ |
| I283T-N285P-N287T-Q290E-S322T-N388M-K394R-i421aP-C430A-L432F-L434W-S436N-dG462-dA463 | +++ |
| P184A-I283T-N285P-N287T-Q290E-S322T-N388M-K394R-C430A-L432F-L434W-S436N-dG462-dA463 | +++ |
| I283T-N285P-N287T-Q290E-S322T-N388M-K394C-C430A-L432F-L434W-S436N-dG462-dA463 | +++ |
| I283T-N285P-N287T-Q290E-S322T-N388M-K394R-R422S-C430A-L432F-L434W-S436N-dG462-dA463 | +++ |
| I283T-N285P-N287T-Q290E-S322T-N388M-K394R-H419P-C430A-L432F-L434W-S436N-dG462-dA463 | +++ |
| I283T-N285P-N287T-Q290E-S322T-N388M-K394R-C430A-L432F-L434W-S436N-N460L-dG462-dA463 | +++ |
| I283T-N285P-N287T-Q290E-S322T-N388M-K394R-C430A-L432F-L434W-S436N-S439L-dG462-dA463 | +++ |
| I283T-N285P-N287T-Q290E-S322T-N388M-K394R-C430A-L432F-L434W-S436N-P458L-dG462-dA463 | +++ |
| I283T-N285P-N287T-Q290E-S322T-N388M-K394R-C430A-L432F-L434W-S436N-L457A-dG462-dA463 | +++ |
| I283T-N285P-N287T-Q290E-S322T-N388M-K394R-C430A-L432F-L434W-S436N-P458C-dG462-dA463 | +++ |
| I283T-N285P-N287T-Q290E-S322T-N388M-K394R-C430A-L432F-L434W-S436N-E459T-dG462-dA463 | +++ |
| I283T-N285P-N287T-Q290E-S322T-N388M-K394R-C430A-L432F-L434W-S436N-P458M-dG462-dA463 | +++ |
| I283T-N285P-N287T-Q290E-S322T-N388M-K394R-C430A-L432F-L434W-S436N-P458I-dG462-dA463 | +++ |
| I283T-N285P-N287T-Q290E-S322T-N388M-K394R-C430A-L432F-L434W-S436N-E459G-dG462-dA463 | +++ |
| I283T-N285P-N287T-Q290E-S322T-N388M-K394R-C430A-L432F-L434W-S436N-P458R-dG462-dA463 | +++ |
| I283T-N285P-N287T-Q290E-S322T-N388M-K394R-C430A-L432F-L434W-S436N-P458Q-dG462-dA463 | +++ |
| I283T-N285P-N287T-Q290E-S322T-N388M-K394R-C430A-L432F-L434W-S436N-N460H-dG462-dA463 | +++ |
| I283T-N285P-N287T-Q290E-S322T-N388M-K394R-C430A-L432F-L434W-S436N-N460Y-dG462-dA463 | +++ |
| I283T-N285P-N287T-Q290E-S322T-N388M-K394R-C430A-L432F-L434W-S436N-N460I-dG462-dA463 | +++ |
| I283T-N285P-N287T-Q290E-S322T-N388M-K394R-C430A-L432F-L434W-S436N-N460M-dG462-dA463 | +++ |
| I283T-P284D-N287P-S322T-N388M-K423C-C430N-L432F-L434W-S436N-A463G-Y469D | ++++ |
| I283T-P284D-N285A-N287P-S322T-N388M-C430N-L432F-L434W-S436N-A463G-Y469D | ++++ |
| I283T-P284D-N285I-N287P-S322T-N388M-C430N-L432F-L434W-S436N-A463G-Y469D | ++++ |
| I283T-P284D-N285L-N287P-S322T-N388M-C430N-L432F-L434W-S436N-A463G-Y469D | ++++ |
| I283T-P284D-N287P-S322T-N388M-C430N-L432F-L434W-S436T-A463G-Y469D | ++++ |
| I283F-P284D-N287P-S322T-N388M-C430N-L432F-L434W-S436N-A463G-Y469D | ++++ |
| I283S-P284D-N287P-S322T-N388M-C430N-L432F-L434W-S436N-A463G-Y469D | ++++ |
| I283T-P284D-N287P-S288T-S322T-N388M-C430N-L432F-L434W-S436N-A463G-Y469D | ++++ |
| I283T-P284D-N287P-S322T-N388M-S429A-C430N-L432F-L434W-S436N-A463G-Y469D | ++++ |
| I283T-P284D-N287P-S322T-H384A-N388M-C430N-L432F-L434W-S436N-A463G-Y469D | ++++ |
| I283T-P284D-N287P-S322T-H384T-N388M-C430N-L432F-L434W-S436N-A463G-Y469D | ++++ |
| I283T-P284D-N287P-S322T-N388M-C430N-L432F-L434W-S436N-H444R-A463G-Y469D | ++++ |
| I283T-P284D-N287P-S322T-N388M-P393A-C430N-L432F-L434W-S436N-A463G-Y469D | ++++ |
| I283T-P284D-N287P-S322T-N388M-R422F-C430N-L432F-L434W-S436N-A463G-Y469D | ++++ |
| I283T-P284D-N287P-S322T-N388M-R422Q-C430N-L432F-L434W-S436N-A463G-Y469D | ++++ |
| I283T-P284D-N287P-S322T-N388M-R422V-C430N-L432F-L434W-S436N-A463G-Y469D | ++++ |
| I283T-P284D-N287P-S322T-N388M-E421T-C430N-L432F-L434W-S436N-A463G-Y469D | ++++ |
| I283T-P284D-N287P-S322T-N388M-E421V-C430N-L432F-L434W-S436N-A463G-Y469D | ++++ |
| I283T-P284D-N287P-S322T-N388M-C430N-L432F-L434W-S436N-E459P-A463G-Y469D | ++++ |
| I283T-P284D-N287P-S322T-N388M-C430N-L432F-L434W-S436N-A463G-Y469T | ++++ |
| I283T-P284D-E286C-N287P-S322T-N388M-C430N-L432F-L434W-S436N-A463G-Y469D | ++++ |
| I283T-P284D-N287P-S322T-N388M-C430N-L432F-L434W-S436N-A463F-Y469D | ++++ |
| I283T-P284D-N287P-S322T-N388M-C430N-L432F-L434W-S436N-G462P-A463G-Y469D | ++++ |
| I283T-P284D-N287P-S322T-N388M-i421aG-C430N-L432F-L434W-S436N-A463G-Y469D | ++++ |
| I283F-P284E-N287Q-S322T-N388M-K394R-C430N-L432F-L434W-S436N-dG462 | ++++ |
| P284E-N287Q-S322T-N388M-K394R-C430N-L432F-L434W-S436N-dG462-Y469S | ++++ |
| P284E-N287Q-S322T-N388M-K394R-C430N-L432F-L434W-S436N-dG462-Y469D | ++++ |
| I283L-N285M-N287T-S322T-A337T-N388M-K394R-C430N-L432F-L434W-S436N-dG462 | ++++ |
| N285M-N287T-S322T-N388M-K394R-C430N-L432F-L434W-S436N-N460G-dG462 | ++++ |
| N285M-N287T-S322T-N388M-K394R-C430N-L432F-L434W-S436N-dG462-A463M | ++++ |
| N285M-N287T-S322T-N388M-K394R-C430N-L432F-L434W-S436N-dG462-A463F | ++++ |
| N285M-N287T-S322T-N388M-K394R-C430N-L432F-L434W-S436N-dG462-A463V | ++++ |
| N285M-N287T-S322T-N388M-K394R-C430N-L432F-L434W-S436N-dG462-Y469S | ++++ |
| N285M-N287T-S322T-N388M-K394R-C430N-L432F-L434W-S436N-dG462-Y469T | ++++ |
| N285M-N287T-S322T-N388M-K394R-C430N-L432F-L434W-S436N-dG462-Y469N | ++++ |
| N285M-N287T-S322T-N388M-K394R-C430N-L432F-L434W-S436N-dG462-A470S | ++++ |
| N285M-N287T-S322T-N388M-K394R-C430N-L432F-L434W-S436N-dA463-A470S | ++++ |
| N285M-N287T-S322T-N388M-K394R-C430N-L432F-L434W-S436N-dA463-S465I | ++++ |
| N285M-N287T-S322T-N388M-K394R-C430N-L432F-L434W-S436N-dA463-S465V | ++++ |
| I283Q-P284E-N287Q-S322T-N388M-K394R-C430N-L432F-L434W-S436N-dA463 | ++++ |
| I283C-P284E-N287Q-S322T-N388M-K394R-C430N-L432F-L434W-S436N-dA463 | ++++ |
| 1283L-P284E-N287Q-S322T-N388M-K394R-C430N-L432F-L434 W-S436N-dA463 | ++++ |
| P284D-N287Q-S322T-N388M-K394R-C430N-L432F-L434 W-S436N-dA463 | ++++ |
| P284E-N287Q-L289H-S322T-N388M-K394R-C430N-L432F-L434W-S436N-dA463 | ++++ |
| P284E-N287Q-L289R-S322T-N388M-K394R-C430N-L432F-L434W-S436N-dA463 | ++++ |
| P284E-N287Q-S322T-N388M-K394R-R422H-C430N-L432F-L434W-S436N-dA463 | ++++ |
| P284E-N287Q-S322T-N388M-K394R-C430N-L432F-L434W-S436N-dA463-A470T | ++++ |
| P284E-N287Q-S322T-N388M-K394R-C430N-L432F-L434W-S436N-G462R-dA463 | ++++ |
| P284E-N287Q-S322T-N388M-K394R-C430N-L432F-L434 W-S436N-dA463-S465A | ++++ |
| P284E-N287Q-S322T-N388M-K394R-C430N-L432F-L434W-S436N-dA463-Y469T | ++++ |
| P284E-N287Q-S322T-N388M-K394R-C430N-L432F-L434W-S436N-dA463-Y469E | ++++ |
| P284E-N287Q-S322T-N388M-K394R-C430N-L432F-L434W-S436N-dA463-A470S | ++++ |
| I283T-N285P-E286D-N287T-Q290E-S322T-N388M-K394R-C430A-L432F-L434W-S436N-G462P-A463P | ++++ |
| I283T-N285P-N287T-L289Y-Q290E-S322T-N388M-K394R-C430A-L432F-L434W-S436N-G462P-A463P | ++++ |
| I283T-N285P-N287T-L289A-Q290E-S322T-N388M-K394R-C430A-L432F-L434W-S436N-G462P-A463P | ++++ |
| I283T-N285P-N287T-Q290E-S322T-Q365M-N388M-K394R-C430A-L432F-L434W-S436N-G462P-A463P | ++++ |
| I283T-N285P-N287T-Q290E-S322T-Q365S-N388M-K394R-C430A-L432F-L434W-S436N-G462P-A463P | ++++ |
| I283L-N285P-N287T-Q290E-S322T-N388M-K394R-C430A-L432F-L434W-S436N | ++++ |
| 1283H-P284D-N285M-N287T-S322T-N388M-K394R-C430A-L432F-L434W-S436N-G462P-A463P | ++++ |
| I283Q-P284D-N285M-N287T-S322T-N388M-K394R-C430A-L432F-L434W-S436N-G462P-A463P | ++++ |
| I283T-P284D-N285H-N287T-S322T-N388M-K394R-C430A-L432F-L434W-S436N-G462P-A463P | ++++ |
| I283T-P284D-N285V-N287T-S322T-N388M-K394R-C430A-L432F-L434W-S436N-G462P-A463P | ++++ |
| I283T-P284D-N285L-N287T-S322T-N388M-K394R-C430A-L432F-L434W-S436N-G462P-A463P | ++++ |
| I283T-P284D-N285T-N287T-S322T-N388M-K394R-C430A-L432F-L434W-S436N-G462P-A463P | ++++ |
| I283T-P284D-N285S-N287T-S322T-N388M-K394R-C430A-L432F-L434W-S436N-G462P-A463P | ++++ |
| I283T-P284D-N285M-E286N-N287T-S322T-N388M-K394R-C430A-L432F-L434W-S436N-G462P-A463P | ++++ |
| I283T-P284D-N285M-E286A-N287T-S322T-N388M-K394R-C430A-L432F-L434W-S436N-G462P-A463P | ++++ |
| I283T-P284D-N285M-E286G-N287T-S322T-N388M-K394R-C430A-L432F-L434W-S436N-G462P-A463P | ++++ |
| I283T-P284D-N285M-E286H-N287T-S322T-N388M-K394R-C430A-L432F-L434W-S436N-G462P-A463P | ++++ |
| I283T-P284D-N285M-E286D-N287T-S322T-N388M-K394R-C430A-L432F-L434W-S436N-G462P-A463P | ++++ |
| I283T-P284D-N285M-N287T-S288T-S322T-N388M-K394R-C430A-L432F-L434W-S436N-G462P-A463P | ++++ |
| I283T-P284D-N285M-N287T-L289Q-S322T-N388M-K394R-C430A-L432F-L434W-S436N-G462P-A463P | ++++ |
| I283T-P284D-N285M-N287T-L289R-S322T-N388M-K394R-C430A-L432F-L434W-S436N-G462P-A463P | ++++ |
| I283T-P284D-N285M-N287T-L289M-S322T-N388M-K394R-C430A-L432F-L434W-S436N-G462P-A463P | ++++ |
| I283T-P284D-N285M-N287T-S322T-N388M-K394R-C430T-L432F-L434W-S436N-G462P-A463P | ++++ |
| I283T-P284D-N287P-S322T-H384A-N388M-C430N-L432F-L434W-S436N-dG462 | ++++ |
| P25L-I283T-P284D-N287P-S322T-N388M-C430N-L432F-L434W-S436N-dG462 | ++++ |
| I283T-P284D-N287P-S322T-N388M-R422G-C430N-L432F-L434W-S436N-dG462 | ++++ |
| I283T-P284D-N287P-S322T-P368S-N388M-E421V-C430N-L432F-L434W-S436N-dG462 | ++++ |
| I283T-P284D-N287P-S322T-N388M-R422L-C430N-L432F-L434W-S436N-dG462 | ++++ |
| I283T-P284D-N287P-S322T-N388M-C430N-L432F-L434W-S436N-dG462-A463I | ++++ |
| I283T-P284D-N287P-S322T-N388M-C430N-L432F-L434W-S436N-dG462-A463T | ++++ |
| I283T-P284D-N287P-S322T-N388M-C430N-L432F-L434W-S436N-dG462-A463F | ++++ |
| I283T-P284D-N287P-S322T-N388M-C430N-L432F-L434W-S436N-H461V-dG462 | ++++ |
| I283T-P284D-N287P-S322T-N388M-C430N-L432F-L434W-S436N-dG462-S465V | ++++ |
| I283T-P284D-N287P-S322T-N388M-C430N-L432F-L434W-S436N-H461A-dG462 | ++++ |
| I283T-P284D-N287P-S322T-N388M-C430N-L432F-L434W-S436N-H461T-dG462 | ++++ |
| I283T-P284D-N287P-S322T-N388M-C430N-L432F-L434W-S436N-H461Y-dG462 | ++++ |
| I283T-P284D-N287P-S322T-N388M-C430N-L432F-L434W-S436N-dG462-A463S | ++++ |
| I283T-P284D-N287P-S322T-N388M-C430N-L432F-L434W-S436N-H461A-dG462-A463T | ++++ |
| I283T-P284D-N287P-S322T-N388M-C430N-L432F-L434W-S436N-H461A-dG462-S465V | ++++ |
| I283T-P284D-N287P-S322T-N388M-C430N-L432F-L434W-S436N-H461A-dG462-S465I | ++++ |
| I283T-P284D-N287P-S322T-N388M-C430N-L432F-L434W-S436N-H461Y-dG462-A463T | ++++ |
| I283T-P284D-N287P-S322T-N388M-C430N-L432F-L434W-S436N-H461Y-dG462-S465V | ++++ |
| I283T-P284D-N287P-S322T-N388M-C430N-L432F-L434W-S436N-H461V-dG462-A463T | ++++ |
| I283T-P284D-N287P-S322T-N388M-C430N-L432F-L434W-S436N-H461V-dG462-S465V | ++++ |
| I283T-P284D-N287P-S322T-N388M-C430N-L432F-L434W-S436N-dG462-A463T-S465V | ++++ |
| I283T-P284D-N287P-S322T-N388M-C430N-L432F-L434W-S436N-H461A-dG462-A463T-S465V | ++++ |
| I283T-P284D-N287P-S322T-N388M-C430N-L432F-L434W-S436N-H461Y-dG462-A463T-S465V | ++++ |
| I283T-P284D-N287P-S322T-N388M-C430N-L432F-L434W-S436N-H461V-dG462-A463T-S465V | ++++ |
| I283T-P284D-N287P-S322T-N388M-E421V-R422F-C430N-L432F-L434W-S436N-dG462 | ++++ |
| I283T-P284D-N287P-S322T-N388M-E421V-C430N-L432F-L434W-S436N-H461T-dG462 | ++++ |
| I283T-P284D-N287P-S322T-N388M-E421V-C430N-L432F-L434W-S436N-H461V-dG462 | ++++ |
| I283T-P284D-N287P-S322T-N388M-E421V-C430N-L432F-L434W-S436N-dG462-A463T | ++++ |
| I283T-P284D-N287P-S322T-N388M-R422F-C430N-L432F-L434W-S436N-H461T-dG462 | ++++ |
| I283T-P284D-N287P-S322T-N388M-R422F-C430N-L432F-L434W-S436N-H461V-dG462 | ++++ |
| I283T-P284D-N287P-S322T-N388M-R422F-C430N-L432F-L434W-S436N-dG462-A463T | ++++ |
| I283T-P284D-N287P-S322T-N388M-C430N-L432F-L434W-S436N-H461T-dG462-A463T | ++++ |
| I283T-P284D-N287P-S322T-N388M-E421V-R422F-C430N-L432F-L434W-S436N-H461T-dG462 | ++++ |
| I283T-P284D-N287P-S322T-N388M-E421V-R422F-C430N-L432F-L434W-S436N-H461V-dG462 | ++++ |
| I283T-P284D-N287P-S322T-N388M-E421V-R422F-C430N-L432F-L434W-S436N-dG462-A463T | ++++ |
| I283T-P284D-N287P-S322T-N388M-E421V-C430N-L432F-L434W-S436N-H461T-dG462-A463T | ++++ |
| I283T-P284D-N287P-S322T-N388M-E421V-C430N-L432F-L434W-S436N-H461V-dG462-A463T | ++++ |
| I283T-P284D-N287P-S322T-N388M-R422F-C430N-L432F-L434W-S436N-H461T-dG462-A463T | ++++ |
| I283T-P284D-N287P-S322T-N388M-R422F-C430N-L432F-L434W-S436N-H461V-dG462-A463T | ++++ |
| I283T-P284D-N287P-S322T-N388M-E421V-R422F-C430N-L432F-L434W-S436N-H461T-dG462-A463T | ++++ |
| I283T-P284D-N287P-S322T-N388M-E421V-R422F-C430N-L432F-L434W-S436N-H461V-dG462-A463T | ++++ |
| I283T-P284D-N287P-S322T-N388M-E421V-C430N-L432F-L434W-S436N-dG462 | ++++ |
| I283T-P284D-N287 P-S322T-N388M-R422F-C430N-L432F-L434W-S436N-dG462 | ++++ |
| I283T-N285P-N287T-Q290E-S322T-N388M-K394R-C430A-L432F-L434W-S436N-dH461 | ++++ |
| I283T-N285P-N287T-Q290E-S322T-N388M-K394R-C430A-L432F-L434W-S436N-dG462 | ++++ |
| I283T-N285P-N287T-Q290E-S322T-N388M-K394R-C430A-L432F-L434W-S436N-dA463 | ++++ |
| I283T-P284D-i284aP-N287P-S322T-N388M-C430N-L432F-L434W-S436N-dG462 | ++++ |
| I283T-P284D-N285M-N287T-S322T-N388M-K394R-C430A-L432F-L434W-S436N-C437M-G462P-A463P | ++++ |
| I283T-P284D-N285M-N287T-S322T-N388M-K394R-C430A-L432F-L434W-S436N-G462P-A463P-I464P | ++++ |
| I283T-P284D-N285M-N287T-L289H-S322T-N388M-K394R-C430A-L432F-L434W-S436N-G462P-A463P | ++++ |
| I283T-P284D-N285I-N287T-i287aS-S322T-N388M-K394R-C430A-L432F-L434W-S436N-G462P-A463P | ++++ |
| I283T-P284D-N285M-N287T-S322T-N388M-K394R-C430A-L432F-L434W-S436N-G462T-A463P | ++++ |
| I283T-N285P-N287T-Q290E-S322T-N388M-K394R-dR422-C430A-L432F-L434W-S436N-dG462 | ++++ |
| L223Y-I283T-N285P-N287T-Q290E-S322T-N388M-K394R-K423G-C430A-L432F-L434W-S436N-dG462 | ++++ |
| I283T-P284D-i284aP-N287P-S322T-N388M-E421V-C430N-L432F-L434W-S436N-dG462-A463T | ++++ |
| I283T-P284D-i284aP-N287P-S322T-N388M-E421V-C430N-L432F-L434W-S436N-H461T-dG462-A463T | ++++ |
| I283T-P284D-i284aP-N287P-S322T-N388M-E421V-C430N-L432F-L434W-S436N-H461V-dG462-A463T | ++++ |
| I283T-P284D-i284aP-N287P-S322T-N388M-E421V-R422F-C430N-L432F-L434W-S436N-dG462-A463T | ++++ |
| I283T-P284D-i284aP-N287P-S322T-N388M-E421V-R422F-C430N-L432F-L434W-S436N-H461T-dG462-A463T | ++++ |
| I283T-P284D-i284aP-N287P-S322T-N388M-E421V-R422F-C430N-L432F-L434W-S436N-H461T-dG462 | ++++ |
| I283T-P284D-i284aP-N287P-S322T-N388M-C430N-L432F-L434W-S436N-H461A-dG462-A463T | ++++ |
| I283T-P284D-i284aP-N287P-S322T-N388M-C430N-L432F-L434W-S436N-H461A-dG462 | ++++ |
| E126K-H141Q-I283T-N285P-N287T-Q290E-S322T-N388M-K394R-C430A-L432F-L434W-S436N-dG462 | ++++ |
| P184S-I283T-N285P-N287T-Q290E-S322T-N388M-K394R-C430A-L432F-L434W-S436N-dG462 | ++++ |
| L223Y-I283T-N285P-N287T-Q290E-S322T-N388M-K394R-C430A-L432F-L434W-S436N-dG462 | ++++ |
| V227Y-I283T-N285P-N287T-Q290E-S322T-N388M-K394R-C430A-L432F-L434W-S436N-dG462 | ++++ |
| I283L-N285P-N287T-Q290E-S322T-N388M-K394R-C430A-L432F-L434W-S436N-dG462 | ++++ |
| I283T-N285P-N287T-L289W-Q290E-S322T-N388M-K394R-C430A-L432F-L434W-S436N-dG462 | ++++ |
| I283T-N285P-N287T-L289R-Q290E-S322T-N388M-K394R-C430A-L432F-L434W-S436N-dG462 | ++++ |
| I283T-N285P-N287T-L289C-Q290E-S322T-N388M-K394R-C430A-L432F-L434W-S436N-dG462 | ++++ |
| I283T-N285P-N287T-Q290E-S322T-Q365T-N388M-K394R-C430A-L432F-L434W-S436N-dG462 | ++++ |
| I283T-N285P-N287T-Q290E-S322T-N388M-K394R-K423G-C430A-L432F-L434W-S436N-dG462 | ++++ |
| I283T-N285P-N287T-Q290E-S322T-N388M-K394R-C430V-L432F-L434W-S436N-dG462 | ++++ |
| I283T-N285P-N287T-Q290E-S322T-N388M-K394R-C430A-L432F-L434W-S436N-H444T-dG462 | ++++ |
| I283T-N285P-N287T-Q290E-S322T-N388M-K394R-C430A-L432F-L434W-S436N-H444R-dG462 | ++++ |
| I283T-N285P-N287T-Q290E-S322T-N388M-K394R-C430A-L432F-L434W-S436N-H444F-dG462 | ++++ |
| I283T-N285P-N287T-Q290E-S322T-N388M-K394R-C430A-L432F-L434W-S436N-N460G-dG462 | ++++ |
| I283T-N285P-N287T-Q290E-S322T-N388M-K394R-C430A-L432F-L434W-S436N-dG462-A463F | ++++ |
| I283T-N285P-N287T-Q290E-S322T-N388M-K394R-C430A-L432F-L434W-S436N-dG462-A470S | ++++ |
| I283T-N285P-N287T-Q290E-S322T-N388M-K394R-R422D-C430A-L432 F-L434W-S436N-dG462 | ++++ |
| I283T-N285P-N287T-Q290E-S322T-N388M-K394R-C430A-L432F-L434W-S436N-dG462-A463V | ++++ |
| I283T-N285P-N287T-Q290E-S322T-N388M-K394R-C430A-L432F-L434W-S436N-dG462-Y471F | ++++ |
| I35V-Q140L-I283T-N285P-N287T-Q290E-S322T-N388M-K394R-C430A-L432F-L434W-S436N-dG462 | ++++ |
| E224P-I283T-N285P-N287T-Q290E-S322T-N388M-K394R-C430A-L432F-L434W-S436N-dG462 | ++++ |
| E224Q-I283T-N285P-N287T-Q290E-S322T-N388M-K394R-C430A-L432F-L434W-S436N-dG462 | ++++ |
| L222V-I283T-N285P-N287T-Q290E-S322T-N388M-K394R-C430A-L432F-L434W-S436N-dG462 | ++++ |
| V227L-I283T-N285P-N287T-Q290E-S322T-N388M-K394R-C430A-L432F-L434W-S436N-dG462 | ++++ |
| I283T-N285P-E286A-N287T-Q290E-S322T-N388M-K394R-C430A-L432F-L434W-S436N-dG462 | ++++ |
| I283T-N285P-N287T-Q290E-S322T-R361K-N388M-K394R-C430A-L432F-L434W-S436N-dG462 | ++++ |
| I283T-N285P-N287T-Q290E-S322T-M355V-N388M-K394R-C430A-L432F-L434W-S436N-dG462 | ++++ |
| I283T-N285P-N287T-Q290E-S322T-M355T-N388M-K394R-C430A-L432F-L434W-S436N-dG462 | ++++ |
| I283T-N285P-N287T-Q290E-S322T-M355C-N388M-K394R-C430A-L432F-L434W-S436N-dG462 | ++++ |
| I283T-N285P-N287T-Q290E-S322T-N388M-K394R-E421W-C430A-L432F-L434W-S436N-dG462 | ++++ |
| I283T-N285P-N287T-Q290E-S322T-N388S-K394R-C430A-L432F-L434W-S436N-dG462 | ++++ |
| I283T-N285P-N287T-Q290E-S322T-N388M-K394H-C430A-L432F-L434W-S436N-dG462 | ++++ |
| I283T-N285P-N287T-Q290E-S322T-N388M-K394R-H419P-C430A-L432 F-L434W-S436N-dG462 | ++++ |
| I283T-N285P-N287T-Q290E-S322T-N388M-K394R-H419A-C430A-L432F-L434W-S436N-dG462 | ++++ |
| I283T-N285P-N287T-Q290E-S322T-N388M-K394R-E421L-C430A-L432F-L434W-S436N-dG462 | ++++ |
| I283T-N285P-N287T-Q290E-S322T-N388M-K394R-S429V-C430A-L432F-L434W-S436N-dG462 | ++++ |
| I283T-N285P-N287T-Q290E-S322T-N388M-K394R-C430A-I431K-L432F-L434W-S436N-dG462 | ++++ |
| I283T-N285P-N287T-Q290E-S322T-N388M-K394R-C430A-I431N-L432F-L434W-S436N-dG462 | ++++ |
| I283T-N285P-N287T-Q290E-S322T-N388M-K394R-C430A-I431P-L432F-L434W-S436N-dG462 | ++++ |
| I283T-N285P-N287T-Q290E-S322T-N388M-K394R-C430A-I431C-L432F-L434W-S436N-dG462 | ++++ |
| I283T-N285P-N287T-Q290E-S322T-N388M-K394R-C430A-I431T-L432F-L434W-S436N-dG462 | ++++ |
| I283T-N285P-N287T-Q290E-S322T-N388M-K394R-C430A-I431A-L432F-L434W-S436N-dG462 | ++++ |
| I283T-N285P-N287T-Q290E-S322T-N388M-K394R-C430S-L432F-L434W-S436N-dG462 | ++++ |
| I283T-N285P-N287T-Q290E-S322T-N388M-K394R-C430A-L432F-L434W-S436N-C437L-dG462 | ++++ |
| I283T-N285P-N287T-Q290E-S322T-N388M-K394R-C430A-L432F-L434W-S436N-Y438W-dG462 | ++++ |
| I283T-N285P-N287T-Q290E-S322T-N388M-K394R-C430A-L432F-L434W-S436N-C437T-dG462 | ++++ |
| I283T-N285P-N287T-Q290E-S322T-N388M-K394R-C430A-L432F-L434W-S436N-C437M-dG462 | ++++ |
| I283T-N285P-N287T-Q290E-S322T-N388M-K394R-C430A-L432F-L434W-S436N-C437I-dG462 | ++++ |
| I283T-N285P-N287T-Q290E-S322T-N388M-K394R-C430A-L432F-L434W-S436N-Y438M-dG462 | ++++ |
| I283T-N285P-N287T-Q290E-S322T-N388M-K394R-C430A-L432F-L434W-S436N-S439P-dG462 | ++++ |
| I283T-N285P-N287T-Q290E-S322T-N388M-K394R-C430A-L432F-L434W-S436N-K447T-dG462 | ++++ |
| D27N-I283T-N285P-N287T-Q290E-S322T-N388M-K394R-C430A-L432F-L434W-S436N-G448T-dG462 | ++++ |
| I283T-N285P-N287T-Q290E-S322T-N388M-K394R-C430A-L432F-L434W-S436N-G448C-dG462 | ++++ |
| I283T-N285P-N287T-Q290E-S322T-N388M-K394R-E421F-C430A-L432F-L434W-S436N-dG462 | ++++ |
| I283T-N285P-N287T-Q290E-S322T-N388M-K394R-i421aD-C430A-L432F-L434W-S436N-dG462 | ++++ |
| I283T-N285P-N287T-Q290E-S322T-N388M-K394R-i421aT-C430A-L432F-L434W-S436N-dG462 | ++++ |
| I283T-N285P-N287T-Q290E-S322T-N388M-K394R-i421aS-C430A-L432F-L434W-S436N-dG462 | ++++ |
| I283T-N285P-N287T-Q290E-S322T-N388M-K394R-i421aI-C430A-L432F-L434W-S436N-dG462 | ++++ |
| I283T-N285P-N287T-Q290E-S322T-N388M-K394R-i421aE-C430A-L432F-L434W-S436N-dG462 | ++++ |
| I283T-N285P-N287T-Q290E-S322T-N388M-K394R-i421aR-C430A-L432F-L434W-S436N-dG462 | ++++ |
| I283T-N285P-N287T-Q290E-S322T-N388M-K394R-i421aP-C430A-L432F-L434W-S436N-dG462 | ++++ |
| I283T-N285P-N287T-Q290E-S322T-N388M-K394R-i424aA-C430A-L432F-L434W-S436N-dG462 | ++++ |
| I283T-N285P-N287T-Q290E-S322T-N388M-K394R-i424aW-C430A-L432F-L434W-S436N-dG462 | ++++ |
| I283T-N285P-N287T-Q290E-S322T-N388M-K394R-i424aL-C430A-L432F-L434W-S436N-dG462 | ++++ |
| I283T-N285P-N287T-Q290E-S322T-N388M-K394R-i424aS-C430A-L432F-L434W-S436N-dG462 | ++++ |
| l283T-N285P-N287T-Q290E-S322T-N388M-K394R-i424aD-C430A-L432 F-L434W-S436N-dG462 | ++++ |
| I283T-N285P-N287T-Q290E-S322T-N388M-K394R-i426aL-C430A-L432F-L434W-S436N-dG462 | ++++ |
| I283T-N285P-N287T-Q290E-S322T-N388M-K394R-i429aN-C430A-L432F-L434W-S436N-dG462 | ++++ |
| I283T-N285P-N287T-Q290E-S322T-N388M-K394R-C430A-i430aC-L432F-L434W-S436N-dG462 | ++++ |
| I283T-N285P-N287T-Q290E-S322T-N388M-K394R-C430A-i430aG-L432F-L434W-S436N-dG462 | ++++ |
| l283T-N285P-N287T-Q290E-S322T-N388M-K394R-C430A-i430a N-L432 F-L434W-S436N-dG462 | ++++ |
| I283T-N285P-N287T-Q290E-S322T-N388M-K394R-C430A-i430aS-L432F-L434W-S436N-dG462 | ++++ |
| l283T-N285P-N287T-Q290E-S322T-N388M-K394R-C430A-i430a R-L432F-L434W-S436N-dG462 | ++++ |
| I283T-N285P-N287T-Q290E-S322T-N388M-K394R-C430A-i430aL-L432F-L434W-S436N-dG462 | ++++ |
| I283T-N285P-N287T-Q290E-S322T-N388M-K394R-C430A-i430a I-L432F-L434W-S436N-dG462 | ++++ |
| I283T-N285P-N287T-Q290E-S322T-N388M-K394R-C430A-i430aQ-L432F-L434W-S436N-dG462 | ++++ |
| I283T-N285P-N287T-Q290E-S322T-N388M-K394R-C430A-i430a H-L432F-L434W-S436N-dG462 | ++++ |
| I283T-N285P-N287T-Q290E-S322T-N388M-K394R-C430A-L432F-L434W-S436N-i438aD-dG462 | ++++ |
| I283T-N285P-N287T-Q290E-S322T-N388M-K394R-C430A-L432F-L434W-S436N-i443aR-dG462 | ++++ |
| I283T-N285P-N287T-Q290E-S322T-N388M-K394R-C430A-L432F-L434W-S436N-i443aV-dG462 | ++++ |
| I283T-N285P-N287T-Q290E-S322T-N388M-K394R-C430A-L432F-L434W-S436N-i443aL-dG462 | ++++ |
| I283T-N285P-N287T-Q290E-S322T-N388M-K394R-C430A-L432F-L434W-S436N-i443aT-dG462 | ++++ |
| I283T-N285P-N287T-Q290E-S322T-N388M-K394R-C430A-L432F-L434W-S436N-i444aH-dG462 | ++++ |
| I283T-N285P-N287T-Q290E-S322T-N388M-K394R-C430A-L432F-L434W-S436N-i444aP-dG462 | ++++ |
| I283T-N285P-N287T-Q290E-S322T-N388M-K394R-C430A-L432F-L434W-S436N-i444av-dG462 | ++++ |
| I283T-N285P-N287T-Q290E-S322T-N388M-K394R-C430A-L432F-L434W-S436N-i444aR-dG462 | ++++ |
| I283T-N285P-N287T-Q290E-S322T-N388M-K394R-C430A-L432F-L434W-S436N-i444aN-dG462 | ++++ |
| I283T-N285P-N287T-Q290E-S322T-N388M-K394R-C430A-L432F-L434W-S436N-i444aG-dG462 | ++++ |
| I283T-N285P-N287T-Q290E-S322T-N388M-K394R-C430A-L432F-L434W-S436N-i444aT-dG462 | ++++ |
| I283T-N285P-N287T-Q290E-S322T-N388M-K394R-C430A-L432F-L434W-S436N-i444aL-dG462 | ++++ |
| I283T-N285P-N287T-Q290E-S322T-N388M-K394R-C430A-L432F-L434W-S436N-i444aE-dG462 | ++++ |
| I283T-N285P-N287T-Q290E-S322T-N388M-K394R-C430A-L432F-L434W-S436N-i444aK-dG462 | ++++ |
| I283T-N285P-N287T-Q290E-S322T-N388M-K394R-C430A-L432F-L434W-S436N-i444aS-dG462 | ++++ |
| I283T-N285P-N287T-Q290E-S322T-N388M-K394R-C430A-L432F-L434W-S436N-i445aQ-dG462 | ++++ |
| I283T-N285P-N287T-Q290E-S322T-N388M-K394R-C430A-L432F-L434W-S436N-i447aY-dG462 | ++++ |
| I283T-N285P-N287T-Q290E-S322T-N388M-K394R-C430A-L432F-L434W-S436N-i447aL-dG462 | ++++ |
| I283T-N285P-N287T-Q290E-S322T-N388M-K394R-C430A-L432F-L434W-S436N-i447aK-dG462 | ++++ |
| I283T-N285P-N287T-Q290E-S322T-N388M-K394R-C430A-L432F-L434W-S436N-i447aP-dG462 | ++++ |
| I283T-N285P-N287T-Q290E-S322T-N388M-K394R-C430A-L432F-L434W-S436N-i448aS-dG462 | ++++ |
| I283T-N285P-N287T-Q290E-S322T-N388M-K394R-C430A-i431aG-L432F-L434W-S436N-dG462 | ++++ |
| I283T-N285P-N287T-Q290E-S322T-N388M-K394R-C430A-i431aL-L432F-L434W-S436N-dG462 | ++++ |
| I283T-N285P-N287T-Q290E-S322T-N388M-K394R-C430A-i431aN-L432F-L434W-S436N-dG462 | ++++ |
| I283T-N285P-N287T-Q290E-S322T-N388M-K394R-C430A-L432F-L434W-S436N-i449aS-dG462 | ++++ |
| L223Y-I283T-N285P-N287T-Q290E-S322T-N388M-K394R-E421F-C430A-L432F-L434W-S436N-dG462 | ++++ |
| I283T-N285P-N287T-Q290E-S322T-N388M-K394R-E421F-C430A-i430aG-L432F-L434W-S436N-dG462 | ++++ |
| I283T-N285P-N287T-Q290E-S322T-N388M-K394R-E421F-C430A-i430aL-L432F-L434W-S436N-dG462 | ++++ |
| I283T-N285P-N287T-Q290E-S322T-N388M-K394R-E421F-C430A-i430aN-L432F-L434W-S436N-dG462 | ++++ |
| I283T-N285P-N287T-Q290E-S322T-N388M-K394R-E421F-C430A-L432F-L434W-S436N-i448aS-dG462 | ++++ |
| L223Y-I283T-N285P-N287T-Q290E-S322T-N388M-K394R-E421F-K423G-C430A-L432F-L434W-S436N-dG462 | ++++ |
| L223Y-I283T-N285P-N287T-Q290E-S322T-N388M-K394R-K423G-C430A-i430aG-L432F-L434W-S436N-dG462 | ++++ |
| L223Y-I283T-N285P-N287T-Q290E-S322T-N388M-K394R-K423G-C430A-i430aL-L432F-L434W-S436N-dG462 | ++++ |
| L223Y-I283T-N285P-N287T-Q290E-S322T-N388M-K394R-K423G-C430A-i430aN-L432F-L434W-S436N-dG462 | ++++ |
| L223Y-I283T-N285P-N287T-Q290E-S322T-N388M-K394R-K423G-C430A-L432F-L434W-S436N-i448aS-dG462 | ++++ |
| L223Y-I283T-N285P-N287T-Q290E-S322T-N388M-K394R-i429aN-C430A-L432F-L434W-S436N-dG462 | ++++ |
| L223Y-I283T-N285P-N287T-Q290E-S322T-N388M-K394R-C430A-i430aG-L432F-L434W-S436N-dG462 | ++++ |
| L223Y-I283T-N285P-N287T-Q290E-S322T-N388M-K394R-i429aN-i429bG-C430A-L432F-L434W-S436N-dG462 | ++++ |
| I283T-N285P-N287T-Q290E-S322T-N388M-K394R-C430A-i430aG-L432F-L434W-S436N-C437L-dG462 | ++++ |
| L223Y-I283T-N285P-N287T-Q290E-S322T-N388M-K394R-C430A-i430aG-L432F-L434W-S436N-C437L-dG462 | ++++ |
| L223Y-I283T-N285P-N287T-Q290E-S322T-N388M-K394R-C430A-i430aN-L432F-L434W-S436N-dG462 | ++++ |
| L223Y-I283T-N285P-N287T-Q290E-S322T-N388M-K394R-C430A-L432F-L434W-S436N-C437L-dG462 | ++++ |
| L223Y-I283T-N285P-N287T-Q290E-S322T-N388M-K394R-C430A-i430aL-L432F-L434W-S436N-dG462 | ++++ |
| I283T-N285P-N287T-Q290E-S322T-N388M-K394R-C430A-i430aG-L432F-L434W-S436N-V451S-dG462 | ++++ |
| I283T-N285P-N287T-Q290E-S322T-N388M-K394R-i425aW-C430A-i430aG-L432F-L434W-S436N-dG462 | ++++ |
| H141C-I283T-N285P-N287T-Q290E-S322T-N388M-K394R-C430A-i430aG-L432F-L434W-S436N-dG462 | ++++ |
| L222V-I283T-N285P-N287T-Q290E-S322T-N388M-K394R-C430A-i430aG-L432F-L434W-S436N-dG462 | ++++ |
| I283T-N285P-N287T-Q290E-S322T-I330V-N388M-K394R-C430A-i430aG-L432F-L434W-S436N-dG462 | ++++ |
| I283T-N285P-N287T-Q290E-S322T-N388M-S389H-K394R-C430A-i430aG-L432F-L434W-S436N-dG462 | ++++ |
| H141C-I283T-N285P-N287T-Q290E-S322T-N388M-S389H-K394R-C430A-i430aG-L432F-L434W-S436N-dG462 | ++++ |
| I283T-N285P-N287T-Q290E-S322T-I330V-N388M-S389H-K394R-C430A-i430aG-L432F-L434W-S436N-dG462 | ++++ |
| H141C-I283T-N285P-N287T-Q290E-S322T-I330V-N388M-S389H-K394R-C430A-i430aG-L432F-L434W-S436N-dG462 | ++++ |
| I283T-N285P-N287T-Q290E-S322T-I330V-N388M-S389H-K394R-C430A-i430aG-L432F-L434W-S436N-V451S-dG462 | ++++ |
| E224P-I283T-N285P- N287T-Q290E-S322T-N388 M-K394R-C430A-i430aG-L432 F-L434W-S436N-dG462 | ++++ |
| L222V-E224P-I283T-N285P-N287T-Q290E-S322T-N388M-K394R-C430A-i430aG-L432F-L434W-S436N-dG462 | ++++ |
| I283T-N285P-N287T-Q290E-S322T-N388M-S389H-K394R-C430A-i430aG-L432F-L434W-S436N-I453L-dG462 | ++++ |
| E224A-I283T-N285P-N287T-Q290E-S322T-N388M-S389H-K394R-C430A-i430aG-L432F-L434W-S436N-dG462 | ++++ |
| E224P-I283T-N285P-N287T-Q290E-S322T-N388M-S389H-K394R-C430A-i430aG-L432F-L434W-S436N-dG462 | ++++ |
| I283T-P284D-i284aP-N287P-S322T-N388M-E421V-i421aR-C430N-L432F-L434W-S436N-H461T-dG462-A463T | ++++ |
| I283T-P284D-i284aP-N287P-S322T-N388M-E421V-i422aK-C430N-L432F-L434W-S436N-H461T-dG462-A463T | ++++ |
| I283T-P284D-i284aP-N287P-S322T-N388M-E421V-C430N-I431V-L432F-L434W-S436N-H461T-dG462-A463T | ++++ |
| I283T-P284D-i284aP-N287P-S322T-Q365I-N388M-E421V-C430N-L432F-L434W-S436N-H461T-dG462-A463T | ++++ |
| I283T-P284D-i284aP-N287P-S322T-Q385G-N388M-E421V-C430N-L432F-L434W-S436N-H461T-dG462-A463T | ++++ |
| I283T-P284D-i284aP-N287P-S322T-Q385L-N388M-E421V-C430N-L432F-L434W-S436N-H461T-dG462-A463T | ++++ |
| I283T-P284D-i284aP-N287P-S322T-Q385S-N388M-E421V-C430N-L432F-L434W-S436N-H461T-dG462-A463T | ++++ |
| I283T-P284D-i284aP-N287P-S322T-Q385T-N388M-E421V-C430N-L432F-L434W-S436N-H461T-dG462-A463T | ++++ |
| I283T-P284D-i284aP-N287P-S322T-H384G-N388M-E421V-C430N-L432F-L434W-S436N-H461T-dG462-A463T | ++++ |
| I283T-P284D-i284aP-N287P-S322T-H384L-N388M-E421V-C430N-L432F-L434W-S436N-H461T-dG462-A463T | ++++ |
| I283T-P284D-i284aP-N287P-L289R-S322T-N388M-E421V-C430N-L432F-L434W-S436N-H461T-dG462-A463T | ++++ |
| I283T-P284D-i284aP-E286A-N287P-S322T-N388M-E421V-C430N-L432F-L434W-S436N-H461T-dG462-A463T | ++++ |
| I283T-P284D-i284aP-E286S-N287P-S322T-N388M-E421V-C430N-L432F-L434W-S436N-H461T-dG462-A463T | ++++ |
| I283T-P284D-i284aP-N287P-S322T-N388M-E421V-C430N-L432F-L434W-S436N-H461T-dG462-A463T-D468L | ++++ |
| I283T-P284D-i284aP-N287P-S322T-N388M-E421V-C430N-L432F-L434W-S436N-G448C-H461T-dG462-A463T | ++++ |
| I283T-P284D-i284aP-N287P-S322T-N388M-E421V-C430N-L432F-L434W-S436N-G448E-H461T-dG462-A463T | ++++ |
| I283T-P284D-i284aP-N287P-S322T-N388M-E421V-C430N-L432F-L434W-S436N-G448V-H461T-dG462-A463T | ++++ |
| I283T-P284D-i284aP-N287P-S322T-N388M-E421V-C430N-L432F-L434W-S436N-H461T-dG462-A463T-Y469A | ++++ |
| I283T-P284D-i284aP-N287P-S322T-N388M-E421V-C430N-L432F-L434W-S436N-H461T-dG462-A463T-Y469H | ++++ |
| I283T-P284D-i284aP-N287P-S322T-N388M-E421V-C430N-L432F-L434W-S436N-C437F-H461T-dG462-A463T | ++++ |
| I283T-P284D-i284aP-N287P-S322T-N388M-E421V-C430N-L432F-L434W-S436N-C437G-H461T-dG462-A463T | ++++ |
| I283T-P284D-i284aP-N287P-S322T-N388M-E421V-C430N-i430aR-L432F-L434W-S436N-H461T-dG462-A463T | ++++ |
| I283T-P284D-i284aP-N287P-S322T-N388M-E421V-C430N-I431M-L432F-L434W-S436N-H461T-dG462-A463T | ++++ |
| P45A-I283T-P284D-i284aP-N287P-S322T-N388M-E421V-C430N-L432F-L434W-S436N-i443aG-i443bA-H461T-dG462-A463T | ++++ |
| I283T-P284D-i284aP-N287P-S322T-N388M-E421V-C430N-L432F-L434W-S436N-H461T-G462R-i462aA-A463T | ++++ |
| R15L-I283T-P284D-i284aP-N287P-S322T-K378M-N388M-E421V-C430N-L432F-L434W-S436N-H461T-dG462-A463T | ++++ |
| V272L-I283T-P284D-i284aP-N287P-S322T-N388M-E421V-C430N-L432F-L434W-S436N-H461T-dG462-A463T | ++++ |
| V272R-I283T-P284D-i284aP-N287P-S322T-N388M-E421V-C430N-L432F-L434W-S436N-H461T-dG462-A463T | ++++ |
| V272I-I283T-P284D-i284aP-N287P-S322T-N388M-E421V-C430N-L432F-L434W-S436N-H461T-dG462-A463T | ++++ |
| I283T-P284D-i284aP-N287P-S322T-N388M-E421V-C430N-L432F-L434W-S436N-C437F-G448E-H461T-dG462-A463T | ++++ |
| I283T-P284D-i284aP-N287P-S322T-N388M-E421V-C430N-L432F-L434W-S436N-C437F-G448E-H461T-G462R-i462aA-A463T | ++++ |
| I283T-P284D-i284aP-N287P-S322T-N388M-E421V-C430N-L432F-L434W-S436N-C437F-H461T-G462R-i462aA-A463T | ++++ |
| I283T-P284D-i284aP-N287P-S322T-N388M-E421V-C430N-L432F-L434W-S436N-G448E-H461T-G462R-i462aA-A463T | ++++ |
| V272R-I283T-P284D-i284aP-N287P-S322T-N388M-E421V-C430N-L432F-L434W-S436N-C437F-H461T-dG462-A463T | ++++ |
| V272R-I283T-P284D-i284aP-N287P-S322T-N388M-E421V-C430N-L432F-L434W-S436N-C437F-G448E-H461T-dG462-A463T | ++++ |
| V272R-I283T-P284D-i284aP-N287P-S322T-N388M-E421V-C430N-L432F-L434W-S436N-C437F-G448E-H461T-G462R-i462aA-A463T | ++++ |
| V272R-I283T-P284D-i284aP-N287P-S322T-N388M-E421V-C430N-L432F-L434W-S436N-C437F-H461T-G462R-i462aA-A463T | ++++ |
| V272R-I283T-P284D-i284aP-N287P-S322T-N388M-E421V-C430N-L432F-L434W-S436N-G448E-H461T-dG462-A463T | ++++ |
| V272R-I283T-P284D-i284aP-N287P-S322T-N388M-E421V-C430N-L432F-L434W-S436N-G448E-H461T-G462R-i462aA-A463T | ++++ |
| V272R-I283T-P284D-i284aP-N287P-S322T-N388M-E421V-C430N-L432F-L434W-S436N-H461T-G462R-i462aA-A463T | ++++ |
| V272I-I283T-P284D-i284aP-N287P-S322T-N388M-E421V-C430N-L432F-L434W-S436N-C437F-H461T-G462R-i462aA-A463T | ++++ |
| V272K-I283T-P284D-i284aP-N287P-S322T-N388M-E421V-C430N-L432F-L434W-S436N-C437F-H461T-G462R-i462aA-A463T | ++++ |
| A33P-I283T-P284D-i284aP-N287P-S322T-N388M-E421V-C430N-L432F-L434W-S436N-C437F-H461T-G462R-i462aA-A463T | ++++ |
| I283T-P284D-i284aP-N287P-S322T-H384S-N388M-E421V-C430N-L432F-L434W-S436N-C437F-H461T-G462R-i462aA-A463T | ++++ |
| I283T-P284D-i284aP-N287P-S322T-N388M-I418V-E421V-C430N-L432F-L434W-S436N-C437F-H461T-G462R-i462aA-A463T | ++++ |
| K43E-I283T-P284D-i284aP-N287P-S322T-N388M-E421V-C430P-L432F-L434W-S436N-C437F-H461T-G462R-i462aA-A463T | ++++ |
| I283T-P284D-i284aP-N287P-S322T-N388M-E421V-C430R-L432F-L434W-S436N-C437F-H461T-G462R-i462aA-A463T | ++++ |
| I283T-P284D-i284aP-N287P-S322T-N388M-E421V-C430N-L432F-L434W-S436N-C437F-Y438L-H461T-G462R-i462aA-A463T | ++++ |
| I283T-P284D-i284aP-N287P-S322T-N388M-E421V-C430N-L432F-L434W-S436Q-C437F-H461T-G462R-i462aA-A463T | ++++ |
| I283T-P284D-i284aP-N287P-S322T-N388M-E421V-C430N-L432F-L434W-S436N-C437F-E441V-H461T-G462R-i462aA-A463T | ++++ |
| I283T-P284D-i284aP-N287P-S322T-N388M-E421V-C430N-L432F-L434W-S436N-C437F-i444aT-H461T-G462R-i462aA-A463T | ++++ |
| I283T-P284D-i284aP-N287P-S322T-N388M-E421V-C430R-L432F-L434W-S436N-C437F-Y438L-H461T-G462R-i462aA-A463T | ++++ |
| V272I-I283T-P284D-i284aP-N287P-S322T-N388M-E421V-C430R-L432F-L434W-S436N-C437F-H461T-G462R-i462aA-A463T | ++++ |
| V272I-I283T-P284D-i284aP-N287P-S322T-N388M-E421V-C430R-L432F-L434W-S436N-C437F-Y438L-H461T-G462R-i462aA-A463T | ++++ |
| V272I-I283T-P284D-i284aP-N287P-S322T-N388M-E421V-C430N-L432F-L434W-S436N-C437F-Y438L-H461T-G462R-i462aA-A463T | ++++ |
| V272R-I283T-P284D-i284aP-N287P-S322T-N388M-l418V-E421V-C430N-L432F-L434W-S436N-G448E-H461T-G462R-i462aA-A463T | ++++ |
| G17S-V272R-I283T-P284D-i284aP-N287P-S322T-N388M-E421K-C430N-L432F-L434W-S436N-G448E-H461T-G462R-i462aA-A463T | ++++ |
| V272R-I283T-P284D-i284aP-N287P-S322T-N388M-E421V-E428T-C430N-L432F-L434W-S436N-G448E-H461T-G462R-i462aA-A463T | ++++ |
| V272R-I283T-P284D-i284aP-N287P-S322T-N388M-E421V-C430N-L432F-L434W-S436N-E441H-G448E-H461T-G462R-i462aA-A463T | ++++ |
| V46A-V272R-I283T-P284D-i284aP-N287P-S322T-N388M-E421V-C430N-L432F-L434W-S436N-G448E-i458aG-H461T-G462R-i462aA-A463T | ++++ |
| S39G-V272R-I283T-P284D-i284aP-N287P-S322T-N388M-E421V-F426L-E428T-C430N-L432F-L434W-S436N-G448EH461T-G462R-i462aA-A463T | ++++ |
| V272R-I283T-P284D-i284aP-N287P-S322T-N388M-E421V-E428T-C430N-L432F-L434W-S436N-K447l-G448EH461T-G462R-i462aA-A463T | ++++ |
| E224P-I283T-N285P-N287T-Q290E-S322T-N388M-S389H-K394R-C430A-i430aG-L432F-L434W-S436N-I453L-dG462 | ++++ |

As regards the determination of sequence identity, the following should apply: When the sequences which are compared do not have the same length, the degree of identity either refers to the percentage of amino acid residues in the shorter sequence which are identical to amino acid residues in the longer sequence or to the percentage of amino acid residues in the longer sequence which are identical to amino acid residues in the shorter sequence. Preferably, it refers to the percentage of amino acid residues in the shorter sequence which are identical to amino acid residues in the longer sequence. The degree of sequence identity can be determined according to methods well known in the art using preferably suitable computer algorithms such as CLUSTAL.

When using the Clustal analysis method to determine whether a particular sequence is, for instance, at least 60% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.

In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.
Preferably, the degree of identity is calculated over the complete length of the sequence.

### Increasing the pool of coenzyme A in the organism or microorganism

It has been disclosed in WO 2020/188033 that increasing the pool of coenzyme A (CoA) can result in an increased production of isobutene. In WO 2020/188033, increased pools of CoA are achieved due to:
(i) an increased uptake of pantothenate; and/or
(ii) an increased conversion of pantothenate into CoA.

Accordingly, the recombinant organism or microorganism according to the invention may further be improved by increasing the uptake of pantothenate and/or by increasing the conversion of pantothenate into CoA. The transporters and enzymes to achieve an increased uptake of pantothenate and/or an increased conversion of pantothenate into CoA are disclosed in WO 2020/188033, which is fully incorporated herein by reference.

### The organism or microorganism

Within the present invention, the organism may be any organism, preferably any organism that is suitable for the use in biotechnological processes at an industrial scale. Preferably, the organism is an organism that can be used for the biotechnological production of isobutene or its precursor molecule 3-methylcrotonic acid at an industrial scale. More preferably, the organism according to the invention is a microorganism.

The term "microorganism" in the context of the present invention refers to bacteria, as well as to fungi, such as yeasts, and also to algae and archaea. In one preferred embodiment, the microorganism is a bacterium. In principle any bacterium can be used. Preferred bacteria to be employed in the present invention are bacteria of the genus *Bacillus, Clostridium, Corynebacterium, Pseudomonas, Zymomonas* or *Escherichia.* In a particularly preferred embodiment, the bacterium belongs to the genus *Escherichia* and even more preferred to the species *Escherichia coli.* In another preferred embodiment, the bacterium belongs to the species *Pseudomonas putida* or to the species *Zymomonas mobilis* or to the species *Corynebacterium glutamicum* or to the species *Bacillus subtilis.* It is also possible to employ an extremophilic bacterium such as *Thermus thermophilus,* or anaerobic bacteria from the family *Clostridiae.*

In another particularly preferred embodiment, the microorganism is a microorganism which is capable using carbon monoxide (CO) and gaseous substrates comprising CO like, e.g., syngas, as the source of carbon and energy. Syngas or synthesis gas is a mixture of CO and CO₂ as well as H₂. Thus, in a particularly preferred embodiment, the microorganism is a C1-fixing microorganism. As mentioned above, corresponding naturally occurring (or genetically modified) microorganisms that are capable of utilizing CO and converting it into acetyl-CoA are known in the art. These organisms are often referred to as acetogenic microorganisms (sometimes also termed carboxydotrophic, acetogenic microorganisms) and are referred to as "C1-fixing microorganisms" herein. These microorganisms use the Wood-Ljungdahl patway to fix CO and convert it into acetyl-CoA. Examples of such microorganisms belong to the family *Clostridiae* and are, e.g., described in WO 2009/094485; WO 2012/05905; WO 2013/180584; US 2011/0236941; PNAS 107(29):13087-13092 (2010); Current Opinion in Biotechnology 23:364-381 (2012); Applied and Environmental Microbiology 77(15):5467-5475 (2011). The use of C1-fixing microorganisms is extensively discussed in WO 2020/188033, which is incorporated herein in its entirety.

The skilled person is capable of identifying aldehyde lyases and aldehyde dehydrogenases in the above-mentioned species. Further, the skilled person is capable of identifying homologs of the *E. coli* genes *deoC* and *puuC* in other bacterial species, for example in any one of the species listed above.

In another preferred embodiment the microorganism is a fungus, more preferably a fungus of the genus *Saccharomyces, Schizosaccharomyces, Aspergillus, Trichoderma, Kluyveromyces, Clostridium* or *Pichia* and even more preferably of the species *Saccharomyces cerevisiae, Schizosaccharomyces pombe, Aspergillus niger, Trichoderma reesei, Kluyveromyces marxianus, Kluyveromyces lactis, Pichia pastoris, Pichia torula* or *Pichia utilis.*

In another embodiment, the present invention makes use of a photosynthetic microorganism expressing at least one enzyme for the conversion according to the invention as described above. Preferably, the microorganism is a photosynthetic bacterium, or a microalgae. In a further embodiment the microorganism is an algae, more preferably an algae belonging to the diatomeae.

It is also conceivable to use in accordance with the present invention a combination of microorganisms, wherein different microorganisms express different enzymes as described above.

In a preferred embodiment, the organism, preferably the microorganism, of the present invention is an organism which is capable of consuming CO and/or syngas. In another preferred embodiment, the organism, preferably the microorganism, of the present invention is an organism which is capable of consuming a mixture of CO and/or CO₂ as well as H₂.

In another embodiment, said organism and/or microorganism is genetically modified in order to be capable of consuming glucose, fructose, xylose, mannose and/or CO (or syngas) and/or genetically modified in order to increase the organism's and/or microorganism's capability of consuming glucose, fructose, xylose, mannose and/or CO (or syngas). Corresponding genetic modifications are known in the art.

In another preferred embodiment, the organism, preferably the microorganism, of the present invention is an organism which is capable of consuming sugar through a Phosphotransferase Transport System (PTS).

In a preferred embodiment, the organism, preferably the microorganism, of the present invention is an organism which is capable of consuming sugar through a non-Phosphotransferase Transport System (non-PTS).

Organisms and/or microorganisms which are capable of consuming sugar through a Phosphotransferase Transport System (PTS) and/or through a non-Phosphotransferase Transport System (non-PTS) are known in the art.

In another embodiment, said organism and/or microorganism is genetically modified in order to be capable of consuming sugar through a Phosphotransferase Transport System (PTS) or through a non-Phosphotransferase Transport System (non-PTS). In another preferred embodiment, said organism and/or microorganism is genetically modified in order to increase the organism's and/or microorganism's capability of consuming sugar through a Phosphotransferase Transport System (PTS) or through a non-Phosphotransferase Transport System (non-PTS). Corresponding genetic modifications are known in the art.

In another preferred embodiment, the organism, preferably the microorganism, of the present invention is an organism having a diminished or inactivated Phosphotransferase Transport System (PTS). Such an organism, preferably a microorganism, may preferably be genetically modified by deletion or inactivation (a) gene(s) of said Phosphotransferase Transport System (PTS). Corresponding genetic modifications are known in the art.

In another preferred embodiment, the organism, preferably the microorganism, of the present invention is an organism having an enhanced non-Phosphotransferase Transport System (non-PTS) for sugar uptake. Such an organism, preferably a microorganism, may preferably be genetically modified by overexpression (a) gene(s) of said non-Phosphotransferase Transport System (non-PTS) for sugar uptake. Corresponding genetic modifications are known in the art.

In another preferred embodiment, the organism, preferably the microorganism, of the present invention is an organism having a diminished or inactivated Phosphotransferase Transport System (PTS) and an enhanced non-Phosphotransferase Transport System (non-PTS) for sugar uptake.

In another preferred embodiment, the organism, preferably the microorganism, of the present invention is an organism which is capable of consuming sucrose through a non-Phosphotransferase Transport System (non-PTS).

In another preferred embodiment, the organism, preferably the microorganism, of the present invention is an organism consuming sucrose, wherein said organism, preferably said microorganism, has genetically been modified by the introduction of at least one gene of a non-Phosphotransferase Transport System (non-PTS). Without being bound to theory, such an organism and/or microorganism has genetically been modified by introducing a gene selected from the group consisting of *cscA, cscB,* and *cscK* from Escherichia coli W (M. Bruschi et al., Biotechnology Advances 30 (2012) 1001-1010).

In another preferred embodiment, the organism, preferably the microorganism, of the present invention is an organism which has genetically been modified to have a diminished or inactivated Phosphotransferase Transport System (PTS) and an overexpression of at least one gene selected from the group consisting of *galP, glk and glf.*

In a preferred embodiment, the organism, preferably the microorganism, of the present invention is an organism which is genetically modified in order to avoid the leakage of acetyl-CoA, thereby increasing the intracellular concentration of acetyl-CoA. Genetic modifications leading to an increase in the intracellular concentration of acetyl-CoA are known in the art. Such an organism, preferably a microorganism, may preferably be genetically modified by deleting or inactivating the following genes: Δ*ackA* (acetate kinase), Δ*ldh* (lactate dehydrogenase), Δ*adhE* (alcohol dehydrogenase), Δ*frdB* and/or Δ*frdC* (fumarate reductase and fumarate dehydrogenase).

In another embodiment, the method of the invention comprises the step of providing the organism, preferably the microorganism, of the present invention in the form of a (cell) culture, preferably in the form of a liquid cell culture, a subsequent step of cultivating the organism, preferably the microorganism, in a fermenter (often also referred to a bioreactor) under suitable conditions and further comprising the step of effecting an enzymatic conversion of a method of the invention as described herein. Suitable fermenter or bioreactor devices and fermentation conditions are known to the person skilled in the art. A bioreactor or a fermenter refers to any manufactured or engineered device or system known in the art that supports a biologically active environment. Thus, a bioreactor or a fermenter may be a vessel in which a chemical/biochemical reaction like the method of the present invention is carried out which involves organisms, preferably microorganisms and/or biochemically active substances. In a bioreactor or a fermenter, this process can either be aerobic or anaerobic. These bioreactors are commonly cylindrical, and may range in size from litres to cubic metres, and are often made of stainless steel. In this respect, without being bound by theory, the fermenter or bioreactor may be designed in a way that it is suitable to cultivate the organisms, preferably microorganisms, in, e.g., a batch-culture, fed-batch-culture, perfusion culture or chemostate-culture, all of which are generally known in the art.

The culture medium can be any culture medium suitable for cultivating the respective organism or microorganism. When carried out by making use of an organism or microorganism, the method according to the present invention may, e.g. be designed as a continuous fermentation culturing method or as a batch culture or any suitable culture method known to the person skilled in the art.

### The production of 3-methylcrotonic acid and/or isobutene

In a particular embodiment, the invention relates to a method for the production of 3-methylcrotonic acid and/or isobutene, the method comprising a step of culturing a recombinant organism or microorganism according to the invention in a suitable culture medium under suitable conditions.

That is, the organism or microorganism according to the invention may be used in an industrial process for the production of isobutene. In certain embodiments, all enzymatic steps for the synthesis of isobutene will be carried out in the organism or microorganism of the invention. In such embodiments, the organism or microorganism preferably comprises the enzyme ferulic acid decarboxylase, which catalyzes the conversion of 3-methylcrotonic acid into isobutene.

Alternatively, isobutene may be produced in a two-step process. That is, the organism or microorganism according to the invention may be used for the production of the precursor molecule 3-methylcrotonic acid. The produced 3-methylcrotonic acid may then be converted into isobutene in vivo or *in vitro* in a biotransformation reaction.

That is, in a particular embodiment, the invention relates to a method for the production of isobutene, the method comprising the steps of: a) producing 3-methylcrotonic acid by culturing a recombinant organism or microorganism of the invention in a suitable culture medium under suitable conditions; and b) enzymatically converting said produced 3-methylcrotonic acid into isobutene in vivo or *in vitro.*

In a preferred embodiment the method according to the present invention also comprises the step of recovering the isobutene produced by the method. For example, when the method according to the present invention is carried out *in vivo* by fermenting a corresponding organism or microorganism expressing the necessary enzymes/transporters, the isobutene can be recovered from the fermentation off-gas by methods known to the person skilled in the art.

As mentioned above, the recombinant organism and microorganism as well as the method according to the present invention is in particular useful for large scale production of isobutene *in vivo,* in particular for a commercial production. The present invention describes novel means and ways to commercially and cost-effectively produce large quantities of isobutene which has not been obtainable to date. The generated large quantities of isobutene can then be further converted, in a commercial setting, to produce large quantities of, e.g., drop-in gasoline (e.g. isooctane, ETBE, MTBE), jet-fuel, cosmetics, chemicals, such as methacrylic acid, polyisobutene, or butyl rubber. As used herein, "large scale production", "commercial production" and "bioprocessing" of isobutene in a fermentation reactor or *in vitro* is carried out at a capacity greater than at least 100 liters, and preferably greater than at least 400 liters, or more preferably production of 1,000 liters of scale or more, even more preferably production of 5,000 liters of scale or more. As used herein, "large quantities" specifically excludes trace amounts that may be produced inherently in an organism or microorganism.

For example, in preferred embodiments, the yields for continuous cultures according to methods described herein are at least about 0.2 grams of isobutene per liter per day, at least about 0.3 grams of isobutene per liter per day, at least about 0.4 grams of isobutene per liter per day, at least about 0.5 grams of isobutene per liter per day, at least about 0.6 grams of isobutene per liter per day, at least about 0.7 grams of isobutene per liter per day, at least about 0.8 grams of isobutene per liter per day, or at least about 1.0 grams of isobutene per liter per day. In further embodiments, the yields for continuous cultures according to methods described herein are between about 0.3 grams and about 1.0 grams of isobutene per liter per day, between about 0.4 grams to about 1.0 grams of isobutene per liter per day, and between about 0.5 grams and about 1.0 grams of isobutene per liter per day. In other specific embodiments, the yields for continuous cultures according to methods described herein are between about 0.5 grams to about 0.75 grams of isobutene per liter per day. In other specific embodiments, the yields for continuous cultures according to methods described herein are between about 0.5 grams and about 1.5 grams of isobutene per liter per day.

In further embodiments, the yields for batch cultures according to methods described herein are at least about 2 grams per liter in batch culture, at least about 5 grams per liter in batch culture, at least about 10 grams per liter in batch culture, and at least about 15 grams per liter in batch culture. In some embodiments, the yields for batch cultures according to methods described herein are between about 2 grams and about 5 grams per liter in batch culture, between about 5 grams and about 10 grams per liter in batch culture, and still more preferably between about 10 grams and about 20 grams per liter in batch culture. In other specific embodiments, the yields for batch cultures according to methods described herein are between about 2.4 grams and about 4.8 grams per liter, and preferably between about 4.8 grams and about 9.4 grams per liter in batch culture, and still more preferably between about 9.4 grams and about 18.6 grams per liter in batch culture.

### The production of other alkenes

Within the present invention, the recombinant organism or microorganism is preferably used for the production of isobutene, wherein 3-methylcrotonic acid is converted to isobutene in a final reaction step by the enzyme ferulic acid decarboxylase (FDC). However, it is known in the art that FDCs can be used for the production of other alkenes.

For example, Mori et al. (Direct 1,3-butadiene biosynthesis in Escherichia coli via a tailored ferulic acid decarboxylase mutant, Nature Communications volume 12, Article number: 2195 (2021)) disclosed the production of 1,3-butadiene biosynthesis from *cis*,*cis*-muconic acid and pentadienoic acid using FDC. As it is shown herein that crotonic acid can inhibit both wild type and highly engineered FDC variants, it is likely that also other biotechnological processes that involve activity of an FDC variant, such as the production of 1,3-butadiene, will be significantly inhibited in the presence of even trace amounts of crotonic acid. Thus, the recombinant organism or microorganism according to this invention may be used as a universal platform for FDC-based alkene production.

Accordingly, in a particular embodiment, the invention relates to a recombinant organism or microorganism having a decreased pool of crotonic acid compared to the organism or microorganism from which it is derived due to at least one of:
(i) a decreased conversion of acetaldehyde into crotonaldehyde; and/or
(ii) a decreased conversion of crotonyl-CoA into crotonaldehyde; and/or
(iii) a decreased conversion of crotonaldehyde into crotonic acid;
wherein the recombinant organism of microorganism encodes a ferulic acid decarboxylase.

The FDC may be any one of the FDC variants that have been disclosed herein for the production of isobutene. Further, the FDC may be an FDC from Aspergillus niger (*An*FDC) or Saccharomyces cerevisiae (scFDC) or any mutant variant thereof. In particular, the FDC may be any variant of *An*FDC or *Sc*FDC that has been disclosed by Mori et al. (see citation above) for the production of 1-3-butadiene.

In a particular embodiment, the invention relates to a recombinant organism or microorganism having a decreased pool of crotonic acid compared to the organism or microorganism from which it is derived due to at least one of:
(i) a decreased conversion of acetaldehyde into crotonaldehyde; and/or
(ii) a decreased conversion of crotonyl-CoA into crotonaldehyde; and/or
(iii) a decreased conversion of crotonaldehyde into crotonic acid;
wherein the recombinant organism or microorganism is used in the production of an alkene.

The alkene may be any alkene that can be produced with a wild type or engineered FDC variant. In a particular embodiment, the alkene is isobutene. In another embodiment, the alkene is 1,3-butadiene. It is to be noted that the recombinant organism or microorganism does not necessarily need to comprise a gene encoding an FDC. That is, the recombinant organism or microorganism may also be used for the production of a precursor of an alkene, which is then converted into the alkene by an FDC in a subsequent step. Thus, the recombinant organism or microorganism may be used only for a certain part of an alkene production process.

For example, a recombinant organism or microorganism according to the invention may be used in the production of the precursor molecules 3-methylcrotonic acid or *cis*,*cis*-muconic acid or pentadienoic acid. Producing these precursors in the recombinant organism or microorganism according to the invention will minimize the formation of crotonic acid as a side product. As a result, the fermentation broth can be used directly for the production of the corresponding alkenes by an FDC.

In certain embodiments, 1,3-butadiene is obtained by:
(i) enzymatically converting phosphoenolpyruvate and erythrose-4-phosphate into 3-deoxy-D-heptulosonate-7-phosphate into,
(ii) enzymatically converting 3-deoxy-D-heptulosonate-7-phosphate into 3-dehydroshikimate,
(iii) enzymatically converting 3-dehydroshikimate into protocatechuic acid,
(iv) enzymatically converting protocatechuic acid into catechol,
(v) enzymatically converting catechol into *cis*,*cis*-muconate;
(vi) enzymatically converting *cis*,*cis*-muconate into (Z)-pentadienoate; and
(vii) enzymatically converting (Z)-pentadienoate into 1,3-butadiene.

Preferably, the conversion of *cis*,*cis*-muconate into (Z)-pentadienoate and (Z)-pentadienoate into 1,3-butadiene are catalyzed by a ferulic acid decarboxylase. In certain embodiments, the ferulic acid decarboxylase is an engineered *An*FDC or ScFDC as disclosed by Mori *et al.*

In a particular embodiment, the invention relates to a recombinant organism or microorganism having a decreased pool of crotonic acid compared to the organism or microorganism from which it is derived due to at least:
(i) a decreased conversion of acetaldehyde into crotonaldehyde; and/or
(ii) a decreased conversion of crotonyl-CoA into crotonaldehyde; and/or
(iii) a decreased conversion of crotonaldehyde into crotonic acid;
wherein said recombinant organism or microorganism is capable of converting phosphoenolpyruvate and erythrose-4-phosphate into 1,3-butadiene, wherein in said microorganism:
(i) phosphoenolpyruvate and erythrose-4-phosphate are enzymatically converted into 3-deoxy-D-heptulosonate-7-phosphate,
(ii) 3-deoxy-D-heptulosonate-7-phosphate is enzymatically converted into 3-dehydroshikimate,
(iii) 3-dehydroshikimate is enzymatically converted into protocatechuic acid,
(iv) protocatechuic acid is enzymatically converted into catechol,
(v) catechol is enzymatically converted into *cis*,*cis*-muconate;
(vi) *cis*,*cis*-muconate is enzymatically converted into (Z)-pentadienoate; and
(vii) (Z)-pentadienoate is enzymatically converted into 1,3-butadiene.

In a particular embodiment, the invention relates to a recombinant organism or microorganism which is capable of converting phosphoenolpyruvate and erythrose-4-phosphate into 1,3-butadiene, wherein in said microorganism:
(i) phosphoenolpyruvate and erythrose-4-phosphate are enzymatically converted into 3-deoxy-D-heptulosonate-7-phosphate,
(ii) 3-deoxy-D-heptulosonate-7-phosphate is enzymatically converted into 3-dehydroshikimate,
(iii) 3-dehydroshikimate is enzymatically converted into protocatechuic acid,
(iv) protocatechuic acid is enzymatically converted into catechol,
(v) catechol is enzymatically converted into *cis*,*cis*-muconate;
(vi) *cis*,*cis*-muconate is enzymatically converted into (Z)-pentadienoate; and
(vii) (Z)-pentadienoate is enzymatically converted into 1,3-butadiene.
wherein in said organism the expression level and/or the activity of
(i) an aldehyde lyase capable of condensing two molecule of acetaldehyde to form crotonaldehyde; and/or
(ii) an aldehyde dehydrogenase capable of reducing crotonyl-CoA to crotonaldehyde; and/or
(iii) an aldehyde dehydrogenase capable of oxidizing crotonaldehyde to crotonic acid;
is decreased.

Preferably, phosphoenolpyruvate is obtained from glucose-6-phosphate during glycolysis and erythrose-4-phosphate is obtained from glucose-6-phosphate during the pentose-6-phosphate pathway.

In a particular embodiment, the invention relates to a recombinant organism or microorganism which is capable of converting acetyl-CoA into 3-methylcrotonic acid, wherein in said microorganism:
(i) acetyl-CoA is enzymatically converted into acetoacetyl-CoA,
(ii) acetoacetyl-CoA is enzymatically converted into 3-hydroxy-3-methylglutaryl-CoA,
(iii) 3-hydroxy-3-methylglutaryl-CoA is enzymatically converted into 3-methylglutaconyl-CoA,
(iv) 3-methylglutaconyl-CoA is enzymatically converted into 3-methylcrotonyl-CoA, and
(v) 3-methylcrotonyl-CoA is enzymatically converted into 3-methylcrotonic acid;
wherein in said organism the expression level and/or the activity of
(i) an aldehyde lyase capable of condensing two molecule of acetaldehyde to form crotonaldehyde; and/or
(ii) an aldehyde dehydrogenase capable of reducing crotonyl-CoA to crotonaldehyde; and/or
(iii) an aldehyde dehydrogenase capable of oxidizing crotonaldehyde to crotonic acid;
is decreased.

In a particular embodiment, the invention relates to a recombinant organism or microorganism which is capable of converting acetyl-CoA into isobutene, wherein in said microorganism:
(i) acetyl-CoA is enzymatically converted into acetoacetyl-CoA,
(ii) acetoacetyl-CoA is enzymatically converted into 3-hydroxy-3-methylglutaryl-CoA,
(iii) 3-hydroxy-3-methylglutaryl-CoA is enzymatically converted into 3-methylglutaconyl-CoA,
(iv) 3-methylglutaconyl-CoA is enzymatically converted into 3-methylcrotonyl-CoA,
(v) 3-methylcrotonyl-CoA is enzymatically converted into 3-methylcrotonic acid; and
(vi) 3-methylcrotonic acid is enzymatically converted into isobutene;
wherein in said organism the expression level and/or the activity of
(i) an aldehyde lyase capable of condensing two molecule of acetaldehyde to form crotonaldehyde; and/or
(ii) an aldehyde dehydrogenase capable of reducing crotonyl-CoA to crotonaldehyde; and/or
(iii) an aldehyde dehydrogenase capable of oxidizing crotonaldehyde to crotonic acid;
is decreased.

The aldehyde lyase and the aldehyde dehydrogenase may be, without limitation any one of the aldehyde lyases or aldehyde dehydrogenases disclosed herein.

### List of Embodiments

1. A recombinant organism or microorganism having a decreased pool of crotonic acid compared to the organism or microorganism from which it is derived due to at least:
   (i) a decreased conversion of acetaldehyde into crotonaldehyde; and/or
   (ii) a decreased conversion of crotonyl-CoA into crotonaldehyde; and/or
   (iii) a decreased conversion of crotonaldehyde into crotonic acid.
2. The recombinant organism or microorganism according to embodiment 1, wherein said recombinant organism or microorganism is a recombinant microorganism.
3. The recombinant organism or microorganism according to embodiment 1 or 2, wherein said recombinant microorganism is a fungus or a bacterium.
4. The recombinant organism or microorganism according to embodiment 3, wherein said bacterium is *Escherichia coli.*
5. The recombinant organism or microorganism according to any one of embodiments 1 to 4, wherein the decreased conversion of acetaldehyde into crotonic acid is due to a reduced level and/or activity of an aldehyde lyase (EC 4.1.2) in said organism or microorganism.
6. The recombinant organism or microorganism according to embodiment 5, wherein the aldehyde lyase (EC 4.1.2) is a deoxyribose-phosphate aldolase (EC 4.1.2.4).
7. The recombinant organism or microorganism according to embodiment 6, wherein the deoxyribose-phosphate aldolase (EC 4.1.2.4) is DeoC from *Escherichia coli.*
8. The recombinant organism or microorganism according to any one of embodiments 1 to 4, wherein the decreased conversion of crotonyl-CoA into crotonaldehyde is due to a reduced level and/or activity of an aldehyde dehydrogenase (EC 1.2) is said organism or microorganism.
9. The recombinant organism or microorganism according to embodiment 8, wherein the aldehyde dehydrogenase (EC 1.2) is an acetaldehyde dehydrogenase (acetylating) (EC 1.2.1.10).
10. The recombinant organism or microorganism according to embodiment 8 or 9, wherein the acetaldehyde dehydrogenase (acetylating) (EC 1.2.1.10) is MhpF from *Escherichia coli,* and/or AdhE from *Escherichia coli* and/or EutE from *Escherichia coli.*
11. The recombinant organism or microorganism according any one of embodiments 1 to 4, wherein the decreased conversion of crotonaldehyde into crotonic acid is due to a reduced level and/or activity of an aldehyde dehydrogenase (EC 1.2) in said organism or microorganism.
12. The recombinant organism or microorganism according to embodiment 11, wherein the aldehyde dehydrogenase (EC 1.2) is an NAD+ or NADP+-dependent aldehyde dehydrogenase (EC 1.2.1), or an iron-sulfur protein-dependent aldehyde dehydrogenase (EC 1.2.7).
13. The recombinant organism or microorganism according to embodiment 11 or 12, wherein the aldehyde dehydrogenase (EC 1.2) is PuuC from *Escherichia coli* (EC 1.2.1.19 and 1.2.1.24 and 1.2.1.99), FeaB from *Escherichia coli* (EC 1.2.1.39), PatD from *Escherichia coli* (EC 1.2.19), AldA from *Escherichia coli* (EC 1.2.1.21 and 1.2.1.22), Sad from *Escherichia coli* (EC 1.2.1.24), AstD from *Escherichia coli* (EC 1.2.1.71), BetB from *Escherichia coli* (EC 1.2.1.8), AldB from *Escherichia coli* (EC 1.2.1.4), and/or GabD from *Escherichia coli* (EC 1.2.1.79).
14. The recombinant organism or microorganism according to any one of embodiments 5 to 13, wherein the reduced level of an enzyme is due to
   (i) a complete or partial deletion of a gene encoding the respective enzyme in said organism or microorganism; and/or
   (ii) a deletion or an inactivating mutation in a regulatory element of a gene encoding the respective enzyme in said organism or microorganism.
15. The recombinant organism or microorganism according to any one of embodiments 5 to 13, wherein the reduced activity of an enzyme is due to
   (i) an inactivating mutation in a gene encoding the respective enzyme in said organism or microorganism; and/or
   (ii) the addition of an inhibitor of the respective enzyme.
16. The recombinant organism or microorganism according to any one of embodiments 1 to 15 further encoding a ferulic acid decarboxylase.
17. The recombinant organism or microorganism according to embodiment 16, wherein the ferulic acid decarboxylase catalyzes the formation of an alkene from a corresponding carboxylic acid, in particular wherein the formation of the alkene is catalyzed by a ferulic acid decarboxylase.
18. The recombinant organism or microorganism according to any one of embodiments 1 to 17, wherein the recombinant organism or microorganism is capable of:
   (i) enzymatically converting acetyl-CoA into 3-methylcrotonic acid and/or isobutene; and/or
   (ii) enzymatically converting 3-methylcrotonic acid into isobutene; and/or
   (iii) enzymatically converting *cis*,*cis*-muconic acid or pentadienoic acid into 1-3- butadiene.
19. The recombinant organism or microorganism according to embodiment 18, wherein the conversion of acetyl-CoA into 3-methylcrotonic acid comprises the steps of:
   (i) enzymatically converting acetyl-CoA into acetoacetyl-CoA,
   (ii) enzymatically converting said produced acetoacetyl-CoA into 3-hydroxy-3-methylglutaryl-CoA,
   (iii) enzymatically converting said produced 3-hydroxy-3-methylglutaryl-CoA into 3-methylglutaconyl-CoA,
   (iv) enzymatically converting said produced 3-methylglutaconyl-CoA into 3-methylcrotonyl-CoA, and
   (v) enzymatically converting said produced 3-methylcrotonyl-CoA into 3-methylcrotonic acid.
20. The recombinant organism or microorganism according to embodiment 19, wherein the recombinant organism or microorganism is further capable of enzymatically converting the produced 3-methylcrotonic acid into isobutene.
21. The recombinant organism or microorganism according to embodiment 20, wherein the conversion of 3-methylcrotonic acid into isobutene is catalyzed by a ferulic acid decarboxylase.
22. Use of the recombinant organism or microorganism according to any one of embodiments 1 to 21 for the production of an alkene, in particular wherein the alkene is produced by a ferulic acid decarboxylase.
23. The use according to embodiment 22, wherein the alkene is isobutene or 1,3-butadiene.
24. A method for the production of 3-methylcrotonic acid and/or isobutene, the method comprising a step of culturing a recombinant organism or microorganism as defined in any one of embodiments 19 to 21 in a suitable culture medium under suitable conditions.
25. A method for the production of isobutene, the method comprising the steps of:
   a) producing 3-methylcrotonic acid by culturing a recombinant organism or microorganism as defined in any one of embodiments 19 to 21 in a suitable culture medium under suitable conditions; and
   b) enzymatically converting said produced 3-methylcrotonic acid into isobutene.
26. The method according to embodiment 25, wherein the conversion of 3-methylcrotonic acid into isobutene is catalyzed by a ferulic acid decarboxylase.

### BRIEF DESCRIPTION OF THE DRAWINGS

**FIG.1** shows artificial pathways for isobutene production from acetyl-CoA via 3-methylcrotonic acid. Moreover, enzymatic recycling of metabolites which may occur during the pathway are shown in steps Xa, Xb, XI and XII.
**FIG.2** shows that an FDC variant that has been evolved to specifically decarboxylate 3-methylcrotonic acid cannot undergo the cycloelimination step when bound to crotonic acid, resulting in an irreversible inhibition of the enzyme.

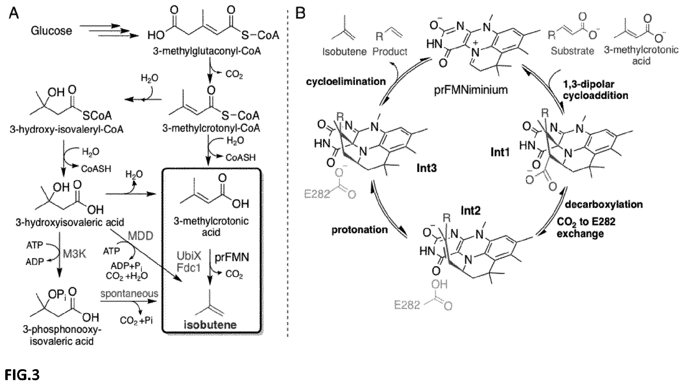

**FIG.3** (A) Routes to isobutene via modified mevalonate pathways. Previously published mevalonate diphosphate decarboxylase (MVD) and mevalonate-3-kinase (M3K) produce isobutene via 3-hydroxyisovaleric acid. Fdc1, co-expressed with UbiX, catalyses the decarboxylation of 3-methylcrotonic acid to give isobutene. (B) Fdc decarboxylation reaction mechanism with 3-methylcrotonic acid and common Fdc substrates with a conjugated R-group. First, the 1,3-dipolar cycloaddition of the substrate to prFMNiminium leads to the first pyrrolidine cycloadduct, Int1. Decarboxylation and ring-opening forms the noncyclic alkene adduct Int2. Protonation by a conserved glutamic acid residue yields the second pyrrolidine cycloadduct Int3 followed by cycloelimination to give the product.

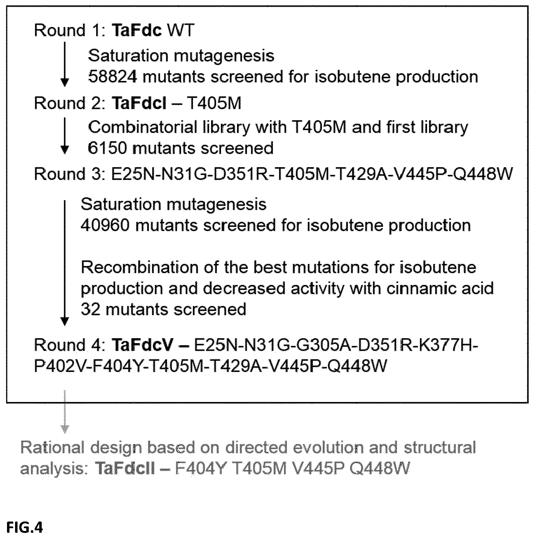

**FIG.4****:** The directed evolution of TaFdc wild-type to TaFdcV with superior isobutene production.

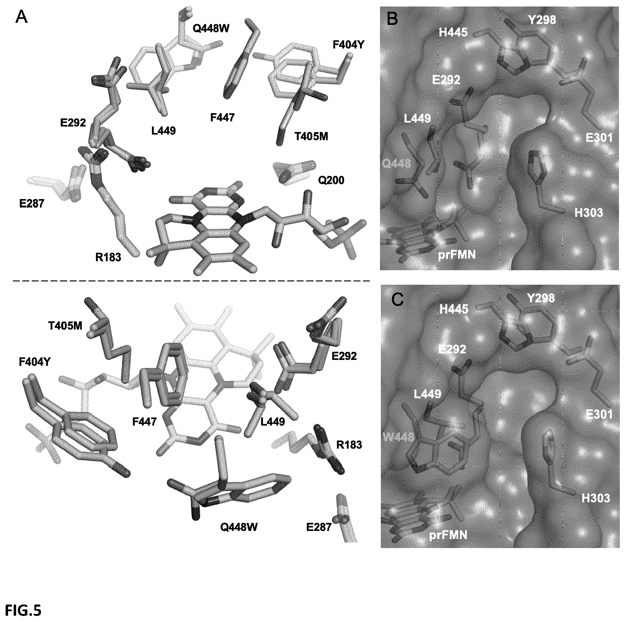

**FIG.5****:** (A) Overlay of *Ta*Fdc wild-type and *Ta*FdcV active sites in two orientations (separated by dotted line). Comparison of *Ta*Fdc (B) and *Ta*FdcV (C) binding pockets. The mobile L449 and E292 gate the entrance to the active site and the Q448W mutation narrows the entrance to the active site.

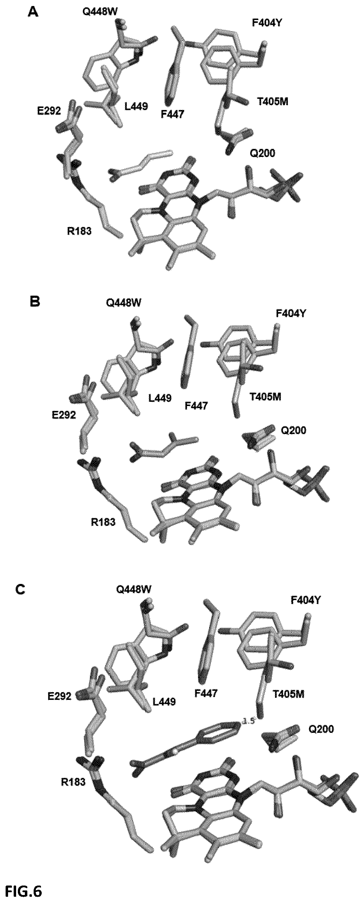

**FIG.6****:** Crotonic (A) and 3-methylcrotonic (B) acid binding at the active site of TaFdcV (blue), overlayed with TaFdc wild-type (green) modelled with Vina docking. (C) TaFdcV (blue) and TaFdc wild-type (green) overlayed with AnFdc wild-type in complex with alpha-fluorocinnamic acid (PDB: 4ZAB) demonstrating the clash between the M405 residue and the phenyl ring.

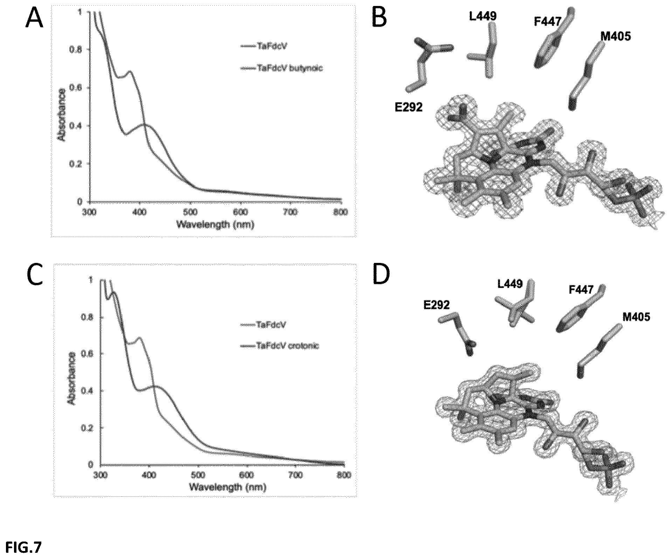

**FIG.7****:** (A) UV-Vis spectra of TaFdcV before and after incubating with 2-butynoic acid. (B) ESI― MS spectra of TaFdcV incubated with 2-butynoic acid and desalted, showing the formation of prFMN-butynoic adduct [M + H] = 607.18. (C) Crystal structure of TaFdcV with prFMN-butynoic adduct (7NF1 [10.2210/pdb7NF1/pdb]). (D) UV-Vis spectra of TaFdcV as is and incubated with crotonic acid (E) ESI-MS spectra of TaFdcV incubated with crotonic acid (and desalted) showing the formation of decarboxylated prFMN-crotonic adduct [M-H]⁻ = 565.21 and traces of prFMN-crotonic with the carboxylate group [M-H]⁻ = 609.20. F Crystal structure of TaFdcV prFMN-crotonic adduct (7NF2 [10.2210/pdb7NF2/pdb]).

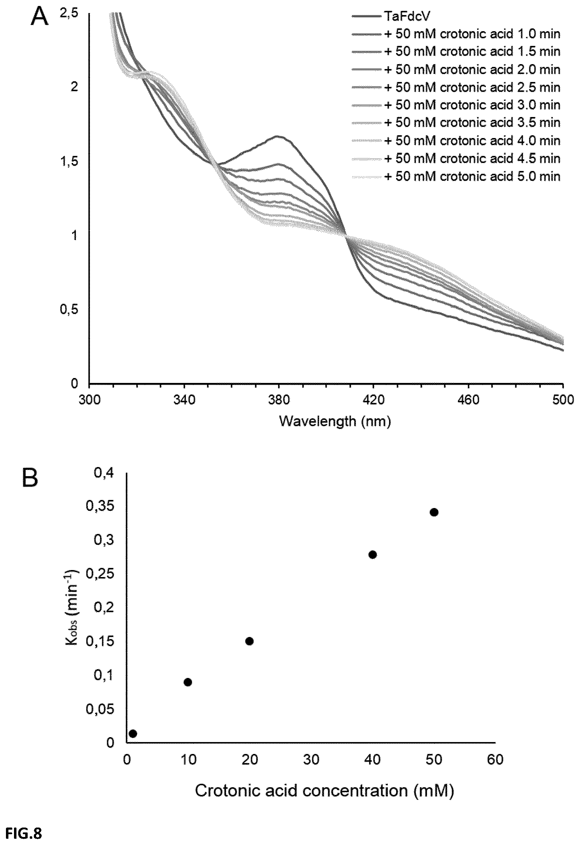

**FIG.8****:** TaFdcV kinetics with crotonic acid A - gradual split of the 380 nm prFMN peak upon addition of crotonic acid. B - the observed rate of cycloadduct formation based on decrease in the 380 nm peak measured with 1 mM, 10 mM, 20 mM, 40 mM and 50 mM crotonic acid showing a linear relationship.

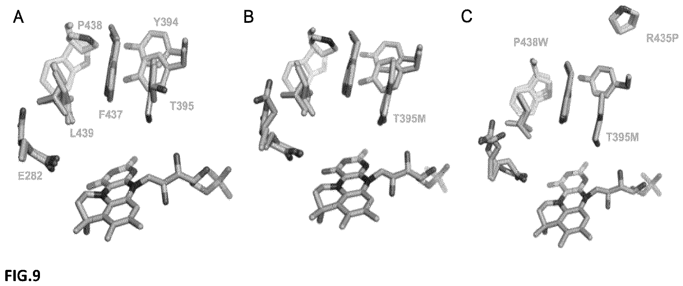

**FIG.9****:** Overlay of *Ta*FdcV with (A) *An*Fdc wild-type; **(B)** *An*Fdcl and (**C)** *An*FdcII crystal structures (AnFdc variant residue numbering according to AnFdc).

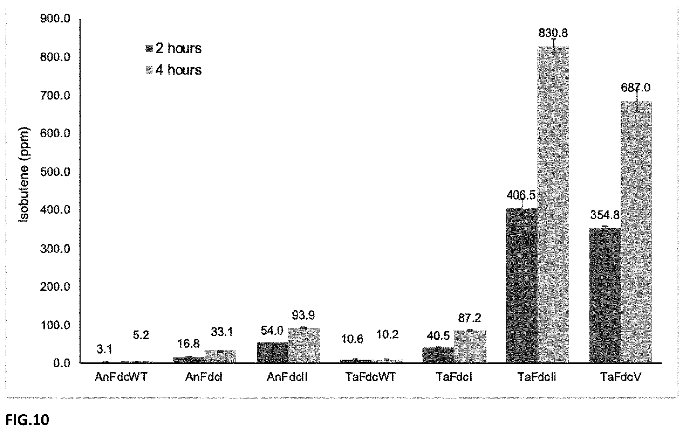

**FIG.10****:** 3-methylcrotonate decarboxylation assay with purified enzyme comparing isobutene production as detected by GC by TaFdc variants (wild-type, I, II, V) and AnFdc variants (wild-type, I, II), both with N-terminal His-tags, over 2 and 4 hours. 10 mM 3-methylcrotonate with 0.3 mg/mL enzyme.

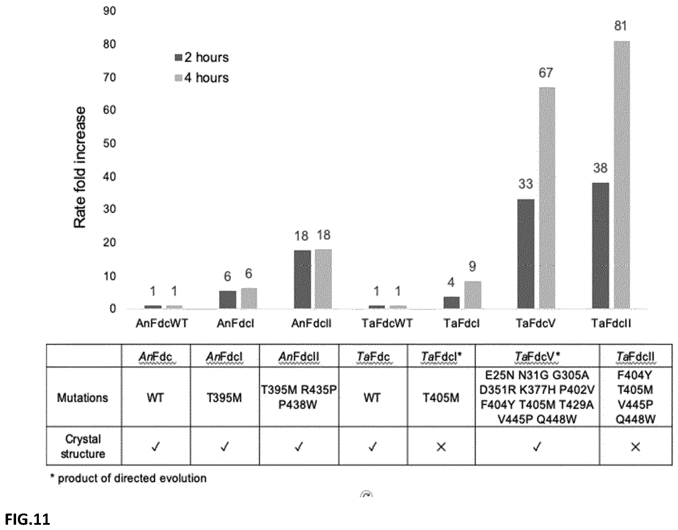

**FIG.11****:** 3-methylcrotonate decarboxylation assay with purified enzyme comparing isobutene production as detected by GC by TaFdc and AnFdc variants, both with N-terminal His-tags, over 2 and 4 h with 10 mM 3-methylcrotonate and 0.3 mg/mL enzyme. Fold increase comparison is shown in Fig.10.

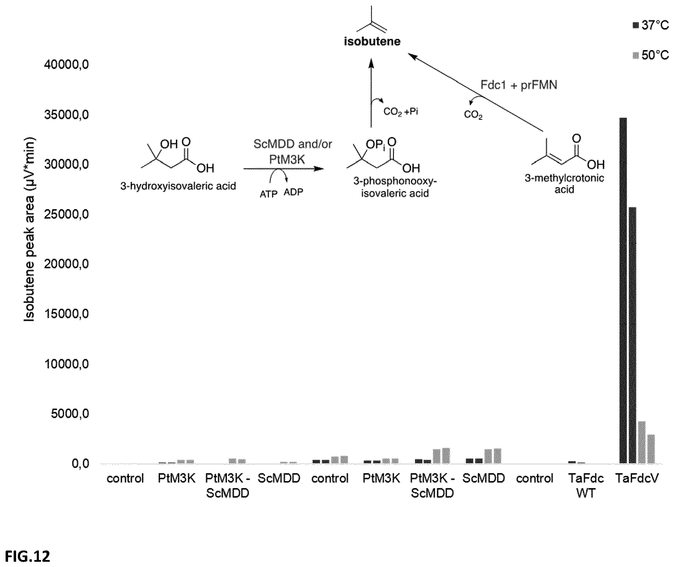

**FIG.12****:** A comparison of isobutene *in vitro* production levels, using *E. coli* cell lysate. An equal amount of lysate obtained from cells expressing respectively *Picrophilus torridus* mevalonate 3-kinase (PtM3K), *Saccharomyces cerevisiae* mevalonate diphosphate decarboxylase (ScMDD) or *T. atroviride* Fdcl/V (in combination with UbiX) was incubated with 50mM of respectively HIV (3-hydroxyisovalerate)/ATP, PIV (3-phosphonooxy-isovalerate)/ADP or 3-methylcrotonate. Following 4 h incubation at 37 (dark grey) and 50 °C (light grey), the isobutene content of the gas phase was analysed using GC. Under these conditions, *Ta*FdcV mediated isobutene levels exceeded the highest PtM3K/ScMDD levels by ^{~}50-fold at 37 °C. The highest TaFdcV isobutene conversion was approximately 11.5% of the substrate provided.

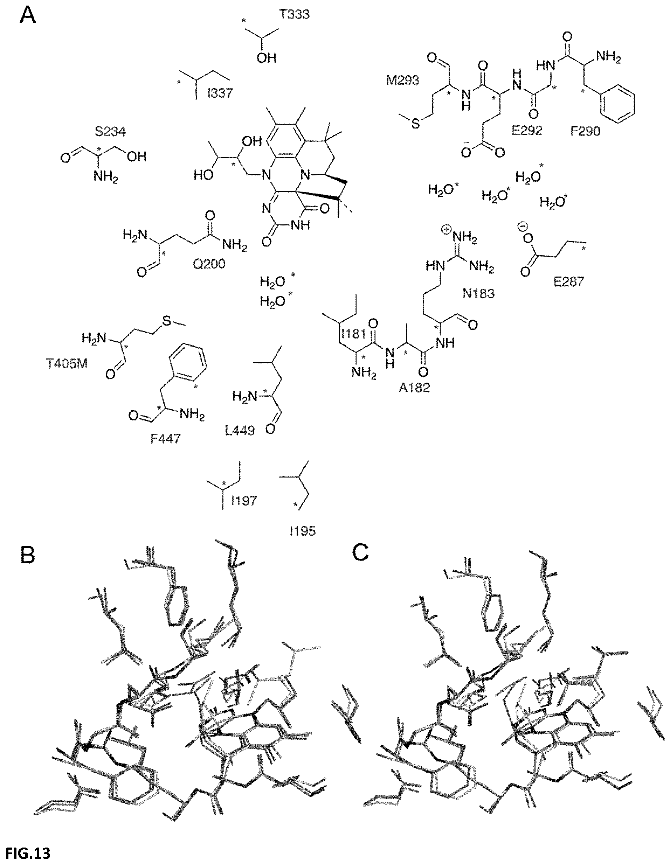

**FIG.13****:** (A) Model of the active site of TaFdcV used for DFT calculations based on the crystal structure of TaFdcV with crotonic acid in **Int3.** Asterisks denote fixed atoms. (B) Overlay of TaFdcV crystal structure with crotonic acid adduct and DFT optimized models of 3-methylcrotonic acid transition state and cycloelimination product isobutene. (C) Overlay of TaFdcV crystal structure with crotonic acid adduct and DFT optimized models of crotonic acid transition state and cycloelimination product propene.

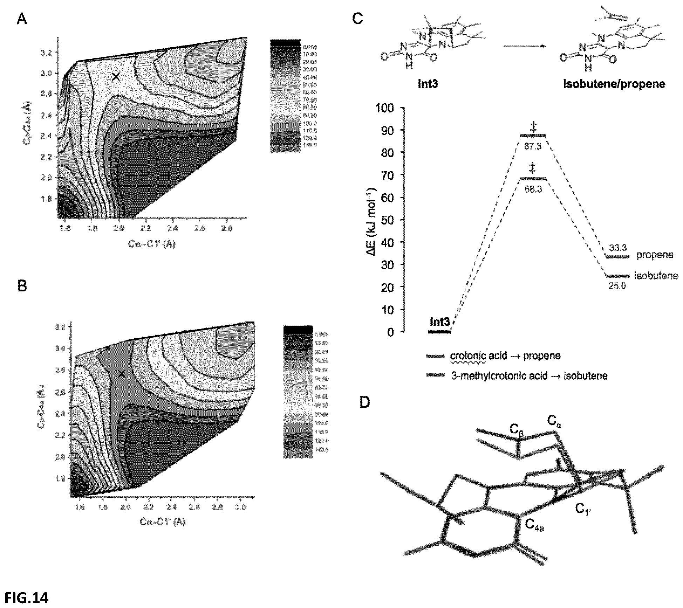

**FIG.14****:** Contour map of the potential energy (kJ mol⁻¹) landscape for 3-methylcrotonic acid (A) and crotonic acid (B) conversion to isobutene and propene, respectively, from Int3 by *Ta*FdcV, projected transition state is marked by X (C) Zero-point energy corrected potential energy (kJ mol⁻¹) scheme for 3-methylcrotonic and crotonic acid with the Int3 set as 0 and the projected approximate transition state denoted with double daggers (D) overlay of the DFT optimized transition states between Int3 and product for 3-methylcrotonic (C_{α}-C_{1'} and C_{β}-C₄ₐ bond lengths of 1.96 and 2.97 Å, respectively) and crotonic acid (C_{α}-C_{1'} and C_{β}-C₄ₐ bond lengths of 1.95 and 2.77 Å, respectively).

### EXPERIMENTAL EXAMPLES

The irrefutable harmful environmental effects and depleting reserves of fossil fuels have powered an extensive amount of research to seek sustainable alternatives for the production of petrochemicals, including the gaseous alkene isobutene. Due to the favourable reactivity, isobutene is widely used as a building block for fuel additives, rubber, plastic and a broad range of fine chemicals. Over 10 million tons of isobutene are produced every year, primarily by steam cracking crude oil. Low levels of microbial production of isobutene were first detected in the 1980s. More recently, isobutene production via a modified mevalonate (MVA) pathway using mevalonate diphosphate decarboxylase (MVD) to decarboxylate 3-hydroxyisovaleric acid was reported (Fig. 3A). Further studies highlighted a more efficient route using mevalonate-3-kinase (M3K, Picrophilus torridus) that catalyses isobutene formation through an unstable phosphorylated intermediate. The highest reported whole-cell isobutene production rate of 507 pmol min⁻¹ g cells⁻¹ was reached using E. coli engineered with M3K, however, this remains about 10-fold lower than is economically viable. The slow conversion could be surpassed by an alternative route, such as the more direct conversion of methylcrotonyl-CoA to isobutene through a combination of a thioesterase with a non-oxidative decarboxylase. The prenylated flavin (prFMN)-dependent ferulic acid decarboxylases (Fdc) catalyse reversible non-oxidative (de)carboxylation of a range of acrylic acids with extended conjugation. Recently, a reversible 1,3-dipolar cycloaddition mechanism was conclusively shown to underpin catalysis in Aspergillus niger Fdc (AnFdc). First, the cycloaddition of the substrate results in cycloadduct Int1 (Fig. 3B). Decarboxylation occurs concomitantly with ring opening to form Int2. Following the exchange of CO₂ with E282, protonation by E282 results in cycloadduct Int3 that releases the product through cycloelimination. Cycloadduct strain, mediated by a clash between the substrate R group and enzyme residues is key in ensuring reversible 1,3-dipolar cycloaddition]. Recent studies have shown rational engineering of AnFdc can expand substrate scope to include aromatic substrates such as naphthoic acid. However, acrylic acid substrates lacking extended conjugation have rarely been reported in the wider UbiD enzyme family. Arguably, the natural UbiD substrate closest to 3-methylcrotonic acid is trans-anhydromevalonate 5-phosphate (tAHMP), which is decarboxylated by a UbiD decarboxylase from a hyperthermophilic archaeon Aeropyrum pernix in an alternative mevalonate pathway. Both 3-methylcrotonic acid and tAHMP contain a secondary beta carbon and lack extended conjugation, however, the phosphate group in tAHMP may facilitate strain manipulation in cycloadduct intermediates.

Herein, the inventors report on discovery and optimization through directed evolution of Fdc decarboxylation activity with 3-methylcrotonic acid to produce isobutene. The inventors seek to understand how a substrate lacking extended conjugation and bulk can be decarboxylated by Fdc. The inventors discuss the structural basis for an increase in activity and selectivity in *Trichoderma atroviride* Fdc (TaFdc) evolved by directed evolution. Surprisingly, the optimized variants remain unable to decarboxylate crotonic acid, suggesting that in the case of the substrate 3-methylcrotonic acid the single additional methyl group plays a key role in the cycloelimination process. Computational studies were used to rationalize the effect of the 3-methyl substitution on product formation.

### Initial screening of Fdc homologues

Initial in vivo screening tested 15 UbiD homologues co-expressed with UbiX (E. coli K-12) in E. coli for conversion of 3-methylcrotonic acid into isobutene as detected by gas chromatography. TaFdc exhibited over twice the isobutene production compared to other homologues (Table 4) and a directed evolution approach was taken to generate a variant of TaFdc with superior isobutene production activity and selectivity for 3-methylcrotonic acid over cinnamic acid (Fig. 4). TaFdcl, with a T405M mutation, was the first variant with a considerable increase in isobutene. TaFdcV generated by 4 rounds of evolution has 11 mutations: E25N, N31G, G305A, D351R, K377H, AQ6 P402V, F404Y, T405M, T429A, V445P and Q448W.

**Table 4: Initial in vivo screening of Fdc variants for wild-type 3-methylcrotonic acid decarboxylation activity. The Fdc was co-expressed with UbiX in a pETDuet plasmid with Fdc in MCS1 (with N-terminal 6His-tag) and UbiX in MCS2.**

| | | ppm | |
|---|---|---|---|
| pETDuet | FdcUniprot code | Average | StdDev |
| CiFdc-UbiX | A0A0D2AQI6 | 16.4 | 0.8 |
| SmFdc-UbiX | M3DF95 | 5.9 | 0.7 |
| PrFdc-UbiX | W6QKP7 | 9.2 | 1.5 |
| ApFdc-UbiX | A0A0F0IHE5 | 108.9 | 8.4 |
| NfFdc-UbiX | A1DCG7 | 108.0 | 7.4 |
| CcFdc-UbiX | W9YNA8 | 125.2 | 9.2 |
| PcFdc-UbiX | A0A0G4P429 | 19.8 | 2.9 |
| AnFdc-UbiX | A2QHE5 | 13.2 | 3.1 |
| BfFdc-UbiX | M7THT1 | 7.8 | 1.1 |
| PpFdc-UbiX | A0A094IED9 | 8.8 | 2.4 |
| CsFdc-UbiX | A0A0D2DPQ1 | 13.4 | 1.1 |
| CpFdc-UbiX | W9WWR1 | 18.0 | 2.2 |
| CdFdc-UbiX | B9WJ66 | 4.8 | 1.3 |
| ScFdc-UbiX | Q03034 | 17.6 | 1.3 |
| TaFdc-UbiX | G9NLP8 | 330.0 | 29.1 |
| UbiX alone | NA | 3.3 | 1.4 |
| empty plasmid | NA | 3.2 | 1.2 |

### Characterization of TaFdc and TaFdcV

TaFdc wild-type and TaFdcV with an N-terminal hexa-histidine tag were co-expressed with E. coli K-12 UbiX in E. coli and purified with Ni-NTA resin. UV-Vis spectra of both purified proteins exhibit a distinct peak at 380 nm, thought to correspond to the cofactor active form prFMNiminium. ESI-MS confirmed the presence of prFMNiminium in both enzyme variants. The shape of the 380 nm peak and cofactor content (assessed by the ratio of absorbances at 280 and 380 nm) varied from batch to batch.

TaFdc showed decarboxylation activity with cinnamic and sorbic acid, with rates k_{obs} = 7.2 ± 0.3 and 3.2 ± 0.3 s, respectively (reported for a batch with a 380:280 nm ratio of 0.067). These values are comparable to those reported for AnFdc. In contrast, the TaFdcV variant showed compromised activity with sorbic acid (k_{obs} = 0.33 ± 0.03 s ) and no activity was detected with cinnamic acid. When exposed to light, TaFdc sorbic acid decarboxylation activity steadily deteriorates with a half-life of 1 h compared to enzyme stored in dark. This is consistent with Fdc light-sensitivity as described previously. Upon irradiation with a 405 nm LED lamp, the characteristic 380 nm peak in the UV-visible absorbance spectra of TaFdc and TaFdcV irreversibly splits to peaks at 365 and 425 nm.

Incubation of both TaFdc and TaFdcV with 3-methylcrotonic acid triggered a change in the protein UV-Vis spectrum to reveal peaks at 340 and 425 nm, suggestive of a covalent substrate:prFMN adduct accumulating under turnover conditions. Following a desalting step, the spectrum returns to the as-isolated 380 nm single feature, confirming that a long-lived, inhibitory covalent complex with 3-methylcrotonic acid is not formed. Incubation of 80 µmol TaFdcV with 10 mM 3-methylcrotonic acid led to a complete shift in the corresponding UV-Vis spectrum. In contrast, the wild-type TaFdc required prolonged incubation with 50 mM 3-methylcrotonic acid to achieve full spectral conversion, suggesting a substantially higher *K_{D}* and/or adduct formation rate for the wild-type enzyme. An ESI-MS spectrum of the desalted sample showed peaks corresponding to both prFMNiminium and a putative Int3 prFMN cycloadduct with 3-methylcrotonic acid. This may be due to a small proportion of 3-methylcrotonic acid remains bound to prFMN as Int3, suggesting Int3 elimination is the rate limiting step, or that a proportion of the Int3 species has irreversibly isomerized to a more stable conformation.

In order to assess the scope for activity with acrylic acids lacking extended conjugation, TaFdc and TaFdcV were incubated with trans-2-pentenoic and trans-2-hexenoic acid, compounds that have previously been reported to undergo some AnFdc-mediated decarboxylation. UV-Vis absorbance spectra indicated that TaFdc bound both acids, whereas the TaFdcV variant preferred the smaller pentenoic acid and required higher concentrations to fully bind hexenoic acid. In contrast to samples incubated with 3-methylcrotonic acid, the UV-Vis spectra of samples incubated with pentenoic or hexenoic acid were unaffected by a desalting step, indicating that pentenoic and hexenoic acid irreversibly binds to TaFdc/TaFdcV. Quantitative GC assay indicates that pentene production from hexenoic acid by AnFdc is limited to a single turnover.

### Crystal structures of TaFdc and TaFdcV reveal mutation impact on the substrate-binding pocket

In order to understand how TaFdcV mutations aid in isobutene production, crystal structures of TaFdc and TaFdcV were solved at a resolution of 1.74 and 1.89 Å, respectively. An overlay of the wild-type and the variant crystal structures shows that the key residues F447, Q200 and the catalytic network of E287-R183-E292 are unaffected by the mutations (Fig. 5A). The T405M mutation is located at the active site, extending towards the space above the prFMN uracil ring while the Q448W and F404Y mutations are situated in the second shell from the active site. The E292 residue side chain occupies 'up' and 'down' conformations, while weak electron density suggests a high degree of mobility for the L449 side chain. The mobile E292 and L449 gate access to the active site (Fig. 5B) while the Q448W mutation in TaFdcV narrows the binding pocket (Fig. 5C). The T405M and Q448W mutations are likely to be responsible for the increased selectivity for 3-methylcrotonic acid in TaFdcV by enhancing the substrate/active site shape complementarity, blocking access to larger substrates (Fig. 6). While comparison of TaFdc and TaFdcV crystal structures reveals the basis for increased selectivity in the evolved enzyme, it is not immediately clear why 3-methylcrotonic acid can yield isobutene from Int3.

### Formation of stable cycloadducts with inhibitors

The effects of crotonic and 2-butynoic acid on TaFdcV were studied to determine whether the mutations that increase in 3-methylcrotonic acid turnover also affected activity with related compounds. Incubation of TaFdcV with 2-butynoic and crotonic acid led to the familiar split of the 380 nm prFMN peak in the UV-Vis spectrum (Fig. 7A and D), similar to 3-methylcrotonic acid. However, as previously observed with pentenoic and hexenoic acid, the spectrum did not recover the following desalting, suggesting that a covalent inhibitory adduct is formed. Similar trends were observed with TaFdc, however, incubation at higher inhibitor concentration was required to drive changes in the UV-Vis spectrum.

Upon addition of crotonic acid, a gradual shift in UV-Vis spectrum occurs over minutes, allowing estimation of adduct formation rate (Fig. 8A). The observed rate remains first order with respect to crotonic acid, with k_{obs} = 0.34 ± 0.03 min at the highest concentration tested (50 mM) (Fig. 8B). In contrast, a similar shift in UV-Vis spectrum upon addition of the substrate 3-methylcrotonic acid occurs rapidly within seconds, and at substantially lower 3-methylcrotonic acid concentrations. This suggests that crotonic acid adduct formation is hindered by a higher K_{D} and/or slower rate of cycloaddition.

ESI-MS and co-crystallization studies confirmed that the TaFdcV 2-butynoic acid adduct stalls as Intl, while the TaFdcV crotonic acid adduct undergoes decarboxylation to stall at the Int3 species (Fig. 7). No decarboxylation of the Int1 with 2-butynoic acid was detected, even in 1-month-old crystals.

### AnFdcll with three point-mutations has an identical active site conformation to TaFdcV

To further understand how the architecture of the active site affects the decarboxylation of 3-methylcrotonic acid, corresponding key mutations from TaFdcV were introduced in AnFdc. AnFdc has been established as a model system due to the fact that it readily yields atomic resolution crystal structures. Two variants were studied: AnFdc T395M (AnFdcl) and the triple mutant AnFdc T395M R435P P438W (AnFdcll). Overlay of the AnFdc wild-type and TaFdcV crystal structures reveals a downward shift of the Y404 residue in TaFdcV in the secondary shell compared to the corresponding Y394 in AnFdc (Fig. 9A). The Y394 residue is unaffected in the AnFdcl variant compared to wild-type (Fig. 9B). In contrast, the active site of the AnFdcll variant matches that of TaFdcV in the conformation of Y394 and M395 (Fig. 9C).

As expected, neither AnFdcl nor AnFdcll were active with cinnamic acid, likely due to a clash between the substrate phenyl ring and M395. While binding of crotonic acid in AnFdc wild-type cannot be detected by the UV-Vis spectra over 2-h incubation, both mutants AnFdcl and AnFdcll readily bind the inhibitor, evident from UV-Vis spectra, demonstrating increased selectivity towards smaller substrates.

### TaFdcll has comparable isobutene production activity to TaFdcV

Selected TaFdc variants (wild-type, TaFdcl i.e. T405M, TaFdcV, TaFdcll), and AnFdc variants (wildtype, AnFdcl, AnFdcll) were purified and assayed for isobutene production. TaFdcll (F404Y, T405M, V445P, Q448W) was created by rational design based on the structural analysis of TaFdc wild-type, TaFdcV and AnFdcll. A comparison of the isobutene titre obtained following 2 and 4 h incubation revealed TaFdcl and AnFdcl produced 4-9 times the amount of isobutene compared to the wild-type enzymes. Additional mutations in AnFdcll and TaFdcll led to a substantial further increase of 18 and 81 fold, respectively, in isobutene production (Fig. 10). Surprisingly, the in vitro titer obtained with TaFdcll was slightly higher than the corresponding TaFdcV levels obtained (Fig. 11). Thus, the 4 point mutations in TaFdcll (F404Y, T405M, V445P, Q448W) and 3 point mutations in AnFdcll (T395M, R435P, P438W), that create an active site architecture identical to TaFdcV (Fig.9), appear largely responsible for the increased isobutene activity compared to wild-type TaFdc and AnFdc.

While AnFdc wild-type was included in the initial UbiD screen, the AnFdc wild-type was 90 times lower in activity in vivo compared to TaFdc. Hence, AnFdc was not selected for further directed evolution, despite having comparable in vitro activity to TaFdc. The disparate and lower activity in vivo might be attributed to AnFdc-specific inhibition by metabolites such as phenylacetaldehyde. An initial comparison of in vitro isobutene production levels using crude cell lysate from cells expressing TaFdc variants with those expressing MVD and/or M3K reveals a ^{~}50-fold increase is observed for TaFdcV compared to MVD/M3K levels (Fig.12). This demonstrates that the evolved TaFdcV is vastly superior in catalysing the decarboxylative step compared to the previously described enzyme systems.

### Computational studies reveal a mechanistic basis for isobutene production

It is curious that a single methyl group difference, as occurs between crotonic acid and 3-methylcrotonic acid, determines whether the compound is a substrate or inhibitor for the evolved Fdc variants. The marked influence of the additional methyl group on Int3 cycloelimination suggests this step may proceed via a cationic or radical beta carbon stabilized through additional hyperconjugation effects. A density functional theory (DFT) active site 'cluster' model (Fig.13) was used to investigate why 3-methylcrotonic acid is decarboxylated and eliminated by TaFdcV in contrast to crotonic acid.

The potential energy surface for the cycloelimination of Int3 to the non-covalent product complex was computed for both crotonic acid and 3-methylcrotonic acid by varying the C_{α}―C₁ and C_{β}―C₄ₐ bond lengths (Fig. 14). These suggest that 3-methylcrotonic acid undergoes a more asynchronous elimination, with the transition state C_{α}―C₁ and C_{β}―C₄ₐ bond lengths of 1.96 and 2.97 Å, respectively, compared to 1.95 and 2.77 Å for crotonic acid, respectively. This is linked to an increased charge separation occurring between the C and prFMN for the 3-methylcrotonic acid compared to crotonic acid (Tables 5 and 6), possibly affected by additional hyperconjugation in the case of 3-methylcrotonic acid. The release of propene from crotonic acid Int3 cycloadduct is more endothermic by ^{~}8 kJ mol⁻¹ and has a higher energy barrier by 19 kJ mol⁻¹ compared to the release of isobutene from Int3 with 3-methylcrotonic acid. If the activation entropy is similar for the two reactions then the transition state energy difference translates to a ^{~}2200 slower rate for the release of propene from Int3 at 293 K, explaining the lack of crotonic acid turnover under conditions tested.

**Table 5: Natural charge analysis for DFT models with 3-methylcrotonic acid**

| moiety | summed natural charges | | | | |
|---|---|---|---|---|---|
| | Int3 | TS | Product | Prod - Int3 | TS - Int3 |
| substrate (3-methyl crotonic acid) | 0.11 | 0.26 | -0.02 | -0.13 | 0.15 |
| Cα | -0.47 | -0.53 | -0.49 | -0.02 | -0.06 |
| Cβ | -0.03 | 0.15 | -0.05 | -0.02 | 0.18 |
| C4a | 0.01 | -0.07 | -0.05 | -0.06 | -0.09 |
| N5 | -0.46 | -0.36 | -0.30 | 0.16 | 0.11 |
| C1' | -0.04 | 0.08 | 0.15 | 0.19 | 0.12 |
| prFMN | 0.01 | 0.05 | 0.05 | 0.05 | 0.05 |
| isoalloxazine | -0.62 | -0.89 | -0.69 | -0.07 | -0.27 |
| additional prenyl carbons | 0.25 | 0.43 | 0.51 | 0.26 | 0.18 |
| 'tail' | 0.26 | 0.25 | 0.26 | -0.01 | -0.01 |
| Cγ | -0.69 | -0.72 | -0.69 | 0.00 | -0.03 |
| Cγ' | -0.70 | -0.73 | -0.70 | 0.00 | -0.03 |

**Table 6: Natural charge analysis for DFT models with crotonic acid**

| moiety | summed natural charges | | | | |
|---|---|---|---|---|---|
| | Int3 | TS | Product | Prod - Int3 | TS - Int3 |
| substrate (crotonic acid) | 0.12 | 0.21 | -0.02 | -0.14 | 0.09 |
| Cα | -0.47 | -0.52 | -0.49 | -0.02 | -0.05 |
| Cβ | -0.23 | -0.10 | -0.24 | -0.01 | 0.14 |
| C4a | 0.01 | -0.06 | -0.06 | -0.06 | -0.06 |
| N5 | -0.47 | -0.35 | -0.29 | 0.18 | 0.12 |
| C1' | -0.04 | 0.06 | 0.17 | 0.21 | 0.11 |
| prFMN | 0.03 | 0.05 | 0.06 | 0.03 | 0.02 |
| isoalloxazine | -0.62 | -0.83 | -0.70 | -0.08 | -0.21 |
| additional prenyl carbons | 0.26 | 0.42 | 0.53 | 0.27 | 0.16 |
| 'tail' | 0.27 | 0.25 | 0.25 | -0.01 | -0.01 |
| Cγ | -0.69 | -0.72 | -0.71 | -0.02 | -0.03 |
| H | 0.26 | 0.24 | 0.23 | -0.03 | -0.02 |

### The limit of prFMN-dependent (de)carboxylation by UbiD enzymes

Directed evolution of TaFdc to TaFdcV resulted in a marked increase in activity with 3-methylcrotonic acid. Surprisingly, the evolved mutant remained unable to convert crotonic acid to the corresponding propene. This contrasts with previous evolved studies aimed at expanding the AnFdc substrate repertoire to include (hetero)aromatic compounds. In this case, the evolution of activity against heteroaromatic bicyclic compounds yielded a broad specificity variant able to convert even naphthoic acid. It is thus possible that 3-methylcrotonic acid represents a limit for bona fide UbiD-substrates, indicating that prFMN-dependent catalysis requires more than an α,β-unsaturated acrylic acid (i.e. a secondary C carbon) to yield reversible cycloelimination.

Indeed, crotonic acid readily forms irreversible adducts with (evolved) Fdc that proceed to the last step prior to product formation. In the case of smaller substrates such as (3-methyl) crotonic acid, the scope for enzyme-induced strain as a tool to optimize the energy landscape is minimal. In this case, cycloelimination of isobutene appears feasible at ambient conditions whereas propene production is not. Computational studies provide a rationale behind these observations, suggesting a ^{~}2200 fold slower rate for the release of propene from Int3. Thus, further optimization of isobutene production and future evolution of propene producing Fdc variants will need to focus on the energetics of the hydrocarbon elimination step.

### Methods

### In vivo isobutene assay

In vivo screenings were carried out on a 96-well plate (DW96, 2.2 mL wells, sealed with a foil sheet). TaFdc and other UbiD homologues were co-expressed with UbiX (E. coli, K-12) in a petDuet vector (UbiD in MCS1 and UbiX in MCS2) in E. coli (BL21, DE3). Isobutene production from 0.4 mL reaction mix with 10 mM 3-methylcrotonic acid was detected from the headspace by gas chromatography. The GC method consisted of 100 µL of headspace with a split ratio of 10 injected to RTX-1 column (15 m, 0.32 mm internal diameter, 5 µm film thickness, from RESTEK 10178-111) using nitrogen as a carrier gas (1 mL/min flow rate). The oven temperature was held at 100 °C and the injector and detector were maintained at 250 °C. Isobutene was calibrated at 1000, 5000 and 10,000 ppm with standards from Messer.

### Mutagenesis

Point mutations (TaFdcl, TaFdcll, AnFdcl, AnFdcll) were generated with a Q5 mutagenesis kit from New England Biolabs. Primers were designed with NEBaseChanger (New England Biolabs). The presence of the point mutation was confirmed by sequencing (Eurofins).

### Protein expression

A pETDuet-1 vector containing genes for *T. atroviride* Fdc (with an N-terminal 6-histidine affinity tag) and UbiX (E. coli, K-12) was transformed into BL21(DE3) competent cells following the manufacturer's protocol (Novagen). A colony was inoculated into Lysogeny Broth (supplemented with 100 µg/mL ampicillin) and incubated by shaking overnight at 37 °C. 5 mL of LB culture was inoculated into 1 L of Terrific Broth (TB, Formedium), supplemented with 100 µg/mL ampicillin. The culture was incubated by shaking at 37 °C until the optical density of 0.6-0.8. The cells were induced with 0.4 mM isopropyl β-D-1- thiogalactopyranoside (IPTG) and supplemented with 0.5 mM MnCl₂. The cultures were incubated by shaking at 18 °C for 24 h. The cells were harvested by centrifugation (10 min, 8939 × g) and frozen.

### Protein purification

Frozen cells were supplemented with EDTA-free protease inhibitor mixture (Roche Applied Science), lysozyme, DNAse, and RNAse (Sigma) and resuspended (50 mM HEPES, 300 mM KCI, pH 6.8). The cells were lysed by sonication on ice (Bandelin Sonoplus sonicator, TT13/F2 tip, 30% power with 20 s on/40 s off for 15 min) and centrifuged (1 h, 174,000-185,500 × g, Beckman Optima LE-80k ultracentrifuge, Ti50.2 rotor). The cell-free extract was loaded on Ni-NTA resin, washed with 4 column volumes of 40 mM imidazole buffer (40 mM imidazole, 50 mM HEPES, 300 mM KCI, pH 6.8) and eluted in 1 mL fractions with 250 mM imidazole buffer (250 mM imidazole, 50 mM HEPES, 300 mM KCI, pH 6.8). Fractions containing protein were combined and desalted into 25 mM HEPES, 150 mM KCI, pH 6.8. Exposure to light was minimized by covering with foil and using black Eppendorf tubes.

### Cycloadduct formation

TaFdcV (500 µL, 0.45 mM protein, 25 mM HEPES, 150 mM KCI, pH 6.8) was incubated with crotonic or 2-butynoic acid. The formation of the prFMN-crotonic acid cycloadduct was followed by UV-Vis spectroscopy and additional acid were added until full conversion (complete loss of the 380 nm peak). The protein was desalted to 25 mM HEPES, 150 mM KCI, pH 6.8 and plated for crystal trials.

### Crystallization and X-ray structure determination

Crystallization was performed by sitting-drop vapour diffusion. Screening of 0.3 µL of 1 mg/mL TaFdcV in 25 mM HEPES, 150 mM KCI, pH 6.8, and 0.3 µL of reservoir solution at 4 °C resulted in a number of hits in the BCS plate from molecular dimensions. Seed stocks were used to reproduce TaFdc wild-type crystals and co-crystals with 2-butynoic and crotonic acids in the BCS plate. Crystals were cryoprotected with PEG200 and flash-frozen in liquid nitrogen. AnFdc wild-type and variants were crystallized in 0.2 M potassium thiocyanate, Bis-Tris propane 6.5, 20% w/v PEG 3350 at 4 °C. Diffraction data were collected at Diamond beamlines and processed using the CCP4 suite version 7.1. Phaser MR version 2.8.3 was used to perform molecular replacement using 4ZA4 [https://doi.org/10.2210/pdb4ZA4/pdb] as a model. Refinement was carried out with REFMAC5 and manual rebuilding in COOT version 0.9.5. Ligand definitions and coordinates were generated with AceDRG.

### In vitro isobutene assay comparing TaFdc and AnFdc variants

All variants were grown in BL21(DE3) cells with a pETDuet plasmid with the Fdc variant (N-terminal 6-His-tag) in the first multiple cloning site and UbiX (untagged, wild-type from E. coli K12) in second multiple cloning site. The cells were grown in a ZYM-5052 autoinducing medium (30 °C for 6 h, followed by 18 °C for 24 h). The Fdc variants were purified with Protino Ni-IDA column and stored at -80 °C (in 50 mM Tris-HCI pH 7.5, 1 mM MnCl₂, 20 mM NaCl, 200 mM KCI, 10% glycerol).

Decarboxylation of 3-methylcrotonate was set up in triplicates in 50 mM Tris-HCI pH 7.5, 1 mM MnCl₂, 20 mM NaCl, 200 mM KCI with 10 mM 3-methylcrotonate and 0.3 mg/mL enzyme in DW384 plates (40 µL per well, sealed with foil sheet). Isobutene production was measured from headspace by gas chromatography after 2 and 4 h.

### In vitro isobutene assay comparing TaFdc, ScMVD and PtM3K

An equal amount of E. coli cells containing either empty pETDuet (as control) or one of the following plasmids: pETDuet TaFdc_UbiX, pETDuet TaFdcV_UbiX, pETDuet PtM3K (P. torridus mevalonate 3-kinase, Uniprot: Q6KZB1), pETDuet ScMVD (S. cerevisiae MVD, Uniprot: P32377) or pETDuet PtM3K-ScMVD, were lysed in 50 mM Tris-HCI pH 7.5, 20 mM KCI, 2 mM MgCl₂, 1 g/L lysozyme, 0.03 g/L DNAse for 1 h at 37 °C. A total of 150 µL of lysate was transferred to a 2 mL GC-vial and MgCl₂ (10 mM final concentration) was added. Substrates were added to 50 mM final concentration and 200 µL total volume, and consisted of either 3-hydroxyisovalerate/ATP, 3-phosphonooxy-isovalerate/ADP or 3-methylcrotonate. Following 4 h of incubation at either 37 or 50 °C, the reaction mixture was inactivated by incubation at 90 °C for 5 min. GC analysis of the gas phase was carried out as described above to determine isobutene levels produced. All reactions were carried out in duplicates.

### DFT calculations

TaFdcV active site cluster model with crotonic AQ7 (365 atoms) and 3-methylcrotonic acid (368 atoms) was built based on the TaFdcV crystal structure with crotonic acid bound as Int3 adduct and modelled at the B3LYP/6-31 G(d,p) level of theory with the D3 version of Grimme's dispersion with Becke-Johnson damping and a generic polarizable continuum with ε = 5.7 using the polarizable continuum model[ 25 ]. C_{α}―C₁ and C_{β}―C₄ₐ bonds were both fixed for any single DFT optimization and substrate release was modelled using Gaussian 09 revision D.01. by lengthening one bond by 0.05 Å at a time, resulting in a 3D energy landscape consisting of ^{~}

## Claims

1. A recombinant organism or microorganism having a decreased pool of crotonic acid compared to the organism or microorganism from which it is derived due to at least:
(i) a decreased conversion of acetaldehyde into crotonaldehyde; and/or
(ii) a decreased conversion of crotonyl-CoA into crotonaldehyde; and/or
(iii) a decreased conversion of crotonaldehyde into crotonic acid.

2. The recombinant organism or microorganism according to claim 1, wherein said recombinant organism or microorganism is a recombinant microorganism, wherein said recombinant microorganism is *Escherichia coli.*

3. The recombinant organism or microorganism according to claim 1 or 2, wherein the decreased conversion of acetaldehyde into crotonic acid is due to a reduced level and/or activity of an aldehyde lyase (EC 4.1.2) in said organism or microorganism.

4. The recombinant organism or microorganism according to claim 3, wherein the aldehyde lyase (EC 4.1.2) is a deoxyribose-phosphate aldolase (EC 4.1.2.4), in particular wherein the deoxyribose-phosphate aldolase (EC 4.1.2.4) is DeoC from *Escherichia coli.*

5. The recombinant organism or microorganism according to claim 1 or 2, wherein the decreased conversion of crotonyl-CoA into crotonaldehyde is due to a reduced level and/or activity of an aldehyde dehydrogenase (EC 1.2) is said organism or microorganism.

6. The recombinant organism or microorganism according to claim 5, wherein the aldehyde dehydrogenase (EC 1.2) is an acetaldehyde dehydrogenase (acetylating) (EC 1.2.1.10) in particular wherein the acetaldehyde dehydrogenase (acetylating) (EC 1.2.1.10) is MhpF from *Escherichia coli,* AdhE from *Escherichia coli* and/or EutE from *Escherichia coli.*

7. The recombinant organism or microorganism according to claim 1 or 2, wherein the decreased conversion of crotonaldehyde into crotonic acid is due to a reduced level and/or activity of an aldehyde dehydrogenase (EC 1.2) in said organism or microorganism.

8. The recombinant organism or microorganism according to claim 7, wherein the aldehyde dehydrogenase (EC 1.2) is an NAD+ or NADP+-dependent aldehyde dehydrogenase (EC 1.2.1), or an iron-sulfur protein-dependent aldehyde dehydrogenase (EC 1.2.7), in particular wherein the aldehyde dehydrogenase (EC 1.2) is PuuC from *Escherichia coli* (EC 1.2.1.19 and 1.2.1.24 and 1.2.1.99), FeaB from *Escherichia coli* (EC 1.2.1.39), PatD from *Escherichia coli* (EC 1.2.19), AldA from *Escherichia coli* (EC 1.2.1.21 and 1.2.1.22), Sad from *Escherichia coli* (EC 1.2.1.24), AstD from *Escherichia coli* (EC 1.2.1.71), BetB from *Escherichia coli* (EC 1.2.1.8), AldB from *Escherichia coli* (EC 1.2.1.4), and/or GabD from *Escherichia coli* (EC 1.2.1.79).

9. The recombinant organism or microorganism according to any one of claims 3 to 8, wherein the reduced level of an enzyme is due to
(i) a complete or partial deletion of a gene encoding the respective enzyme in said organism or microorganism; and/or
(ii) a deletion or an inactivating mutation in a regulatory element of a gene encoding the respective enzyme in said organism or microorganism; and/or
wherein the reduced activity of an enzyme is due to
(i) an inactivating mutation in a gene encoding the respective enzyme in said organism or microorganism; and/or
(ii) the addition of an inhibitor of the respective enzyme.

10. The recombinant organism or microorganism according to any one of claims 1 to 9 further encoding a ferulic acid decarboxylase, in particular wherein the ferulic acid decarboxylase catalyzes the formation of an alkene from a corresponding carboxylic acid, in particular wherein the formation of the alkene is catalyzed by a ferulic acid decarboxylase.

11. The recombinant organism or microorganism according to any one of claims 1 to 10, wherein the recombinant organism or microorganism is capable of:
(i) enzymatically converting acetyl-CoA into 3-methylcrotonic acid and/or isobutene; and/or
(ii) enzymatically converting 3-methylcrotonic acid into isobutene; and/or
(iii) enzymatically converting *cis*,*cis*-muconic acid or pentadienoic acid into 1-3- butadiene.

12. The recombinant organism or microorganism according to claim 11, wherein the conversion of acetyl-CoA into 3-metyhlcrotonic acid comprises the steps of:
(i) enzymatically converting acetyl-CoA into acetoacetyl-CoA,
(ii) enzymatically converting said produced acetoacetyl-CoA into 3-hydroxy-3-methylglutaryl-CoA,
(iii) enzymatically converting said produced 3-hydroxy-3-methylglutaryl-CoA into 3-methylglutaconyl-CoA,
(iv) enzymatically converting said produced 3-methylglutaconyl-CoA into 3-methylcrotonyl-CoA, and
(v) enzymatically converting said produced 3-methylcrotonyl-CoA into 3-methylcrotonic acid.

13. The recombinant organism or microorganism according to claim 12, wherein the recombinant organism or microorganism is further capable of enzymatically converting the produced 3-methylcrotonic acid into isobutene; in particular wherein the conversion of 3-methylcrotonic acid into isobutene is catalyzed by a ferulic acid decarboxylase.

14. Use of the recombinant organism or microorganism according to any one of claims 1 to 13 in the production of an alkene, in particular wherein the alkene is produced by a ferulic acid decarboxylase; and/or wherein the alkene is isobutene or 1,3-butadiene.

15. A method for the production of isobutene, the method comprising the steps of:
a) producing 3-methylcrotonic acid by culturing a recombinant organism or microorganism as defined in any one of embodiments 11 to 13 in a suitable culture medium under suitable conditions; and
b) enzymatically converting said produced 3-methylcrotonic acid into isobutene;
in particular wherein the conversion of 3-methylcrotonic acid into isobutene is catalyzed by a ferulic acid decarboxylase.
